# EUROPÄISCHE PATENTSCHRIFT

(11) **EP 3 994 520 B1**
(45) Veröffentlichungstag und Bekanntmachung des Hinweises auf die Patenterteilung: **09.12.2026**
(21) Anmeldenummer: 20740252.0
(22) Anmeldetag: 01.07.2020
(51) Int. Cl.: G02C 7/02, A61B 3/10, A61B 3/00, A61B 3/04

(54) **VERFAHREN UND VORRICHTUNG ZUR OPTIMIERUNG VON BRILLENGLÄSERN FÜR TRÄGER VON IMPLANTIERTEN INTRAOKULARLINSEN**
METHOD AND APPARATUS FOR OPTIMIZING SPECTACLE LENSES FOR WEARERS OF IMPLANTED INTRAOCULAR LENSES
PROCÉDÉ ET DISPOSITIF D'OPTIMISATION DE VERRES DE LUNETTES POUR PORTEURS DE CRISTALLINS INTRAOCULAIRES IMPLANTÉS

(30) Priorität: 02.07.2019 DE 102019004654
(43) Veröffentlichungstag der Anmeldung: 11.05.2022
(73) Patentinhaber: Rodenstock GmbH, 80687 München (DE)
(72) Erfinder: TRUMM, Stephan, 80999 München (DE); BECKEN, Wolfgang, 82061 Neuried (DE); MUSCHIELOK, Adam, 81476 München (DE); SEIDEMANN, Anne, 81375 München (DE); ALTHEIMER, Helmut, 87650 Baisweil-Lauchdorf (DE); ESSER, Gregor, 80686 München (DE)
(74) Vertreter: Müller-Boré & Partner Patentanwälte PartG mbB
(86) Internationale Anmeldenummer: PCT/EP2020/068572
(87) Internationale Veröffentlichungsnummer: WO 2021/001452

(56) Entgegenhaltungen:
- EP-A1- 3 355 098
- WO-A1-02/088830
- WO-A2-03/022137
- DE-A1- 102013 020 706
- DE-A1- 102017 007 975

## Beschreibung

Die vorliegende Erfindung betrifft ein Verfahren und eine Vorrichtung zur Berechnung (Optimierung) und Herstellung eines Brillenglases mit Hilfe eines teilweise individuellen Augenmodells, wobei relevante individuelle Parameter zumindest eines Auges eines Brillenträgers ermittelt werden, und ein Computerprogrammerzeugnis zur Berechnung (Optimierung) des Brillenglases.
Das zumindest eine Auge des Brillenträgers weist dabei eine implantierte Intraokularlinse (IOL) auf. Beispielsweise kann im Rahmen einer Operation anstelle oder zusätzlich zu der natürlichen Augenlinse eine Intraokularlinse in das zumindest eine Auge implantiert worden sein. Mit anderen Worten handelt es sich bei dem Brillenträger um einen Träger einer implantierten Intraokularlinse. Ferner betrifft die vorliegende Erfindung ein Verfahren, eine Vorrichtung und ein entsprechendes Computerprogrammerzeugnis zur Berechnung (Optimierung) und Herstellung eines Brillenglases für einen Träger einer implantierten Intraokularlinse, mit Hilfe eines teilweise individuellen Augenmodells.

Für die Herstellung bzw. Optimierung von Brillengläsern, insbesondere von individuellen Brillengläsern wird jedes Brillenglas so gefertigt, dass für jede gewünschte Blickrichtung oder jeden gewünschten Objektpunkt eine möglichst gute Korrektur eines Refraktionsfehlers des jeweiligen Auges des Brillenträgers erreicht wird. Im Allgemeinen gilt ein Brillenglas für eine gegebene Blickrichtung dann als vollkorrigierend, wenn die Werte für Sphäre, Zylinder und Achse der Wellenfront beim Passieren der Scheitelpunktkugel mit den Werten für Sphäre, Zylinder und Achse der Verordnung für das fehlsichtige Auge übereinstimmen. Bei der Refraktionsbestimmung für ein Auge eines Brillenträgers werden dioptrische Werte (insbesondere Sphäre, Zylinder, Achslage - also insbesondere sphäro-zylindrische Abweichungen) für eine weite (in der Regel unendliche) Entfernung und gegebenenfalls (für Mehrstärkengläser bzw. Gleitsichtgläser) eine Addition oder eine vollständige Nahrefraktion für eine nahe Entfernung (z.B. nach DIN 58208) bestimmt. Bei modernen Brillengläsern können zusätzlich auch von der Norm abweichende Objektentfernungen, die bei der Refraktionsbestimmung verwendet wurden, angegeben werden. Damit ist die Verordnung (insbesondere Sphäre, Zylinder, Achslage und gegebenenfalls Addition oder Nahrefraktion) festgelegt, die an einen Brillenglashersteller übermittelt wird. Kenntnisse über eine spezielle bzw. individuelle Anatomie des jeweiligen Auges oder die tatsächlich im Einzelfall vorliegenden Brechwerte des fehlsichtigen Auges sind dafür nicht erforderlich.

Eine vollständige Korrektur für alle Blickrichtungen gleichzeitig ist aber im Normalfall nicht möglich. Daher werden die Brillengläser derart gefertigt, dass sie vor allem in den hauptsächlichen Nutzungsbereichen, insbesondere in den zentralen Durchblicksbereichen eine gute Korrektur von Fehlsichtigkeiten des Auges und nur geringe Abbildungsfehler bewirken, während in peripheren Bereichen größere Abbildungsfehler zugelassen werden.

Um ein Brillenglas derart fertigen zu können, erfolgt zunächst eine Berechnung der Brillenglasflächen bzw. zumindest einer der Brillenglasflächen derart, dass dadurch die gewünschte Verteilung der unvermeidlichen Abbildungsfehler bewirkt wird. Diese Berechnung und Optimierung erfolgt üblicherweise mittels eines iterativen Variationsverfahrens durch Minimieren einer Zielfunktion. Als Zielfunktion wird insbesondere eine Funktion F mit folgendem funktionalen Zusammenhang zur sphärischen Wirkung S, zum Betrag der zylindrischen Wirkung Z und zur Achslage des Zylinders *α* (auch als "SZA"-Kombination bezeichnet) berücksichtigt und minimiert: $F = {\sum }_{i = 1}^{m} \left[{g}_{i , S Δ}{\left({S}_{Δ , i}-{S}_{Δ , i , Soll}\right)}^{2}+{g}_{i , Z Δ}{\left({Z}_{Δ , i}-{Z}_{Δ , i , Soll}\right)}^{2}+…\right]$

Dabei werden in der Zielfunktion F an den Bewertungsstellen *i* des Brillenglases zumindest die tatsächlichen Refraktionsdefizite der sphärischen Wirkung *S_{Δ,i}* und der zylindrischen Wirkung *Z_{Δ,i}* sowie Sollvorgaben für die Refraktionsdefizite der sphärischen Wirkung *S_{Δ,i,Soll}* und der zylindrischen Wirkung *Z_{Δ,i,Soll}* berücksichtigt.

Bereits in DE 103 13 275 wurde erkannt, dass es vorteilhaft ist, die Sollvorgaben nicht als absolute Werte der zu optimierenden Eigenschaften sondern als deren Abweichungen von der Verordnung, also als geforderte lokale Fehlanpassung anzugeben. Dies hat den Vorteil, dass die Sollvorgaben unabhängig von der Verordnung (*Sph_{V},Zyl_{V},Achse_{V},Pr_{V},B_{V}*) sind und die Sollvorgaben nicht für jede individuelle Verordnung geändert werden müssen. Als "Ist"-Werte der zu optimierenden Eigenschaften fließen in die Zielfunktion somit auch nicht absolute Werte dieser optischen Eigenschaften, sondern die Abweichungen von der Verordnung ein. Dies hat den Vorteil, dass die Sollvorgaben unabhängig von der Verordnung vorgegeben werden können und nicht für jede individuelle Verordnung geändert werden müssen.

Die jeweiligen Refraktionsdefizite an den jeweiligen Bewertungsstellen werden vorzugsweise mit Gewichtungsfaktoren *g_{i,sΔ}* bzw. *g_{i,zΔ}* berücksichtigt. Dabei bilden die Sollvorgaben für die Refraktionsdefizite der sphärischen Wirkung *S*_{*Δ*,*i,Soll*} und/oder der zylindrischen Wirkung *Z_{Δ,i,Soll}* insbesondere zusammen mit den Gewichtungsfaktoren *g_{i,sΔ}* bzw. *g_{i,zΔ}* das sogenannte Brillenglasdesign. Darüber hinaus können insbesondere auch weitere Residuen, insbesondere weitere zu optimierende Größen, wie z.B. Koma und/oder sphärische Aberration und/oder Prisma und/oder Vergrößerung und/oder anamorphotische Verzerrung, usw., berücksichtigt werden, was insbesondere durch den Ausdruck "+..." in der oben genannten Formel für die Zielfunktion F angedeutet ist.

In manchen Fällen kann es zu einer deutlichen Verbesserung insbesondere einer individuellen Anpassung eines Brillenglases beitragen, wenn bei der Optimierung des Brillenglases nicht nur Abbildungsfehler bis zur zweiten Ordnung (Sphäre, Betrag des Astigmatismus und Achslage), sondern auch höherer Ordnung (z.B. Koma, Dreiblattfehler, sphärische Aberration) berücksichtigt werden.

Es ist aus dem Stand der Technik bekannt, für optische Elemente und insbesondere für Brillengläser, die durch mindestens zwei brechende, refraktive Grenzflächen begrenzt sind, die Form einer Wellenfront zu bestimmen. Dies kann beispielsweise durch numerische Berechnung einer ausreichenden Anzahl an Nachbarstrahlen erfolgen, verbunden mit einem anschließenden Fit der Wellenfront durch Zernike-Polynome. Ein anderer Ansatz beruht auf einer lokalen Wellenfrontdurchrechnung bei der Refraktion (siehe WO 2008/089999 A1). Hierbei wird pro Durchblickpunkt nur ein einziger Strahl berechnet (der Hauptstrahl) und begleitend die Ableitungen der Pfeilhöhen der Wellenfront nach den transversalen (zum Hauptstrahl senkrechten) Koordinaten. Diese Ableitungen können bis zu einer bestimmten Ordnung gebildet werden, wobei die zweiten Ableitungen die lokalen Krümmungseigenschaften der Wellenfront (wie z.B. Brechwert, Astigmatismus) beschreiben und die höheren Ableitungen mit den Abbildungsfehlern höherer Ordnung zusammenhängen.

Bei einer Durchrechnung von Licht durch ein Brillenglas werden die lokalen Ableitungen der Wellenfronten an einer geeigneten Position im Strahlverlauf berechnet, um sie dort mit erwünschten Werten, die aus der Refraktion des Brillenglasträgers hervorgehen, zu vergleichen. Als solche Position, an der eine Auswertung der Wellenfronten stattfindet, wird in der Regel die Scheitelpunktkugel oder beispielsweise die Hauptebene des Auges bei der entsprechenden Blickrichtung herangezogen. Dabei wird angenommen, dass eine sphärische Wellenfront vom Objektpunkt ausgeht und bis zur ersten Brillenglasfläche propagiert. Dort wird die Wellenfront gebrochen und propagiert anschließend zur zweiten Brillenglasfläche, wo sie wieder gebrochen wird. Die letzte Propagation findet dann von der zweiten Grenzfläche bis zur Scheitelpunktkugel (oder der Hauptebene des Auges) statt, wo die Wellenfront mit vorgegebenen Werten für die Korrektion der Refraktion des Auges des Brillenträgers verglichen wird.

Um diesen Vergleich auf Basis der ermittelten Refraktionsdaten des jeweiligen Auges durchzuführen, wird der Auswertung der Wellenfront an der Scheitelpunktkugel ein etabliertes Modell des fehlsichtigen Auges unterstellt, in welchem einem rechtsichtigen Grundauge eine Fehlsichtigkeit (Refraktionsdefizit) überlagert wird. Dies hat sich besonders bewährt, da hierfür weitergehende Kenntnisse über die Anatomie bzw. Optik des jeweiligen Auges (z.B. Verteilung der Brechwerte, Augenlänge, Längenametropie und/oder Brechwertametropie) nicht erforderlich sind. Ausführliche Beschreibungen dieses Modells aus Brillenglas und Refraktionsdefizit sind beispielsweise in Dr. Roland Enders "Die Optik des Auges und der Sehhilfen", Optische Fachveröffentlichung GmbH, Heidelberg, 1995, Seiten 25 ff. und in Diepes, Blendowske "Optik und Technik der Brille", Optische Fachveröffentlichung GmbH, Heidelberg, 2002, Seiten 47 ff. enthalten. Als bewährtes Modell wird insbesondere das darin beschriebene Korrektionsmodell nach REINER verwendet.

Dabei wird als Refraktionsdefizit der Mangel oder der Überschuss an Brechwert des optischen Systems des fehlsichtigen Auges im Vergleich zu einem gleich langen rechtsichtigen Auge (Restauge) angesehen. Der Brechwert des Refraktionsdefizits ist insbesondere annähernd gleich der Fernpunktrefraktion mit negativem Vorzeichen. Für eine vollständige Korrektur der Fehlsichtigkeit bilden das Brillenglas und das Refraktionsdefizit zusammen ein Fernrohrsystem (afokales System). Das Restauge (fehlsichtiges Auge ohne eingefügtes Refraktionsdefizit) wird als rechtsichtig angenommen. Ein Brillenglas gilt damit als vollkorrigierend für die Ferne, wenn sein bildseitiger Brennpunkt mit dem Fernpunkt des fehlsichtigen Auges und damit auch mit dem objektseitigen Brennpunkt des Refraktionsdefizits zusammenfällt.

In der Druckschrift DE 10 2017 007 975 A1 bzw. WO 2018/138140 A2, worauf hierin ausdrücklich Bezug genommen wird, sind ein Verfahren und eine Vorrichtung beschrieben, die es erlauben, die Berechnung oder Optimierung eines Brillenglases zu verbessern, wobei das Brillenglas bereits mit einfachen Messungen individueller, optischer und Augen-anatomischer Daten sehr wirkungsvoll an die individuellen Anforderungen des Brillenträgers angepasst wird.

Patienten mit implantierten Intraokularlinsen (IOL), d.h. Patienten, bei denen eine Katarakt-Operation (z.B. infolge eines grauen Stars) durchgeführt wurde, werden derzeit lediglich mit handelsüblichen Brillengläsern, welche auch für Patienten mit natürlichen Augenlinsen verwendet werden, versorgt. Für Patienten mit IOLs gibt es derzeit also keine speziell angepassten Brillengläser. Somit können Spezifika von implantierten IOLs nicht berücksichtigt werden, obwohl sich die Eigenschaften implantierter Linsen deutlich von denen natürlicher Linsen unterscheiden. Beispielsweise weisen IOLs eine veränderte sphärische Wirkung zum zumindest teilweisen Ausgleich einer Hornhaut- bzw. Längenametropie und/oder eine veränderte zylindrische Wirkung zum zumindest teilweisen Ausgleich eines Hornhautastigmatismus bzw. zum zumindest teilweisen Aufheben eines Linsenastigmatismus auf. Bei herkömmlich optimierten Brillengläsern werden modellbasierte Eigenschaften für die Augenlinse angenommen, so dass gegebenenfalls das für die Optimierung verwendete Augenmodell in diesen Fällen nicht mehr dem tatsächlichen Aufbau des Auges mit einer IOL entspricht, z.B. wenn durch die IOL zumindest teilweise eine Längenametropie durch die Wirkung (mittlere Sphäre) der IOL ausgeglichen wird.

Die Druckschrift EP 3 355 098 A1 beschreibt ein Verfahren, eine Vorrichtung und ein entsprechendes Computerprogrammerzeugnis zur Berechnung (Optimierung) und Herstellung eines Brillenglases mit Hilfe eines teilweise individuellen Augenmodells.

Die Druckschrift DE 10 2013 020706 A1 beschreibt ein Verfahren zur optimierten Auswahl der in ein Auge zu implantierenden IOL.

Die Druckschrift WO 02/088830 A1 beschreibt ein Verfahren zum Entwerfen einer kundenspezifischen Augenlinse, insbesondere einer Intraokularlinse.

Die Druckschrift WO 03/022137 A2 beschreibt ein System zum Ermitteln einer für das Auge eines Patienten am besten passenden IOL.

Aufgabe der vorliegenden Erfindung ist es, die Berechnung oder Optimierung eines Brillenglases, vorzugsweise eines progressiven Brillenglases, zu verbessern. Insbesondere kann es eine Aufgabe sein, Patienten nach einer Katarakt-Operation speziell angepasste Brillengläser zur Verfügung zu stellen. Insbesondere kann es eine Aufgabe sein, die Berechnung oder Optimierung eines Brillenglases, vorzugsweise eines progressiven Brillenglases, im Hinblick auf Patienten mit einer implantierten Intraokularlinse zu verbessern. Diese Aufgabe wird insbesondere durch ein computerimplementiertes Verfahren, eine Vorrichtung, ein Computerprogrammerzeugnis, ein Speichermedium und ein entsprechendes Brillenglas mit den in den unabhängigen Ansprüchen angegebenen Merkmalen gelöst. Bevorzugte Ausführungsformen sind Gegenstand der abhängigen Ansprüche.

Ein erster Aspekt zur Lösung der Aufgabe betrifft ein computerimplementiertes Verfahren zum Berechnen oder Optimieren eines Brillenglases für zumindest ein Auge eines Brillenträgers zum Zweck der Herstellung des Brillenglases für das zumindest eine Auge, welchem im Rahmen einer Operation eine Intraokularlinse implantiert wurde, umfassend:
- ein Verfahren zum Ermitteln relevanter individueller Parameter des zumindest einen Auges des Brillenträgers;
- Vorgeben einer ersten Fläche und einer zweiten Fläche für das zu berechnende bzw. optimierende Brillenglas;
- Ermitteln des Verlaufs eines Hauptstrahls durch zumindest einen Durchblickspunkt zumindest einer zu berechnenden oder optimierenden Fläche des Brillenglases in das Modellauge;
- Auswerten einer Aberration einer entlang des Hauptstrahls aus einer auf die erste Fläche des Brillenglases auftreffenden sphärischen Wellenfront resultierenden Wellenfront an einer Bewertungsfläche im Vergleich zu einer in einem Punkt auf der Netzhaut des Augenmodells konvergierenden Wellenfront;
- iteratives Variieren der zumindest einen zu berechnenden oder optimierenden Fläche des Brillenglases, bis die ausgewertete Aberration einer vorgegebenen Sollaberration entspricht;

wobei das Verfahren zum Ermitteln relevanter individueller Parameter des zumindest einen Auges des Brillenträgers die folgenden Schritte umfasst:
   - Bereitstellen von individuellen Refraktionsdaten des zumindest einen Auges des Brillenträgers; und
   - Festlegen eines individuellen Augenmodells, in welchem zumindest
      -- eine Form und/oder Wirkung einer Hornhaut, insbesondere einer Hornhautvorderfläche, eines Modellauges;
      -- ein Hornhaut-Linsen-Abstand;
      -- Parameter der Linse des Modellauges; und
      -- ein Linsen-Netzhaut-Abstand
   als Parameter des individuellen Augenmodells festgelegt werden, wobei das Festlegen der Parameter des individuellen Augenmodells derart anhand von Daten zur Sehschärfekorrektur des zumindest einen, die Intraokularlinse aufweisenden Auges und ferner anhand von individuellen Messwerten für das Auge des Brillenträgers und/oder von Standardwerten und/oder anhand der bereitgestellten individuellen Refraktionsdaten erfolgt, dass das Modellauge die bereitgestellten individuellen Refraktionsdaten aufweist, wobei die Daten zur Sehschärfekorrektur einen ermittelten Linsen-Netzhaut-Abstand und/oder Intraokularlinsendaten umfassen;
wobei das Augenmodell ein konsistentes Augenmodell ist, und wobei eine initiale Verteilung von Parametern des Augenmodells und individuelle Daten von Eigenschaften des zumindest einen Auges bereitgestellt werden, und wobei die Parameter des individuellen Augenmodells auf Basis der initialen Verteilung von Parametern des Augenmodells und der individuellen Daten unter Verwendung von Wahrscheinlichkeitsrechnung bestimmt werden.

**In** einer bevorzugten Ausführungsform umfassen die Daten zur Sehschärfekorrektur des zumindest einen, die Intraokularlinse aufweisenden Auges (insbesondere individuelle) Intraokularlinsendaten. Somit erfolgt in dieser Ausführungsform das Festlegen der Parameter des individuellen Augenmodells derart anhand von Intraokularlinsendaten und ferner anhand von individuellen Messwerten für das Auge des Brillenträgers und/oder von Standardwerten und/oder anhand der bereitgestellten individuellen Refraktionsdaten, dass das Modellauge die bereitgestellten individuellen Refraktionsdaten aufweist, wobei das Festlegen der Parameter der Linse des Modellauges anhand der Intraokularlinsendaten erfolgt.

In einer weiteren bevorzugten Ausführungsform wird ein Linsen-Netzhaut-Abstand des Auges des Brillenträgers ermittelt, und das Festlegen der Parameter des individuellen Augenmodells erfolgt derart anhand des ermittelten Linsen-Netzhaut-Abstands und ferner anhand von individuellen Messwerten für das Auge des Brillenträgers und/oder von Standardwerten und/oder anhand der bereitgestellten individuellen Refraktionsdaten, dass das Modellauge die bereitgestellten individuellen Refraktionsdaten aufweist, wobei der Linsen-Netzhaut-Abstand des Modellauges durch den ermittelten Linsen-Netzhaut-Abstand des Auges des Brillenträgers festgelegt wird. Mit anderen Worten umfassen in dieser Ausführungsform die Daten zur Sehschärfekorrektur des zumindest einen, die Intraokularlinse aufweisenden Auges einen ermittelten Linsen-Netzhaut-Abstand.

Die Daten zur Sehschärfekorrektur umfassen einen ermittelten Linsen-Netzhaut-Abstand und/oder Intraokularlinsendaten.

Die vorliegende Erfindung bietet somit insbesondere ein computerimplementiertes Verfahren zum Ermitteln relevanter individueller Parameter zumindest eines Auges eines Brillenträgers für die Berechnung oder Optimierung eines Brillenglases für das zumindest eine Auge des Brillenträgers, wobei im Rahmen einer Operation eine Intraokularlinse in das zumindest eine Auge des Brillenträgers implantiert wurde bzw. das zumindest eine Auge des Brillenträgers (insbesondere anstelle oder zusätzlich zu der natürlichen Augenlinse) eine Intraokularlinse aufweist, umfassend die Schritte:
- Bereitstellen von individuellen Refraktionsdaten des zumindest einen Auges des Brillenträgers; und
- Festlegen eines individuellen Augenmodells, in welchem zumindest
   -- eine Form und/oder Wirkung einer Hornhaut, insbesondere einer Hornhautvorderfläche, eines Modellauges;
   -- ein Hornhaut-Linsen-Abstand;
   -- Parameter der Linse des Modellauges; und
   -- ein Linsen-Netzhaut-Abstand
als Parameter des individuellen Augenmodells festgelegt werden, wobei:
a) das Festlegen der Parameter des individuellen Augenmodells derart anhand von Intraokularlinsendaten und ferner anhand von individuellen Messwerten für das Auge des Brillenträgers und/oder von Standardwerten und/oder anhand der bereitgestellten individuellen Refraktionsdaten erfolgt, dass das Modellauge die bereitgestellten individuellen Refraktionsdaten aufweist, wobei das Festlegen der Parameter der Linse des Modellauges anhand der Intraokularlinsendaten erfolgt, und/oder
b) ein Linsen-Netzhaut-Abstand des Auges des Brillenträgers ermittelt wird und das Festlegen der Parameter des individuellen Augenmodells derart anhand des ermittelten Linsen-Netzhaut-Abstands und ferner anhand von individuellen Messwerten für das Auge des Brillenträgers und/oder von Standardwerten und/oder anhand der bereitgestellten individuellen Refraktionsdaten erfolgt, dass das Modellauge die bereitgestellten individuellen Refraktionsdaten aufweist, wobei der Linsen-Netzhaut-Abstand des Modellauges durch den ermittelten Linsen-Netzhaut-Abstand des Auges des Brillenträgers festgelegt wird.

Das Verfahren kann also sowohl die unter Punkt a) als auch die unter Punkt b) definierte Vorgehensweise umfassen. Vorzugsweise wird die unter Punkt a) definierte Vorgehensweise durchgeführt, wenn (insbesondere individuelle) Intraokularlinsendaten bekannt sind. Weiter vorzugsweise wird die unter Punkt b) definierte Vorgehensweise durchgeführt, wenn keine Intraokularlinsendaten bekannt sind. Mit anderen Worten wird die unter Punkt a) definierte Vorgehensweise im Fall von bekannten Intraokularlinsendaten durchgeführt, und die unter Punkt b) definierte Vorgehensweise wird im Fall von unbekannten Intraokularlinsendaten durchgeführt. Es ist jedoch auch möglich, dass im Fall von bekannten Intraokularlinsendaten die unter Punkt b) beschriebene Vorgehensweise durchgeführt wird (z.B. um Inkonsistenzen im Vorhinein zu vermeiden und/oder um nur einen Algorithmus oder Bestellweg vorhalten zu müssen). Ferner ist es möglich, dass im Fall von unbekannten Intraokularlinsendaten die unter Punkt a) definierte Vorgehensweise durchgeführt wird, indem bestimmte Intraokularlinsendaten angenommen werden. Insbesondere stellen die unter Punkt a) und die unter Punkt b) definierten Vorgehensweisen (voneinander unabhängige) alternative Vorgehensweisen des Verfahrens dar.

Das Verfahren kann alternativ auch entweder nur die unter Punkt a) oder die unter Punkt b) definierte Vorgehensweise umfassen. Im Rahmen der unter Punkt a) definierten Vorgehensweise werden die individuellen Intraokularlinsendaten insbesondere bereitgestellt. Dabei können die Intraokularlinsendaten bekannte (z.B. gemessene oder vom Hersteller vorgegebene) Intraokularlinsendaten umfassen bzw. sein. Alternativ oder zusätzlich können die Intraokularlinsendaten angenommene Intraokularlinsendaten umfassen bzw. sein. Insbesondere sind die Intraokularlinsendaten individuelle Intraokularlinsendaten.

Bei der Intraokularlinse kann es sich insbesondere um eine aphake Intraokularlinse oder um eine phake Intraokularlinse handeln. Eine aphake Intraokularlinse ersetzt die natürliche Augenlinse, d.h. das zumindest eine Auge des Brillenträgers weist nach der Operation nur noch die Intraokularlinse (nicht aber mehr die natürliche Augenlinse) auf. Eine phake Intraokularlinse dagegen wird zusätzlich zur natürlichen Augenlinse in das zumindest eine Auge des Brillenträgers eingesetzt bzw. implantiert, d.h. das zumindest eine Auge des Brillenträgers weist nach der Operation sowohl die Intraokularlinse als auch die natürliche Augenlinse auf.

Der Begriff "Linse des Modellauges" kann sich im Rahmen der vorliegenden Erfindung nicht nur auf eine einzelne reale Linse (z.B. die natürliche Augenlinse oder eine Intraokularlinse) beziehen, sondern auch auf ein Linsensystem. Dabei kann das Linsensystem eine oder mehrere Linsen, insbesondere zwei Linsen (nämlich die natürliche Augenlinse und ferner eine Intraokularlinse), umfassen. Mit anderen Worten kann im Rahmen der vorliegenden Erfindung die "Linse des Modellauges" eine, insbesondere modellbasierte bzw. virtuelle, Linse sein, welche die natürliche Augenlinse oder eine (aphake) Intraokularlinse beschreibt. Alternativ kann im Rahmen der vorliegenden Erfindung die "Linse des Modellauges" aber auch ein, insbesondere modellbasiertes bzw. virtuelles, Linsensystem sein, welches die natürliche Augenlinse und zusätzlich eine (phake) Intraokularlinse beschreibt. Beispielsweise kann die "Linse des Modellauges" als eine dicke Linse des Modellauges aufgefasst werden (bzw. die "Linse des Modellauges" kann eine dicke Linse des Modellauges sein), welche sowohl die Eigenschaften der natürlichen Augenlinse als auch die Eigenschaften einer zusätzlichen Intraokularlinse kombiniert und/oder beschreibt. Insbesondere wird also im Rahmen der vorliegenden Erfindung unter dem Begriff "Linse des Modellauges" ein "Linsensystem des Modellauges" verstanden. Entsprechend wird im Rahmen der vorliegenden Erfindung unter dem Begriff "Linsen-Netzhaut-Abstand" insbesondere ein "Linsensystem-Netzhaut-Abstand" verstanden. Der Einfachheit halber werden im Folgenden jedoch nur die Begriffe "Linse des Modellauges" und "Linsen-Netzhaut-Abstand" verwendet.

Eine phake Intraokularlinse kann z.B. im Augenmodell als ein eigenes bzw. zusätzliches Element berücksichtigt werden. Alternativ kann die Wirkung einer phaken Intraokularlinse in die Wirkung (bzw. in eine der Flächen oder in beide Flächen) der Hornhaut eingehen. Die Intraokularlinse kann in diesem Fall z.B. eine Vorderkammerlinse sein. Weiter alternativ kann die Wirkung einer phaken Intraokularlinse in die Wirkung (bzw. in eine der Flächen oder in beide Flächen) der natürlichen Augenlinse eingehen. In diesem Fall kann die Intraokularlinse z.B. eine Hinterkammerlinse sein. Wird die Intraokularlinse als zusätzliches Element eingebracht, können ihre Eigenschaften (insbesondere Wirkung, Flächen und/oder Dicke) als zusätzliche Parameter des Augenmodells im Augenmodell berücksichtigt werden. Beispielsweise kann im Augenmodell die Position dieser zusätzlichen Linse entweder als eine bekannte Position festgelegt oder als ein zusätzlicher Parameter (z.B. gemessen oder modellbasiert) berücksichtigt werden. Die festgelegte Position kann z.B. (im Fall einer Vorderkammerlinse) direkt oder mit einem bestimmten Abstand hinter der Hornhaut sein, oder z.B. (im Fall einer Hinterkammerlinse) direkt oder mit einem bestimmten Abstand vor der Augenlinse.

Insbesondere wird im Rahmen des ersten Aspekts ein computerimplementiertes Verfahren zum Ermitteln relevanter individueller Parameter zumindest eines Auges eines Brillenträgers für die Berechnung oder Optimierung eines Brillenglases für das zumindest eine Auge des Brillenträgers bereitgestellt, wobei das zumindest eine Auge des Brillenträgers eine implantierte Intraokularlinse aufweist, umfassend die Schritte:
- Bereitstellen von Intraokularlinsendaten der in das Auge des Brillenträgers implantierten Intraokularlinse;
- Bereitstellen von individuellen Refraktionsdaten des zumindest einen Auges des Brillenträgers; und
- Festlegen eines individuellen Augenmodells, in welchem insbesondere zumindest

-- eine Form und/oder Wirkung einer Hornhaut, insbesondere einer Hornhautvorderfläche, eines Modellauges;
-- ein Hornhaut-Linsen-Abstand;
-- Parameter der Linse des Modellauges; und
-- ein Linsen-Netzhaut-Abstand
derart anhand der bereitgestellten Intraokularlinsendaten und ferner anhand von individuellen Messwerten für das Auge des Brillenträgers und/oder von Standardwerten und/oder anhand der bereitgestellten individuellen Refraktionsdaten festgelegt werden, dass das Modellauge die bereitgestellten individuellen Refraktionsdaten aufweist, wobei das Festlegen der Parameter der Linse des Modellauges anhand der bereitgestellten Intraokularlinsendaten erfolgt. Vorzugsweise erfolgt zumindest das Festlegen des Linsen-Netzhaut-Abstands durch Berechnen.

Das Brillenglas kann nach einem der in WO 2013/104548 A1 oder DE 10 2017 007 974 A1 beschrieben Verfahren durch Durchrechnung in das Auge hinein optimiert werden. Das dafür notwendige Augenmodell wird wie in DE 10 2017 007 975 A1 bzw. WO 2018/138140 A2 beschrieben mit individuellen Werten belegt, wobei für die Linse des Augenmodells die bekannten Eigenschaften der IOL, z.B. aus den Angaben des Herstellers, eingehen. Die Intraokularlinsendaten umfassen Daten mit Eigenschaften der implantieren Intraokularlinse. Diese können bei der Bestellung direkt angegeben werden oder mit Hilfe der Angabe des Typs oder der individuellen Seriennummer aus einer Datenbank bezogen werden. Dies ist hilfreich bzw. vorteilhaft, da die in DE 10 2017 007 975 A1 bzw. WO 2018/138140 A2 verwendeten modellbasierten Werte nicht notwendigerweise im Einklang mit den Eigenschaften der tatsächlich implantierten Linse stehen. Dies kann beispielsweise der Fall sein, wenn eine Längenmyopie durch eine schwächer brechende IOL zumindest teilweise kompensiert wird. In diesem Fall würde das in DE 10 2017 007 975 A1 bzw. WO 2018/138140 A2 beschriebene Vorgehen eine zu kurze Augenlänge liefern.

Das Augenmodell umfasst vorzugsweise verschiedene Komponenten wie z.B. Hornhaut, Linse, Netzhaut etc. und Parameter bzw. einen Parametersatz dieser Komponenten. Parameter des Augenmodells sind also z.B. die Form einer Hornhautvorderfläche des Modellauges, der Hornhaut-Linsen-Abstand, die Parameter der Linse des Modellauges, der Linsen-Netzhaut-Abstand, etc.

In einer bevorzugten Ausführungsform wird mit einem der in DE 10 2017 007 975 A1 bzw. WO 2018/138140 A2 beschrieben Formalismen aus dem Defokusterm des Gesamtauges, dem Defokusterm der Hornhautoberfläche, dem Abstand Hornhaut-Linse (im Folgenden DCL oder d_{CL}) und den Daten der IOL der Abstand Linse-Netzhaut (im folgenden DLR oder d_{LR}) berechnet. Der Begriff Defokusterm wird im Folgenden für den Wert des symmetrischen zweiten Terms (c_{2,0}) der Zerlegung nach Zernike der brechenden Wirkung bzw. der Oberfläche eines optischen Elements benutzt.

Insbesondere können einzelne oder mehrere folgender Daten verwendet werden:
- Daten der IOL: Hierbei kann es sich entweder um den Defokus der brechenden Flächen (Vorder- und Rückfläche) und einer Propagationslänge (Dicke der Linse, im Folgenden DLL) oder um den Defokus des Brechwerts der IOL handeln. Während ein Modell auf Basis der Flächen und des Abstandes bei der Optimierung präzisiere Ergebnisse liefern kann, erfordert es bei der Optimierung mehr Rechenschritte (Refraktion-Propagation-Refraktion statt nur Refraktion) und entsprechend detaillierte Informationen über die IOL, die eventuell nicht vorliegen.
- Defokusterm des Gesamtauges: Hier kann das Ergebnis einer aberrometrischen Messung oder einer Autorefraktion, einer subjektiven Refraktion oder einer anderen Ermittlung (z.B. Skiaskopie) verwendet werden. Alternativ kann auch eine sogenannte "optimierte Refraktion", also das Ergebnis einer Berechnung aus mehreren Komponenten (z.B. subjektive Refraktion und Aberrometrie) eingesetzt werden. Beispiele für eine derartige Optimierung sind in DE 10 2017 007 975 A1 bzw. WO 2018/138140 A2 zusammengestellt. Insbesondere umfassen die individuellen Refraktionsdaten des zumindest einen Auges des Brillenträgers einen Defokus bzw. Defokusterm des (Gesamt-)Auges.
- Defokusterm der Hornhaut: Dieser kann z.B. einer Topographie- oder Topometriemessung entnommen oder modellbasiert angenommen werden.
- Abstand Hornhaut-Linse: Dieser kann mit einer Messung (z.B. Scheimpflugaufnahme oder OCT) bestimmt oder modellbasiert angenommen werden.

An Stelle des Defokus oder zusätzlich dazu kann auch eine andere Größe, vorzugsweise ein Term zweiter Ordnung wie z.B. die Wirkung in dem Hauptschnitt mit dem höchsten oder niedrigsten Brechwert oder in einem Meridian mit definierter Lage (z.B. horizontal oder vertikal) verwendet werden.

Je nach Bedarf kann das Augenmodell um den Astigmatismus (Betrag und Achse, bzw. der anderen Größen zweiter Ordnung gemäß vorigem Absatz) sowie um Komponenten höherer Ordnung (siehe DE 10 2017 007 975 A1 bzw. WO 2018/138140 A2) des Gesamtauges und der Komponenten ergänzt werden. Diese können beispielsweise aus den Daten der IOL, Messungen (z.B. Topographie/Topometrie bzw. Aberrometrie/Autorefraktion), Modellannahmen und/oder berechneten Werten (z.B. optimierte Refraktion) entnommen werden.

Oft werden für IOLs Angaben zur Asphärizität oder Aberrationen höherer Ordnung gemacht. Diese können bei der Belegung des Augenmodells mit Aberrationen höherer Ordnung verwendet werden.

Zunächst werden individuelle Refraktionsdaten des zumindest einen Auges des Brillenträgers bereitgestellt. Diese individuellen Refraktionsdaten basieren auf einer individuellen Refraktionsbestimmung. Die Refraktionsdaten umfassen dabei zumindest die sphärische und astigmatische Fehlsichtigkeit des Auges. In einer bevorzugten Ausführungsform beschreiben die erfassten Refraktionsdaten auch Abbildungsfehler höherer Ordnung (HOA). Vorzugsweise werden die Refraktionsdaten (insbesondere soweit sie Abbildungsfehler höherer Ordnung umfassen auch aberrometrische Daten genannt) beispielsweise vom Augenoptiker mittels eines Autorefraktometers oder eines Aberrometers gemessen (objektive Refraktionsdaten). Alternativ oder zusätzlich kann auch eine subjektiv bestimmte Refraktion herangezogen werden. Die Refraktionsdaten werden anschließend vorzugsweise an einen Brillenglashersteller übermittelt und/oder einem Berechnungs- bzw. Optimierungsprogramm zur Verfügung gestellt. Sie stehen damit zur Verfügung, um für das erfindungsgemäße Verfahren erfasst, insbesondere in digitaler Form ausgelesen und/oder empfangen zu werden.

Vorzugsweise umfasst das Bereitstellen der individuellen Refraktionsdaten ein Bereitstellen bzw. Ermitteln der Vergenzmatrix **S***_{M}* der Fehlsichtigkeit des zumindest einen Auges. Dabei beschreibt die Vergenzmatrix eine Wellenfront vor dem Auge des von einem Punkt auf der Netzhaut auslaufenden bzw. in einem Punkt auf der Netzhaut konvergierenden Lichts. Messtechnisch können solche Refraktionsdaten beispielsweise dadurch ermittelt werden, dass mittels eines Lasers ein Punkt auf der Netzhaut des Brillenträgers beleuchtet wird, von welchem aus sich dann Licht ausbreitet. Während das Licht von dem beleuchteten Punkt aus zunächst im Glaskörper des Auges im Wesentlichen kugelförmig divergiert, kann sich die Wellenfront beim Durchlaufen des Auges insbesondere an optischen Grenzflächen im Auge (z.B. der Augenlinse und/oder der Hornhaut) verändern. Durch eine Messung der Wellenfront vor dem Auge sind somit die Refraktionsdaten des Auges messbar.

Außerdem umfasst das Verfahren gemäß dem ersten Aspekt der Erfindung ein Festlegen eines individuellen Augenmodells, welches zumindest gewisse Vorgaben über geometrische und optische Eigenschaften eines Modellauges individuell festlegt. So werden in dem erfindungsgemäßen individuellen Augenmodell zumindest eine Form (Topographie) und/oder Wirkung der Hornhaut, insbesondere einer Hornhautvorderfläche, des Modellauges, ein Hornhaut-Linsen-Abstand *d_{CL}* (dieser Abstand zwischen der Hornhaut und einer Linsenvorderfläche des Modellauges wird auch Vorderkammertiefe bezeichnet), Parameter der Linse des Modellauges, welche insbesondere die optische Wirkung der Linse des Modellauges zumindest teilweise festlegen, und ein Linsen-Netzhaut-Abstand *d_{LR}* (dieser Abstand zwischen der Linse, insbesondere der Linsenrückfläche, und der Netzhaut des Modellauges wird auch als Glaskörperlänge bezeichnet) in bestimmter Weise, nämlich derart festgelegt, dass das Modellauge die bereitgestellten individuellen Refraktionsdaten aufweist, d.h. dass eine im Modellauge von einem Punkt der Netzhaut des Modellauges auslaufende Wellenfront mit der für das reale Auge des Brillenträger ermittelte (z.B. gemessene oder anderweitig ermittelte) Wellenfront (bis zu einer gewünschten Genauigkeit) übereinstimmt. Als Parameter der Linse des Modellauges (Linsenparameter) können beispielsweise entweder geometrische Parameter (Form der Linsenflächen und deren Abstand) und vorzugsweise Materialparameter (z.B. Brechungsindizes der einzelnen Komponenten des Modellauges) so vollständig festgelegt werden, dass diese eine optische Wirkung der Linse zumindest teilweise festlegen. Alternativ oder zusätzlich können als Linsenparameter auch Parameter festgelegt werden, die die optische Wirkung der Linse des Modellauges direkt beschreiben. Hinsichtlich der Hornhaut wird meistens die Form der Hornhautvorderfläche gemessen, alternativ oder zusätzlich kann aber auch die Wirkung der Hornhaut als Ganzes (keine Differenzierung zwischen Vorder- und Rückfläche) angegeben werden. Eventuell kann auch eine Hornhautrückfläche und/oder eine Hornhautdicke angegeben werden.

Sind individuelle Intraokularlinsendaten bekannt bzw. werden diese bereitgestellt, so kann das Festlegen der Parameter der Linse des Modellauges (ausschließlich) anhand der bereitgestellten Intraokularlinsendaten erfolgen. Insbesondere können die Parameter der Linse des Modellauges den bereitgestellten individuellen Intraokularlinsendaten entsprechen. Mit anderen Worten können die bereitgestellten Intraokularlinsendaten als die Parameter der Linse des Modellauges festgelegt werden.

So wird im einfachsten Fall eines Augenmodells die Refraktion des Auges durch das optische System bestehend aus der Hornhautvorderfläche, der Augenlinse und der Netzhaut bestimmt. In diesem einfachen Modell legen die Lichtbrechung an der Hornhautvorderfläche und die Brechkraft der Augenlinse (vorzugsweise einschließlich der sphärischen und astigmatischen Aberrationen und Aberrationen höherer Ordnung) zusammen mit deren Positionierung relativ zur Netzhaut die Refraktion des Modellauges fest.

Dabei werden die einzelnen Größen (Parameter) des Modellauges anhand der bereitgestellten Intraokularlinsendaten und ferner anhand von individuellen Messwerten für das Auge des Brillenträgers und/oder anhand von Standardwerten und/oder anhand der bereitgestellten individuellen Refraktionsdaten entsprechend festgelegt. Insbesondere können manche der Parameter (z.B. die Topographie der Hornhautvorderfläche und/oder die Vorderkammertiefe und/oder zumindest eine Krümmung einer Linsenfläche, usw.) direkt als individuelle Messwerte bereitgestellt werden. Andere Werte können auch - insbesondere dann, wenn es sich um Parameter handelt, deren individuelle Messung sehr aufwendig ist - aus Werten von Standardmodellen für ein menschliches Auge übernommen werden. Insgesamt müssen aber nicht alle (geometrischen) Parameter des Modellauges aus individuellen Messungen oder aus Standardmodellen vorgegeben werden. Vielmehr wird im Rahmen der Erfindung für einen oder mehrere (freie) Parameter eine individuelle Anpassung durch Berechnung unter Berücksichtigung der vorgegebenen Parameter derart vorgenommen, dass das dann resultierende Modellauge die bereitgestellten individuellen Refraktionsdaten aufweist. Je nach Anzahl der in den bereitgestellten individuellen Refraktionsdaten enthaltenen Parameter können entsprechend viele (freie) Parameter des Augenmodells individuell angepasst (gefittet) werden. Abweichend von einem beispielsweise in WO 2013/104548 A1 vorgeschlagenen Modell, kann im Rahmen der vorliegenden Erfindung dabei zumindest der Linsen-Netzhaut-Abstand durch Berechnen festgelegt werden.

Für die Berechnung bzw. Optimierung des Brillenglases werden eine erste Fläche und eine zweite Fläche des Brillenglases insbesondere als Startflächen mit einer vorgegebenen (individuelle) Position relativ zum Modellauge vorgegeben. In einer bevorzugten Ausführungsform wird nur eine der beiden Flächen optimiert.

Vorzugsweise handelt es sich hierbei um die Rückfläche des Brillenglases. Vorzugsweise wird dabei sowohl für die Vorderfläche als auch für die Rückfläche des Brillenglases eine entsprechende Startfläche vorgegeben. In einer bevorzugten Ausführungsform wird während des Optimierungsverfahrens aber nur eine Fläche iterativ verändert bzw. optimiert. Die andere Fläche des Brillenglases kann zum Beispiel eine einfache sphärische oder rotationssymmetrische asphärische Fläche sein. Allerdings ist es auch möglich, beide Flächen zu optimieren.

Ausgehend von den beiden vorgegebenen Flächen umfasst das Verfahren zum Berechnen oder Optimieren ein Ermitteln des Verlaufs eines Hauptstrahls durch zumindest einen Durchblickspunkt (i) zumindest einer zu berechnenden oder optimierenden Fläche des Brillenglases in das Modellauge. Der Hauptstrahl beschreibt den geometrischen Strahlverlauf ausgehend von einem Objektpunkt durch die beiden Brillenglasflächen, die Hornhautvorderfläche und die Linse des Modellauges vorzugsweise bis zur Netzhaut des Modellauges.

Außerdem umfasst das Verfahren zum Berechnen oder Optimieren gemäß diesem Aspekts der Erfindung ein Auswerten einer Aberration einer entlang des Hauptstrahls aus einer auf die erste Fläche des Brillenglases auftreffenden sphärischen Wellenfront resultierenden Wellenfront an einer Bewertungsfläche insbesondere vor oder innerhalb des Modellauges im Vergleich zu einer in einem Punkt auf der Netzhaut des Augenmodells konvergierenden Wellenfront (Referenzlicht).

Insbesondere wird dazu eine auf die erste Fläche (Vorderfläche) des Brillenglases entlang des Hauptstrahls auftreffende, sphärische Wellenfront (*w₀*) vorgegeben. Diese sphärische Wellenfront beschreibt das von einem Objektpunkt ausgehende Licht (Objektlicht). Die Krümmung der sphärischen Wellenfront beim Auftreffen auf die erste Fläche des Brillenglases entspricht dem Kehrwert des Objektabstandes. Vorzugsweise umfasst das Verfahren somit ein Vorgeben eines Objektabstandsmodells, welches jeder Blickrichtung oder jedem Durchblickspunkt der zumindest einen zu optimierenden Fläche des Brillenglases eine Objektentfernung zuordnet. Damit wird vorzugsweise die individuelle Gebrauchssituation, in der das herzustellende Brillenglas zum Einsatz kommen soll, beschrieben.

Die auf das Brillenglas auftreffende Wellenfront wird nun an der Vorderfläche des Brillenglases vorzugsweise zum ersten Mal gebrochen. Anschießend propagiert die Wellenfront entlang des Hauptstrahls innerhalb des Brillenglases von der Vorderfläche zur Rückfläche, wo sie zum zweiten Mal gebrochen wird. Vorzugsweise propagiert die durch das Brillenglas transmittierte Wellenfront nun entlang des Hauptstrahls weiter bis zur Hornhautvorderfläche des Auges, wo sie vorzugsweise wiederum gebrochen wird. Vorzugsweise wird die Wellenfront nach einer weiteren Propagation innerhalb des Auges bis zur Augenlinse auch dort wiederum gebrochen, um schließlich vorzugsweise bis zur Netzhaut des Auges zu propagieren. Je nach optischen Eigenschaften der einzelnen optischen Elemente (Brillenglasflächen, Hornhautvorderfläche, Augenlinse) führt jeder Brechungsvorgang auch zu einer Deformation der Wellenfront.

Um eine exakte Abbildung des Objektpunktes auf einen Bildpunkt auf der Netzhaut zu erreichen, müsste die Wellenfront die Augenlinse vorzugsweise als konvergierende sphärische Wellenfront verlassen, deren Krümmung genau dem Kehrwert des Abstandes zur Netzhaut entspricht. Ein Vergleich der vom Objektpunkt auslaufenden Wellenfront mit einer (im Idealfall einer perfekten Abbildung) in einem Punkt auf der Netzhaut konvergierenden Wellenfront (Referenzlicht) erlaubt somit die Auswertung einer Fehlanpassung. Dieser Vergleich und damit die Auswertung der Wellenfront des Objektlichts in dem individuellen Augenmodell können dabei an unterschiedlichen Stellen entlang des Verlaufs des Hauptstrahls insbesondere zwischen der zweiten Fläche des optimierenden Brillenglases und der Netzhaut erfolgen. Insbesondere kann damit die Bewertungsfläche an unterschiedlichen Positionen, insbesondere zwischen der zweiten Fläche des Brillenglases und der Netzhaut liegen. Entsprechend weit wird die Brechung und Propagation des vom Objektpunkt auslaufenden Lichts im individuellen Augenmodell vorzugsweise für jeden Durchblickspunkt berechnet. Die Bewertungsfläche kann sich entweder auf den tatsächlichen Strahlengang beziehen oder auf einen virtuellen Strahlengang, wie er beispielsweise zur Konstruktion der Austrittspupille AP benutzt wird. Im Fall des virtuellen Strahlenganges muss das Licht nach der Brechung durch die Rückfläche der Augenlinse zurückpropagiert werden bis zu einer gewünschten Ebene (bevorzugt bis zur Ebene der AP), wobei der dabei benutzte Brechungsindex dem Medium des Glaskörpers entsprechen muss und nicht etwa der Augenlinse. Falls die Bewertungsfläche hinter der Linse bzw. nach der Brechung an der Linsenrückfläche des Modellauges vorgesehen wird, oder falls die Bewertungsfläche durch Rückpropagation entlang eines virtuellen Strahlenganges erreicht wird (wie im Fall der AP), dann kann die resultierende Wellenfront des Objektlichts vorzugsweise einfach mit einer sphärischen Wellenfront des Referenzlichts verglichen werden. Hierzu umfasst das Verfahren somit vorzugsweise ein Vorgeben einer auf die erste Fläche des Brillenglases auftreffenden sphärischen Wellenfront, ein Ermitteln einer durch die Wirkung zumindest der ersten und zweiten Fläche des Brillenglases, der Hornhautvorderfläche und der Linse des Modellauges aus der sphärischen Wellenfront resultierenden Wellenfront in dem zumindest einen Auge, und eine Auswertung der Aberration der resultierenden Wellenfront im Vergleich zu einer auf die Netzhaut konvergierenden sphärischen Wellenfront.

Falls hingegen eine Bewertungsfläche innerhalb der Linse oder zwischen der Linse des Modellauges und dem zu berechnenden bzw. optimierenden Brillenglas vorgesehen sein soll, wird als Referenzlicht einfach eine umgekehrte Propagation von einem Punkt auf der Netzhaut durch die einzelnen Komponenten des Modellauges bis hin zur Bewertungsfläche simuliert, um dort einen Vergleich des Objektlichts mit dem Referenzlicht vorzunehmen.

Wie allerdings bereits eingangs erwähnt, ist eine vollständige Korrektion der Refraktion des Auges gleichzeitig für alle Blickrichtungen des Auges, also für alle Durchblickspunkte der zumindest einen zu optimierenden Brillenglasfläche, im Allgemeinen nicht möglich. Je nach Blickrichtung wird somit vorzugsweise eine absichtliche Fehlanpassung des Brillenglases vorgegeben, welche je nach Anwendungssituation insbesondere in den hauptsächlich genutzten Bereichen des Brillenglases (z.B. zentrale Durchblickspunkte) gering, in den wenig genutzten Bereichen (z.B. periphere Durchblickspunkte) etwas höher sind. Diese Vorgehensweise ist dem Prinzip nach aus herkömmlichen Optimierungsverfahren bereits bekannt.

Um das Brillenglas zu optimieren, wird nun die zumindest eine zu berechnende oder optimierende Fläche des Brillenglases solange iterativ variiert, bis eine Aberration der resultierenden Wellenfront einer vorgegebenen Sollaberration entspricht, also insbesondere um vorgegebene Werte der Aberration von der Wellenfront des Referenzlichts (z.B. einer sphärischen Wellenfront, deren Krümmungsmittelpunkt auf der Netzhaut liegt) abweicht. Die Wellenfront des Referenzlichts wird hier auch als Referenzwellenfront bezeichnet. Vorzugsweise umfasst das Verfahren dazu ein Minimieren einer Zielfunktion F, insbesondere analog zu der eingangs bereits beschriebenen Zielfunktion. Weitere bevorzugte Zielfunktionen, insbesondere bei Berücksichtigung von Abbildungsfehlern höherer Ordnung sind auch weiter unten noch beschrieben. Falls eine Propagation des Objektlichts bis zur Netzhaut berechnet wird, kann eine Auswertung dort anstelle eines Vergleichs von Wellenfront-Parametern beispielsweise mittels einer sogenannten "point spread function" vorgenommen werden.

Im Rahmen der vorliegenden Erfindung wird somit vorgeschlagen, insbesondere für die Berechnung bzw. Optimierung eines Brillenglases ein derart individuelles Augenmodell festzulegen, welches bis zur Netzhaut dadurch individuell an den einzelnen Brillenträger angepasst ist, dass zumindest die Glaskörperlänge des Modellauges in Abhängigkeit von anderen individuell ermittelten insbesondere gemessenen Daten des Auges individuell berechnet wird. Dieser Parameter muss damit weder a-prior festgelegt werden, noch muss er direkt gemessen werden. Im Rahmen der vorliegenden Erfindung stellte sich heraus, dass dies deshalb eine bemerkenswerte Verbesserung der individuellen Anpassung bei vergleichsweise geringem Aufwand brachte, weil sich die Wellenfrontdurchrechnung als sehr empfindlich von diesem Längenparameter abhängig herausstellte.

Die individuelle Berechnung des Augenmodells, insbesondere des Linse-Netzhautabstandes (Glaskörperlänge), kann dabei beispielsweise bereits in einem Aberrometer oder einem Topografen mit entsprechend erweiterter Funktionalität vorgenommen werden. Vorzugsweise erfolgt dabei eine individuelle Ermittlung einer Augenlänge. Besonders bevorzugt wird dem Benutzer die gemessene und/oder berechnete Glaskörperlänge und/oder die ermittelte (gemessene und/oder berechnete) Augenlänge angezeigt. Hierzu weist eine entsprechende Vorrichtung (insbesondere ein Aberrometer bzw. Topograf) eine entsprechende Anzeigeeinrichtung auf.

Insbesondere können im Rahmen der vorliegenden Erfindung, sofern verfügbar, bekannte Eigenschaften der implantierten IOLs bei der Berechnung von Brillengläsern verwendet werden. Dies resultiert vorteilhafterweise in ein für Patienten mit einer implantierten IOL besser angepasstes Brillenglas mit optimierter Abbildung und Designhaltigkeit auf der Netzhaut.

In einer bevorzugten Ausführungsform umfassen die Intraokularlinsendaten zumindest einen Defokus der Vorderfläche der Intraokularlinse, einen Defokus der Rückfläche der Intraokularlinse und eine Dicke der Intraokularlinse. Alternativ oder zusätzlich umfassen die Intraokularlinsendaten zumindest einen Defokus des Brechwerts der Intraokularlinse bzw. eine optische Wirkung der Intraokularlinse. Bei den Intraokularlinsendaten kann es sich also entweder um den Defokus der brechenden Flächen (Vorder- und Rückfläche) und einer Propagationslänge (Dicke der Linse, im Folgenden DLL) oder um den Defokus des Brechwerts der IOL handeln. Während ein Modell auf Basis der Flächen und des Abstandes bei der Optimierung präzisere Ergebnisse liefern kann, erfordert es bei der Optimierung mehr Rechenschritte (Refraktion-Propagation-Refraktion statt nur Refraktion) und entsprechend detaillierte Informationen über die IOL, die eventuell nicht vorliegen. Alternativ oder zusätzlich können die Intraokularlinsendaten eine Angabe, insbesondere einen Wert, zu einer sogenannten A-Konstante umfassen. Bei der A-Konstante handelt es sich um eine individuelle Linsenkonstante, insbesondere um eine Art Korrekturfaktor, die in IOL-Berechnungsformeln mit unterschiedlicher Bezeichnung auftreten kann. Sie wird auch als IOL-Konstante oder "surgeon factor" bezeichnet. Jede IOL von jedem Hersteller hat eine andere A-Konstante, welche für jede Berechnungsformel angegeben wird.

Durch diese Konstante wird die Intraokularlinse in den verschiedenen Berechnungsformeln repräsentiert. Da alle IOL-Konstanten ineinander umgeformt werden können, gibt es im Prinzip nur eine einzige Konstante (Zahl), die eine gegebene Intraokularlinse im gesamten lieferbaren Stärkenbereich charakterisieren soll, unabhängig von Formfaktor, Optikmaterial, IOL-Durchmesser, etc. Durch die Verwendung von A- bzw. IOL-Konstanten werden die Einflüsse individueller chirurgischer Technik, verwendeter Mess- und Operationsausrüstungen sowie individuelle physiologische Unterschiede der operierten Patientenkohorte auf die IOL-Berechnung minimiert. Die A-Konstante gibt insbesondere eventuelle Anpassungen bei der Stärke wieder und kann Teil des Linsenpasses bzw. IOL-Passes sein.

In einer weiteren bevorzugten Ausführungsform werden die (individuellen) Intraokularlinsendaten auf Basis von Typ- oder Seriennummer-Informationen, insbesondere durch die Hersteller der IOL, bereitgestellt. Diese Informationen können z.B. direkt bei der Bestellung angegeben oder aus einer Datenbank bezogen werden.

In einer weiteren bevorzugten Ausführungsform umfasst das Verfahren ferner die Schritte:
- Durchführen einer Konsistenzprüfung des festgelegten Augenmodells, und
- Auflösen etwaiger Inkonsistenzen, insbesondere mit Hilfe von analytischen und/oder numerischen und/oder probabilistischen Verfahren.

Das erfindungsgemäße Vorgehen liefert in jedem Fall ein konsistentes Modell bezüglich des Defokus (bzw. der anderen, bei der Berechnung der Augenlänge benutzten Größen). Bereits bei weiteren Größen zweiter Ordnung (z.B. Betrag und Richtung des Astigmatismus) ist die Konsistenz des Modells jedoch nicht mehr sichergestellt. Mit anderen Worten kann das Augenmodell überbestimmt und folglich nicht mehr konsistent sein. Dies kann zum einen an Fertigungsungenauigkeiten der IOL und Messungenauigkeiten liegen, die z.B. bei der Topographie bzw. Topometrie, der Aberrometrie bzw. Autorefraktion und/oder der Messung der Vorderkammertiefe auftreten können. Zum anderen können sich bei Verwendung der subjektiven Refraktion prinzipiell Inkonsistenzen ergeben, wenn die subjektive oder optimierte Refraktion nicht der objektiven optischen Wirkung des Gesamtauges entspricht. Unter einem konsistenten Augenmodell wird im Rahmen dieser Beschreibung ein Augenmodell verstanden, bei dem eine einfallende Wellenfront, die den Aberrationen des Gesamtauges entspricht, auf der Netzhaut in einem Punkt zusammenläuft. Dies ist geleichbedeutend damit, dass die Wellenfront, die von einem Lichtpunkt auf der Netzhaut ausgeht, nach dem Durchlaufen des gesamten Auges den Aberrationen des Gesamtauges entspricht.

Eine Konsistenzprüfung kann insbesondere mittels probabilistischer Verfahren durchgeführt werden. In diesem Fall könnte ein Konsistenzmaß als eine Wahrscheinlichkeit angegeben werden. Das Auflösen etwaiger Inkonsistenzen könnte z.B. durch das Bestimmen eines Maximums der Wahrscheinlichkeit erfolgen.

Wurde beispielsweise eine phake Intraokularlinse, also eine zu der vorhandenen natürlichen Augenlinse zusätzliche künstliche Linse, in das zumindest eine Auge des Brillenträgers implantiert, so kann das Auflösen etwaiger Inkonsistenzen insbesondere ein Anpassen eines oder mehrerer, auf Grund der zusätzlichen Linse abgeänderter oder hinzugefügter, Parameter des Augenmodells (bzw. Nach-OP-Augenmodells) umfassen, sofern dies zum Erreichen der Konsistenz des Augenmodells (bzw. Nach-OP-Augenmodells) hilfreich oder notwendig ist.

Das Durchführen einer Konsistenzprüfung und die Auflösung etwaiger Inkonsistenzen verbessert insbesondere die Berechnung bzw. Optimierung von Brillengläsern, die für einen Patienten mit IOLs vorgesehen sind. Jedoch sind das Durchführen einer Konsistenzprüfung und die Auflösung etwaiger Inkonsistenzen auch bei Brillengläsern, die nicht speziell für einen Patienten mit IOLs vorgesehen sind, von Vorteil. Somit umfasst die vorliegende Erfindung allgemein ein computerimplementiertes Verfahren zum Ermitteln relevanter individueller Parameter zumindest eines Auges eines Brillenträgers für die Berechnung oder Optimierung eines Brillenglases für das zumindest eine Auge des Brillenträgers, umfassend die Schritte:
- Bereitstellen von individuellen Refraktionsdaten des zumindest einen Auges des Brillenträgers;
- Festlegen eines individuellen Augenmodells, in welchem insbesondere eine oder mehrere der folgenden Informationen bzw. Parameter, nämlich
   -- eine Form und/oder Wirkung einer Hornhaut, insbesondere einer Hornhautvorderfläche (18), eines Modellauges (12); und/oder
   -- ein Hornhaut-Linsen-Abstand; und/oder
   -- Parameter der Linse des Modellauges; und/oder
   -- ein Linsen-Netzhaut-Abstand; und/oder
   -- eine Größe der Eintrittspupille; und/oder
   -- eine Größe und/oder Position einer physikalischen Aperturblende (bzw. Irisöffnung)
   anhand von individuellen Messwerten für das Auge des Brillenträgers und/oder von Standardwerten und/oder anhand der bereitgestellten individuellen Refraktionsdaten festgelegt werden,
- Durchführen einer Konsistenzprüfung des festgelegten Augenmodells insbesondere mit den bereitgestellten individuellen Refraktionsdaten, und gegebenenfalls
- Auflösen etwaiger Inkonsistenzen, insbesondere mit Hilfe von analytischen und/oder numerischen und/oder probabilistischen Verfahren bzw. Methoden.

Enthält das Augenmodell Parameter der Cornea, so kann die Position und Größe der Aperturblende des Auges in die Position und Größe der Eintrittspupille umgerechnet werden und umgekehrt, da die Eintrittspupille die durch die Cornea abgebildete Aperturblende darstellt. Deshalb kann es insbesondere ausreichend sein, wenn in diesem Fall die Position bzw. die Größe von entweder der Aperturblende oder der Eintrittspupille als (ggf. zusätzlicher) Parameter des Augenmodells verwendet wird.

Das "Festlegen eines individuellen Augenmodells" kann ein Festlegen von Modellparametern auf bestimmte Werte bedeuten. Zusätzlich oder alternativ kann das "Festlegen eines individuellen Augenmodells" aber auch ein Festlegen zumindest eines Konsistenzmaßes (bzw. zumindest einer Wahrscheinlichkeit) umfassen. Insbesondere kann eine Vielzahl von Werten der Modellparameter existieren. Für jede Kombination dieser Werte kann ein Konsistenzmaß bzw. eine Wahrscheinlichkeit festgelegt werden. Beispielsweise kann das Festlegen von solchen Konsistenzmaßen bzw. Wahrscheinlichkeiten unter Verwendung des Bayes'schen Verfahrens erfolgen.

Vorzugsweise erfolgt zumindest das Festlegen des Linsen-Netzhaut-Abstands durch Berechnen. Dabei kann der Begriff "Berechnen" im Sinne der vorliegenden Erfindung nicht nur das Berechnen anhand einer Gleichung, sondern auch Schritte umfassen, die bei einem statistischen Verfahren durchgeführt werden, wie z.B. das Auswählen von Werten auf Basis von statistischen Betrachtungen bzw. Wahrscheinlichkeiten. Beim Bayes'schen Verfahren ist es z.B. möglich, dass lediglich ein wahrscheinlicher bzw. wahrscheinlichster Linsen-Netzhaut-Abstand ausgewählt wird oder durch ein Optimierungsproblem definiert wird (das dann noch zu lösen ist). Der Begriff "Berechnen" im Sinne der vorliegenden Erfindung kann somit insbesondere auch das Auswählen von wahrscheinlichen bzw. wahrscheinlichsten Werten eines oder mehrerer Parameter und/oder das Definieren eines Optimierungsproblems umfassen. Insbesondere umfasst der Begriff "Berechnen" auch ein Auswählen, Bestimmen und/oder Definieren im Rahmen eines statistischen Verfahrens, z.B. im Rahmen bzw. unter Verwendung des Bayes'schen Verfahrens. Der Begriff "Berechnen" kann insbesondere auch ein Optimieren umfassen.

Zusätzlich oder alternativ zum Durchführen einer Konsistenzprüfung des festgelegten Augenmodells und Auflösen etwaiger Inkonsistenzen kann das computerimplementierte Verfahren auch das Festlegen bzw. Konstruieren eines konsistenten Augenmodells umfassen, insbesondere unter Verwendung des Bayes'schen Verfahrens und/oder eines Maximum Likelihood Verfahrens. Mit anderen Worten ist das verwendete bzw. festzulegende individuelle Augenmodell ein konsistentes Augenmodell, wobei die Konsistenz durch statistische bzw. probabilistische Methoden, insbesondere unter Verwendung des Bayes'schen Verfahrens und/oder eines Maximum Likelihood Verfahrens, ermöglicht bzw. hergestellt wird.

Insbesondere wird in diesem Aspekt ein computerimplementiertes Verfahren und eine entsprechende Vorrichtung zum Ausführen eines solchen Verfahrens zum Ermitteln relevanter individueller Parameter zumindest eines Auges eines Brillenträgers für die Berechnung oder Optimierung eines Brillenglases für das zumindest eine Auge des Brillenträgers bereitgestellt, umfassend einen oder mehrere der folgenden Schritte bzw. Funktionen:
- Bereitstellen von individuellen Refraktionsdaten des zumindest einen Auges des Brillenträgers; und/oder
- Festlegen eines individuellen Augenmodells, in welchem insbesondere eine oder mehrere der folgenden Informationen bzw. Parameter, nämlich
   -- eine Form und/oder Wirkung einer Hornhaut, insbesondere einer Hornhautvorderfläche, eines Modellauges; und/oder
   -- ein Hornhaut-Linsen-Abstand; und/oder
   -- Parameter der Linse des Modellauges; und/oder
   -- ein Linsen-Netzhaut-Abstand; und/oder
   -- eine Größe der Eintrittspupille; und/oder
   -- eine Größe und/oder Position einer physikalischen Aperturblende (bzw. Irisöffnung)
insbesondere anhand von individuellen Messwerten für das Auge des Brillenträgers und/oder von Standardwerten und/oder anhand der bereitgestellten individuellen Refraktionsdaten festgelegt werden,
wobei eine(r) oder mehrere der Information(en) bzw. Parameter und/oder zumindest teilweise die bereitgestellten individuellen Refraktionsdaten zunächst in Form einer Wahrscheinlichkeitsverteilung festgelegt wird bzw. werden, und wobei das Festlegen des individuellen Augenmodells ein Ermitteln des Modellauges durch Ermitteln von Werten für Informationen bzw. Parameter innerhalb der festgelegten Wahrscheinlichkeitsverteilung durch ein probabilistisches Verfahren umfasst.

Während also in einigen Aspekten zunächst ein Modellauge durch Festlegung von Parameterwerten erstellt wird, um dann auf Basis einer Konsistenzprüfung mittels probabilistischen Verfahrens das Modellauge gegebenenfalls so zu modifizieren, dass das Augenmodell konsistent ist, wird im vorliegenden Fall anstelle von möglicherweise inkonsistenten Parameterwerten gleich mit einer Wahrscheinlichkeitsverteilung für zumindest einen Parameter begonnen, um dann mittels eines probabilistischen Verfahrens den wahrscheinlichsten Parameterwert und damit das wahrscheinlichste Modellauge konsistent zu ermitteln. Parameter der Wahrscheinlichkeitsverteilung(en), wie beispielsweise Mittelwerte und/oder Standardabweichungen, können insbesondere anhand von individuellen Messwerten für das Auge des Brillenträgers und/oder von Standardwerten und/oder anhand der bereitgestellten individuellen Refraktionsdaten festgelegt werden. Weitere Details und konkrete Ausführungsbeispiele solcher Verfahren sind weiter unten beschrieben.

Im Folgenden wird anhand einiger Beispiele beschrieben, wie etwaige Inkonsistenzen im Augenmodell durch Anpassung der Parameter mit Hilfe analytischer Berechnungen ausgeräumt werden können.

Die einfachste Möglichkeit besteht darin, die Abweichungen auf ein Element bzw. eine Komponente des Augenmodells zu übertragen (z.B. Hornhaut, Vorderfläche der IOL, Rückfläche der IOL, brechende Wirkung der IOL). Beispielsweise könnte die Rückfläche der IOL (entgegen den Angaben des Herstellers) so gewählt werden, dass das Modell konsistent wird. Dazu kann (bei Verwendung des Defokusterms) nach der Berechnung von DLR zuerst der Astigmatismus, insbesondere nach Betrag und Richtung (z.B. gemäß des in DE 10 2017 007 975 A1 bzw. WO 2018/138140 A2 beschriebenen Verfahrens) so festgelegt werden, dass das Augenmodell hinsichtlich des Astigmatismus konsistent wird. Weiterhin können z.B. in Folgeschritten Komponenten höherer Ordnung dieser Fläche (z.B. mit Hilfe des in DE 10 2017 007 975 A1 bzw. WO 2018/138140 A2 beschriebenen Verfahrens) so festgelegt werden, dass das Augenmodell auch in diesen Komponenten konsistent wird. Alternativ oder zusätzlich könnte die Hornhautfläche entsprechend angepasst werden. Dies bietet sich insbesondere an, wenn von der Hornhaut nur modellbasierte Informationen oder auf Grund von Topometriemessungen keine Informationen zum Astigmatismus oder Komponenten höherer Ordnung vorliegen.

In einer weiteren bevorzugten Ausführungsform erfolgt das Auflösen etwaiger Inkonsistenzen dadurch, dass ein oder mehrere Parameter des Augenmodells angepasst bzw. neu festgelegt werden. Vorzugsweise wird eine Anpassung von mehreren Parametern des Augenmodells vorgenommen und die Anpassung wird auf die mehreren Parameter des Augenmodells aufgeteilt. Beispielsweise können bekannte Abweichungen auf mehrere Elemente bzw. Komponenten und/oder mehrere Parameter des Augenmodells aufgeteilt werden, z.B. auf die Hornhaut, die Vorderfläche der IOL, die Rückfläche der IOL, und/oder die brechende Wirkung der IOL. Im einfachsten Fall können feste bzw. vorgegebene Faktoren oder Anteile angenommen werden, z.B. 33% auf die Hornhaut und 67% auf die Linse. Alternativ oder zusätzlich kann auch eine physiologisch begründete Verteilung benutzt werden.

Alternativ oder zusätzlich kann ein weiterer bzw. neuer Parameter zum Augenmodell hinzugefügt und derart festgelegt werden, dass das Augenmodell konsistent wird. Beispielsweise kann die Form einer Hornhautrückfläche eines Modellauges ein solch weiterer Parameter sein. Bei torischen Linsen mit festem Astigmatismus kann z.B. auch die Achslage und oder eine laterale Verschiebung oder Verkippung so gewählt werden, dass der resultierende Astigmatismus des Modellauges der Vorgabe (bestmöglich) entspricht.

Alternativ oder zusätzlich können die Längen DCL, DLL und/oder DLR angepasst werden. Gegebenenfalls kann auch die Wirkung des Gesamtauges angepasst werden. Hierbei kann die Zielwirkung des Brillenglases entsprechend geändert werden, um das Augenmodell konsistent zu machen.

In einer weiteren bevorzugten Ausführungsform werden die Parameter des Augenmodells mit Hilfe probabilistischer Verfahren, d.h. unter Verwendung von Wahrscheinlichkeitsrechnung, bestimmt. Hierzu kann insbesondere eine Bayes-Statistik und/oder ein Maximum-Likelihood Algorithmus verwendet werden.

Anstelle der oder zusätzlich zur analytischen Berechnung der Augenlänge auf Basis eines Parametersatzes können insbesondere alle bekannten Parameter (im weiteren Eingangsparameter) zusammengenommen und die Parameter des Augenmodels (im weiteren Ausgangsparameter) mit Hilfe statistischer Verfahren wie Maximum Likelihood und Bayes bestimmt werden. Dabei können ein oder mehrere der folgenden Informationen zumindest einzelner Eingangsparameter verwendet werden:
- Vertrauen in die Richtigkeit;
- Mess- bzw. Fertigungsgenauigkeit;
- Schwankungsbreite in einem Kollektiv bzw. Ensemble;
- Auswirkung auf die Optimierung des Brillenglases.

Eine Beschreibung zweier derartiger Verfahren sowie konkrete Beispiele sind weiter unten in der ausführlichen Beschreibung angeführt.

Erfindungsgemäß werden eine initiale Verteilung von Parametern des Augenmodells und individuelle Daten von Eigenschaften des zumindest einen Auges bereitgestellt, wobei die Parameter des individuellen Augenmodells auf Basis der initialen Verteilung von Parametern des Augenmodells und der individuellen Daten unter Verwendung von Wahrscheinlichkeitsrechnung bestimmt werden. Insbesondere werden ein initiales Augenmodell und individuelle Daten von Eigenschaften des zumindest einen Auges bereitgestellt, wobei die Parameter des individuellen Augenmodells auf Basis des initialen Augenmodells und der individuellen Daten unter Verwendung von Wahrscheinlichkeitsrechnung bestimmt werden.

In einer weiteren bevorzugten Ausführungsform wird eine Augenlänge des Modellauges unter Berücksichtigung des gemessenen und/oder berechneten Linsen-Netzhaut-Abstands ermittelt. Vorzugsweise wird die ermittelte Augenlänge auf einem Anzeigegerät bzw. Display angezeigt.

Das oben beschriebene Verfahren der vorliegenden Erfindung bezieht sich insbesondere auf den Fall, dass Eigenschaften bzw. Daten der implantierten Intraokularlinse, also die Intraokularlinsendaten, bekannt sind. Sind diese Daten jedoch nicht bekannt bzw. können keine individuellen Intraokularlinsendaten der in das zumindest eine Auge des Brillenträgers implantierten Intraokularlinse bereitgestellt werden, so wird im Rahmen dieser Erfindung vorgeschlagen, einen Linsen-Netzhaut-Abstand des Auges des Brillenträgers zu ermitteln, wobei das Festlegen der Parameter des individuellen Augenmodells derart anhand des ermittelten Linsen-Netzhaut-Abstands und ferner anhand von individuellen Messwerten für das Auge des Brillenträgers und/oder von Standardwerten und/oder anhand der bereitgestellten individuellen Refraktionsdaten erfolgt, dass das Modellauge die bereitgestellten individuellen Refraktionsdaten aufweist, wobei der Linsen-Netzhaut-Abstand des Modellauges durch den ermittelten Linsen-Netzhaut-Abstand des Auges des Brillenträgers festgelegt wird. Die bereitgestellten individuellen Refraktionsdaten des zumindest einen Auges des Brillenträgers sind dabei individuelle Nach-OP Refraktionsdaten des zumindest einen Auges des Brillenträgers. Und das individuelle Augenmodell ist folglich ein Nach-OP-Augenmodell. Gemäß dieses zusätzlichen oder alternativen Ansatzes der vorliegenden Erfindung, d.h. bei fehlender direkter Kenntnis von Eigenschaften der implantierten Linse, wird durch Messungen am Patienten auf die Eigenschaften der implantierten IOL(s) geschlossen.

Insbesondere betrifft somit ein zusätzlicher oder alternativer Ansatz zur Lösung der Aufgabe (nämlich für den Fall, dass Eigenschaften bzw. Daten der implantierten Intraokularlinse nicht bekannt sind) ein computerimplementiertes Verfahren zum Ermitteln relevanter individueller Parameter zumindest eines Auges eines Brillenträgers für die Berechnung oder Optimierung eines Brillenglases für das zumindest eine Auge des Brillenträgers, wobei im Rahmen einer Operation eine Intraokularlinse in das zumindest eine Auge des Brillenträgers implantiert wurde, umfassend die Schritte:
- Bereitstellen von individuellen Nach-OP-Refraktionsdaten des zumindest einen Auges des Brillenträgers;
- Ermitteln eines Linsen-Netzhaut-Abstands (bzw. einer Augenlänge) des Auges des Brillenträgers; und
- Festlegen eines individuellen Nach-OP-Augenmodells, in welchem insbesondere zumindest
   -- eine Form und/oder Wirkung einer Hornhaut, insbesondere einer Hornhautvorderfläche, eines Modellauges des Nach-OP-Augenmodells;
   -- ein Hornhaut-Linsen-Abstand des Modellauges des Nach-OP-Augenmodells;
   -- Parameter der Linse des Modellauges des Modellauges des Nach-OP-Augenmodells; und
   -- ein Linsen-Netzhaut-Abstand des Modellauges des Nach-OP-Augenmodells
derart anhand des ermittelten Linsen-Netzhaut-Abstands (bzw. der Augenlänge) und ferner anhand von individuellen Messwerten für das Auge des Brillenträgers (ermittelt vor oder nach der Operation) und/oder von Standardwerten und/oder anhand der bereitgestellten individuellen Nach-OP-Refraktionsdaten festgelegt werden, dass das Modellauge des Nach-OP-Augenmodells die bereitgestellten individuellen Nach-OP-Refraktionsdaten aufweist, wobei der Linsen-Netzhaut-Abstand des Modellauges des Nach-OP-Augenmodells durch den ermittelten Linsen-Netzhaut-Abstand des Auges des Brillenträgers festgelegt wird.

Der Begriff Operation wird allgemein mit OP abgekürzt. Der Begriff "Nach-OP" bezieht sich auf eine Situation nach der Operation, während sich der Begriff "Vor-OP" auf eine Situation vor der Operation bezieht. Beispielsweise ist die Operation eine Katarakt-Operation, bei der die natürliche Augenlinse durch eine Intraokularlinse ersetzt wird. Es kann sich jedoch auch um eine Operation eines aphaken Auges (Auge ohne Linse) handeln, bei der eine Intraokularlinse in das Auge des Patienten eingesetzt bzw. implantiert wird. Die Intraokularlinse kann also insbesondere einen Ersatz der natürlichen Augenlinse darstellen. Insbesondere wurde im Rahmen der Operation die natürliche Linse des Auges des Brillenträgers durch eine Intraokularlinse ersetzt. Es ist jedoch auch möglich, dass eine künstliche Intraokularlinse zusätzlich zur natürlichen Augenlinse in das zumindest eine Auge des Brillenträgers eingesetzt wird bzw. wurde. Der Begriff Operation umfasst somit auch ein Einsetzen bzw. eine Implantation einer künstlichen Linse zusätzlich zur natürlichen Augenlinse.

Das Brillenglas wird vorzugsweise nach einem der in WO 2013/104548 A1 oder DE 10 2017 007 974 A1 beschrieben Verfahren durch Durchrechnung in das Auge hinein optimiert. Das dafür notwendige Augenmodell wird analog zur Beschreibung in DE 10 2017 007 975 A1 bzw. WO 2018/138140 A2 mit individuellen Werten belegt. Allerdings sind in diesem Fall keine Informationen über die IOL verfügbar und modellbasierte Werte stehen nicht notwendigerweise im Einklang mit der tatsächlich implantierten Linse. Dies kann beispielsweise der Fall sein, wenn eine Längenmyopie durch eine schwächer brechende IOL zumindest teilweise kompensiert wird. In diesem Fall würde die Augenlänge nach dem in DE 10 2017 007 975 A1 bzw. WO 2018/138140 A2 beschriebenem Vorgehen zu kurz angenommen. Daher wird zunächst ausgehend von Daten, die einer Situation entsprechen, in der sich die ursprüngliche Linse im Auge befunden hat, die Augenlänge bzw. ein Linsen-Netzhaut-Abstand, wie in DE 10 2017 007 975 A1 bzw. WO 2018/138140 A2 beschrieben, berechnet.

Anschließend werden die weiteren Parameter (d.h. die Parameter der Augenlinse, in diesem Fall die implantierte IOL) ausgehend von der so berechneten Augenlänge (bzw. des berechneten Linsen-Netzhaut-Abstands) und den Nach-OP Werten für die Abbildungsfehler des Gesamtauges, die Oberfläche der Hornhaut und der Abstand Hornhaut-Linse derart bestimmt, dass die Wirkung dieses Augenmodells der Abbildungsfehler des Gesamtauges entspricht. Dieser unterscheidet sich zu dem Vorgehen in DE 10 2017 007 975 A1 bzw. WO 2018/138140 A2 fundamental dadurch, dass nun alle Werte der Linse (in diesem Fall die implantierte IOL) bestimmt werden und nicht wie in DE 10 2017 007 975 A1 bzw. WO 2018/138140 A2 ein Term zweiter Ordnung (z.B. Defokus) bereits bekannt ist. Nach der Bestimmung dieses Terms können aber weitere Terme wie in DE 10 2017 007 975 A1 bzw. WO 2018/138140 A2 beschrieben bestimmt werden. Dies können weitere Terme zweiter Ordnung und gegebenenfalls (z.B. in weiteren Schritten) Terme höherer Ordnung sein.

Für die Berechnung der Augenlänge bzw. des Linsen-Netzhaut-Abstands werden vorzugsweise konkret folgende Daten verwendet:
- Defokus des Gesamtauges vor dem Austausch der Linse: Dieser kann das Ergebnis einer aberrometrischen Messung oder einer Autorefraktion, einer subjektiven Refraktion oder einer anderen Ermittlung (z.B. Skiaskopie) vor dem Eingriff sein. Alternativ kann auch eine sogenannte "optimierte Refraktion", also das Ergebnis einer Berechnung aus mehreren Komponenten (z.B. subjektive Refraktion und Aberrometrie) verwendet werden. Beispiele für eine derartige Optimierung sind in DE 10 2017 007 975 A1 bzw. WO 2018/138140 A2 zusammengestellt. Weiterhin kann der Defokus der Brechkraft einer älteren, vor dem Eingriff getragenen Brille verwendet werden;
- Modellbasierte Werte für die Brechkraft oder den Aufbau der Augenlinse;
- Gemessene oder modellbasierte Werte für die Abstand Hornhaut-Linse; und
- Gemessene oder modellbasierte Werte für den Defokus der Hornhaut.

Die Daten der letzten beiden Punkte können wahlweise aus Messungen vor dem Eingriff (Operation) oder nach dem Eingriff stammen. Die Verwendung von nach dem Eingriff ermittelten Daten ist insbesondere dann sinnvoll, wenn vor dem Eingriff keine entsprechenden Messungen durchgeführt worden sind.

Für die Berechnung von Eigenschaften der Linse werden analog vorzugsweise folgende Daten verwendet:
- Abbildungsfehler des Gesamtauges nach dem Austausch der Linse: Diese können das Ergebnis einer aberrometrischen Messung oder einer Autorefraktion, einer subjektiven Refraktion oder einer anderen Ermittlung (z.B. Skiaskopie) nach dem Eingriff sein. Alternativ kann auch eine sogenannte "optimierte Refraktion", also das Ergebnis einer Berechnung aus mehreren Komponenten (z.B. subjektive Refraktion und Aberrometrie) verwendet werden. Beispiele für eine derartige Optimierung sind **in** DE 10 2017 007 975 A1 bzw. WO 2018/138140 A2 zusammengestellt.
- Der zuvor ermittelte Abstand Linse-Netzhaut;
- Gemessene oder modellbasierte Werte für den Abstand Hornhaut-Linse; und
- Gemessene oder modellbasierte Werte für die Abbildungsfehler der Hornhaut.

Die Daten der letzten beiden Punkte können wahlweise aus Messungen vor dem Eingriff (Operation) oder nach dem Eingriff stammen. Die Verwendung von vor dem Eingriff ermittelten Daten ist insbesondere dann sinnvoll, wenn nach dem Eingriff keine entsprechenden Messungen durchgeführt worden sind.

Das Bereitstellen von individuellen Intraokularlinsendaten kann insbesondere folgende Schritte umfassen:
- Ermitteln einer Augenlänge auf Basis von Daten, die einer Situation entsprechen, in der sich noch die ursprüngliche natürliche Linse im Auge des Brillenträgers befunden hat (Situation vor dem Implantieren der Intraokularlinse);
- Berechnen der individuellen Intraokularlinsendaten auf Basis der ermittelten Augenlänge, der bereitgestellten individuellen Refraktionsdaten, gemessener oder modellbasierter Werte für den Hornhaut-Linsen-Abstand und gemessener oder modellbasierter Werte für Abbildungsfehler der Hornhaut.

Die folgende Tabelle enthält beispielhaft drei Szenarien. Es versteht, dass jedoch auch andere Kombinationen Bestandteil der Erfindung sind.

| **Szenario** | **Werte für ersten Schritt (Berechnung von DCL)** | **Werte für zweiten Schritt (Belegung der noch offenen Parameter)** |
|---|---|---|
| | **Zu übertragende Daten** | |
| 1 | Nur Daten (Aberrometrie, Topographie, ggf. Abstand Hornhaut-Linse, ggf. subjektive Refraktion) von vor der OP (z.B. aus einer Bestellung) | Nur Daten (Aberrometrie, Topographie, ggf. Abstand Hornhaut-Linse, subjektive Refraktion) von nach der OP (z.B. aus einer Bestellung) |
| | Referenznummer der Bestellung vor der OP, Daten (Aberrometrie, Topographie, ggf. Abstand Hornhaut-Linse, subjektive Refraktion) von nach der OP (z.B. aus einer Bestellung) | |
| 2 | Nur subjektive Refraktion/Werte der Brille von vor der OP | Nur Daten (Aberrometrie, Topographie, ggf. Abstand Hornhaut-Linse, subjektive Refraktion) von nach der OP (z.B. aus einer Bestellung) |
| | Topographie und ggf. Abstand Hornhaut-Linse von vor der OP | |
| | Subjektive Refraktion/Werte der Brille von vor der OP sowie Daten (Aberrometrie, Topographie, ggf. Abstand Hornhaut-Linse, subjektive Refraktion) von nach der OP (z.B. aus einer Bestellung) | |
| 3 | Nur Daten (Aberrometrie, Topographie, ggf. Abstand Hornhaut-Linse, ggf. subjektive Refraktion) von vor der OP z.B. aus einer Bestellung) | Nur subjektive Refraktion von nach der OP, Weitere Daten (Topographie, ggf. Abstand Hornhaut-Linse) von vor der OP |
| | Referenznummer der Bestellung vor der OP, subjektive Refraktion von nach der OP | |

Das Ermitteln eines Linsen-Netzhaut-Abstands bzw. einer Augenlänge des Auges des Brillenträgers kann z.B. durch ein direktes Messen erfolgen.

In einer bevorzugten Ausführungsform umfasst das Verfahren ferner ein Bereitstellen von individuellen Vor-OP-Refraktionsdaten des zumindest einen Auges des Brillenträgers, wobei das Bestimmen eines Linsen-Netzhaut-Abstands bzw. einer Augenlänge des Auges des Brillenträgers auf Basis eines individuellen Vor-OP-Augenmodells unter Verwendung der bereitgestellten individuellen Vor-OP-Refraktionsdaten basiert.

In einer bevorzugten Ausführungsform werden in dem Vor-OP-Augenmodell insbesondere zumindest
-- eine Form und/oder Wirkung einer Hornhaut, insbesondere einer Hornhautvorderfläche, eines Modellauges des Vor-OP-Augenmodells;
-- ein Hornhaut-Linsen-Abstand des Modellauges des Vor-OP-Augenmodells;
-- Parameter der Linse des Modellauges des Vor-OP-Augenmodells; und
-- ein Linsen-Netzhaut-Abstand des Modellauges des Vor-OP-Augenmodells derart anhand von individuellen Messwerten für das Auge des Brillenträgers (ermittelt vor oder nach der OP) und/oder von Standardwerten und/oder anhand der bereitgestellten individuellen Vor-OP-Refraktionsdaten festgelegt, dass das Modellauge die bereitgestellten individuellen Vor-OP-Refraktionsdaten aufweist, wobei zumindest das Festlegen des Linsen-Netzhaut-Abstands durch Berechnen erfolgt.

Vorzugsweise wird dabei die Hornhautvorderfläche individuell gemessen und die Augenlinse des individuellen Vor-OP-Augenmodells entsprechend berechnet, um die individuell bestimmten Vor-OP-Refraktionsdaten zu erfüllen. Dabei wird in einer bevorzugten Ausführungsform die Hornhautvorderfläche (bzw. ihre Krümmung) entlang der Hauptschnitte individuell gemessen (Topometrie). In einer weiteren bevorzugten Ausführungsform wird die Topographie der Hornhautvorderfläche (d.h. die vollständige Beschreibung der Fläche) individuell gemessen. In einer weiteren bevorzugten Ausführungsform erfolgt das Festlegen des Hornhaut-Linsen-Abstandes anhand von individuellen Messwerten für den Hornhaut-Linsen-Abstand.

Besonders bevorzugt umfasst das Festlegen der Parameter der Linse des Vor-OP-Modellauges ein Festlegen folgender Parameter:
-- eine Form der Linsenvorderfläche;
-- eine Linsendicke; und
-- eine Form der Linsenrückfläche.

Auch wenn es für die Nutzung der Erfindung nicht essenziell ist, kann durch dieses genauere Modell der Linse die individuelle Anpassung nochmals verbessert werden.

In diesem Fall erfolgt in einer besonders bevorzugten Ausführungsform das Festlegen der Linsendicke und der Form der Linsenrückfläche anhand von vorgegebenen Werten (Standardwerten, beispielsweise aus der Fachliteratur), wobei weiter bevorzugt das Festlegen der Form der Linsenvorderfläche umfasst:
- Bereitstellen von Standardwerten für eine mittlere Krümmung der Linsenvorderfläche; und
- Berechnen der Form der Linsenvorderfläche unter Berücksichtigung der bereitgestellten individuellen Refraktionsdaten.

In einer weiteren bevorzugten Ausführungsform des detaillierteren Linsenmodells umfasst das Festlegen der Form der Linsenvorderfläche:
- Bereitstellen eines individuellen Messwerts einer Krümmung in einem Normalschnitt der Linsenvorderfläche.

In diesen Fall ist es besonders bevorzugt, wenn außerdem das Festlegen der Linsendicke und der Form der Linsenrückfläche anhand von Standardwerten erfolgt, und noch weiter bevorzugt das Festlegen der Form der Linsenvorderfläche umfasst:
- Berechnen der Form der Linsenvorderfläche unter Berücksichtigung der bereitgestellten individuellen Refraktionsdaten und des bereitgestellten individuellen Messwerts der Krümmung in einem Normalschnitt der Linsenvorderfläche.

Alternativ oder zusätzlich zur Form der Linse bzw. der Linsenflächen kann das Festlegen der Linsenparameter ein Festlegen einer optischen Wirkung der Linse umfassen. Insbesondere wird dabei zumindest eine Position zumindest einer Hauptebene und eine sphärische Wirkung (bzw. zumindest eine Brennweite) der Linse des Modellauges festgelegt. Besonders bevorzugt wird auch eine zylindrische Wirkung (Betrag und Achslage) der der Linse des Modellauges festgelegt. **In** einer weiteren bevorzugten Ausführungsform können auch optische Abbildungsfehler höherer Ordnungen der Linse des Modellauges festgelegt werden.

Gemäß des oben genannten ersten unabhängigen Aspekts zur Lösung der Aufgabe werden u.a. die folgenden Schritte durchgeführt:
- ein erfindungsgemäßes Verfahren zum Ermitteln relevanter individueller Parameter des zumindest einen Auges des Brillenträgers;
- Vorgeben einer ersten Fläche und einer zweiten Fläche für das zu berechnende bzw. optimierende Brillenglas;
- Ermitteln des Verlaufs eines Hauptstrahls durch zumindest einen Durchblickspunkt zumindest einer zu berechnenden oder optimierenden Fläche des Brillenglases in das Modellauge;
- Auswerten einer Aberration einer entlang des Hauptstrahls aus einer auf die erste Fläche des Brillenglases auftreffenden sphärischen Wellenfront resultierenden Wellenfront an einer Bewertungsfläche im Vergleich zu einer in einem Punkt auf der Netzhaut des Augenmodells konvergierenden Wellenfront;
- iteratives Variieren der zumindest einen zu berechnenden oder optimierenden Fläche des Brillenglases bis die ausgewertete Aberration einer vorgegebenen Sollaberration entspricht.

Vorzugsweise liegt die Bewertungsfläche zwischen der Hornhautvorderfläche und der Netzhaut. In einer besonders bevorzugten Ausführungsform liegt die Bewertungsfläche zwischen der Linse und der Netzhaut des Modellauges. **In** einer anderen besonders bevorzugten Ausführungsform liegt die Bewertungsfläche an der Austrittspupille (AP) des Modellauges. Dabei kann die Austrittspupille vor der Linsenrückfläche des Modellauges liegen. Bei diesen Positionierungen kann eine besonders genaue, individuelle Anpassung des Brillenglases erreicht werden.

Ein weiterer unabhängiger Aspekt zur Lösung der Aufgabe betrifft ein Verfahren zum Herstellen eines Brillenglases umfassend:
Berechnen oder Optimieren eines Brillenglases nach dem erfindungsgemäßen Verfahren zum Berechnen oder Optimieren eines Brillenglases; und
Fertigen des so berechneten oder optimierten Brillenglases.

Insbesondere umfasst eine Vorrichtung zum Ermitteln relevanter individueller Parameter zumindest eines Auges eines Brillenträgers für die Berechnung oder Optimierung eines Brillenglases für das zumindest eine Auge des Brillenträgers:
- zumindest eine Datenschnittstelle zum Bereitstellen von individuellen Refraktionsdaten des zumindest einen Auges des Brillenträgers; und
- ein Modellierungsmodul zum Festlegen eines individuellen Augenmodells, welches insbesondere zumindest
   -- eine Form und/oder Wirkung einer Hornhaut, insbesondere einer Hornhautvorderfläche, eines Modellauges;
   -- einen Hornhaut-Linsen-Abstand;
   -- Parameter der Linse des Modellauges; und
   -- einen Linsen-Netzhaut-Abstand
anhand von individuellen Messwerten für das Auge des Brillenträgers und/oder von Standardwerten und/oder anhand der bereitgestellten individuellen Refraktionsdaten festlegt, wobei zumindest das Festlegen des Linsen-Netzhaut-Abstands durch Berechnen erfolgt; und wobei
das Modellierungsmodul ausgelegt ist, eine Konsistenzprüfung des festgelegten Augenmodells mit den bereitgestellten individuellen Refraktionsdaten durchzuführen und etwaige Inkonsistenzen, insbesondere mit Hilfe von analytischen und/oder probabilistischen Verfahren, aufzulösen.

Ein weiterer unabhängiger Aspekt zur Lösung der Aufgabe betrifft eine Vorrichtung zum Berechnen oder Optimieren eines Brillenglases für zumindest ein Auge eines Brillenträgers zum Zweck der Herstellung des Brillenglases für das zumindest eine Auge, welches eine implantierte Intraokularlinse aufweist, umfassend:
- eine Vorrichtung zum Ermitteln relevanter individueller Parameter des zumindest einen Auges des Brillenträgers;
- eine Flächenmodelldatenbank zum Vorgeben einer ersten Fläche und einer zweiten Fläche für das zu berechnende bzw. optimierende Brillenglas;
- ein Hauptstrahlermittlungsmodul zum Ermitteln des Verlaufs eines Hauptstrahls durch zumindest einen Durchblickspunkt zumindest einer zu berechnenden oder optimierenden Fläche des Brillenglases in das Modellauge;
- ein Auswertemodul zum Auswerten einer Aberration einer entlang des Hauptstrahls aus einer auf die erste Fläche des Brillenglases auftreffenden sphärischen Wellenfront resultierenden Wellenfront an einer Bewertungsfläche im Vergleich zu einer in einem Punkt auf der Netzhaut des Augenmodells konvergierenden Wellenfront; und
- ein Optimierungsmodul zum iterativen Variieren der zumindest einen zu berechnenden oder optimierenden Fläche des Brillenglases, bis die ausgewertete Aberration einer vorgegebenen Sollaberration entspricht;

wobei die Vorrichtung zum Ermitteln relevanter individueller Parameter des zumindest einen Auges des Brillenträgers umfasst:
   - zumindest eine Datenschnittstelle zum Bereitstellen von individuellen Refraktionsdaten des zumindest einen Auges des Brillenträgers; und
   - ein Modellierungsmodul zum Festlegen eines individuellen Augenmodells, welches zumindest
      -- eine Form und/oder Wirkung einer Hornhaut, insbesondere einer Hornhautvorderfläche, eines Modellauges;
      -- einen Hornhaut-Linsen-Abstand;
      -- Parameter der Linse des Modellauges; und
      -- einen Linsen-Netzhaut-Abstand
   als Parameter des individuellen Augenmodells festlegt, wobei das Festlegen der Parameter des individuellen Augenmodells derart anhand von Daten zur Sehschärfekorrektur des zumindest einen, die Intraokularlinse aufweisenden Auges und ferner anhand von individuellen Messwerten für das Auge des Brillenträgers und/oder von Standardwerten und/oder anhand der bereitgestellten individuellen Refraktionsdaten erfolgt, dass das Modellauge die bereitgestellten individuellen Refraktionsdaten aufweist, wobei die Daten zur Sehschärfekorrektur einen ermittelten Linsen-Netzhaut-Abstand und/oder Intraokularlinsendaten umfassen;
wobei das Augenmodell ein konsistentes Augenmodell ist, und wobei die Vorrichtung zum Ermitteln relevanter individueller Parameter des zumindest einen Auges des Brillenträgers ausgelegt ist, die Parameter des individuellen Augenmodells auf Basis einer initialen Verteilung von Parametern des Augenmodells und individuellen Daten von Eigenschaften des zumindest einen Auges unter Verwendung von Wahrscheinlichkeitsrechnung zu bestimmen.

**In** einer bevorzugten Ausführungsform erfolgt das Festlegen der Parameter des individuellen Augenmodells derart anhand von Intraokularlinsendaten und ferner anhand von individuellen Messwerten für das Auge des Brillenträgers und/oder von Standardwerten und/oder anhand der bereitgestellten individuellen Refraktionsdaten, dass das Modellauge die bereitgestellten individuellen Refraktionsdaten aufweist, wobei das Festlegen der Parameter der Linse des Modellauges anhand der Intraokularlinsendaten erfolgt.

**In** einer weiteren bevorzugten Ausführungsform ist das Modellierungsmodul ausgelegt, einen Linsen-Netzhaut-Abstand des Auges des Brillenträgers zu ermitteln. Ferner erfolgt das Festlegen der Parameter des individuellen Augenmodells derart anhand des ermittelten Linsen-Netzhaut-Abstands und ferner anhand von individuellen Messwerten für das Auge des Brillenträgers und/oder von Standardwerten und/oder anhand der bereitgestellten individuellen Refraktionsdaten erfolgt, dass das Modellauge die bereitgestellten individuellen Refraktionsdaten aufweist, wobei der Linsen-Netzhaut-Abstand des Modellauges durch den ermittelten Linsen-Netzhaut-Abstand des Auges des Brillenträgers festgelegt ist.

Insbesondere umfasst eine Vorrichtung zum Ermitteln relevanter individueller Parameter zumindest eines Auges eines Brillenträgers für die Berechnung oder Optimierung eines Brillenglases für das zumindest eine Auge des Brillenträgers, wobei das zumindest eine Auge des Brillenträgers eine implantierte Intraokularlinse aufweist:
- zumindest eine Datenschnittstelle zum Bereitstellen von individuellen Refraktionsdaten des zumindest einen Auges des Brillenträgers; und
- ein Modellierungsmodul zum Festlegen eines individuellen Augenmodells, welches zumindest
   -- eine Form und/oder Wirkung einer Hornhaut, insbesondere einer Hornhautvorderfläche, eines Modellauges;
   -- einen Hornhaut-Linsen-Abstand;
   -- Parameter der Linse des Modellauges; und
   -- einen Linsen-Netzhaut-Abstand
als Parameter des individuellen Augenmodells festlegt, wobei:
a) das Festlegen der Parameter des individuellen Augenmodells derart anhand von Intraokularlinsendaten und ferner anhand von individuellen Messwerten für das Auge des Brillenträgers und/oder von Standardwerten und/oder anhand der bereitgestellten individuellen Refraktionsdaten erfolgt, dass das Modellauge die bereitgestellten individuellen Refraktionsdaten aufweist, wobei das Festlegen der Parameter der Linse des Modellauges anhand der Intraokularlinsendaten erfolgt; und/oder
b) ein Linsen-Netzhaut-Abstand des Auges des Brillenträgers durch das Modellierungsmodul ermittelt wird und das Festlegen der Parameter des individuellen Augenmodells derart anhand des ermittelten Linsen-Netzhaut-Abstands und ferner anhand von individuellen Messwerten für das Auge des Brillenträgers und/oder von Standardwerten und/oder anhand der bereitgestellten individuellen Refraktionsdaten erfolgt, dass das Modellauge die bereitgestellten individuellen Refraktionsdaten aufweist, wobei der Linsen-Netzhaut-Abstand des Modellauges durch den ermittelten Linsen-Netzhaut-Abstand des Auges des Brillenträgers festgelegt ist.

Die Vorrichtung bzw. das Modellierungsmodul kann ausgelegt sein, sowohl die Vorgehensweise a) als auch die Vorgehensweise b) auszuführen. Alternativ kann die Vorrichtung aber auch ausgelegt sein, entweder nur die unter Punkt a) definierte Vorgehensweise oder die unter Punkt b) definierte Vorgehensweise auszuführen.

Dabei ist im ersteren Fall a) die Datenschnittschnelle insbesondere ausgelegt, zusätzlich zu den individuellen Refraktionsdaten des zumindest einen Auges des Brillenträgers auch die Intraokularlinsendaten bereitzustellen.

Insbesondere umfasst eine Vorrichtung zum Ermitteln relevanter individueller Parameter zumindest eines Auges eines Brillenträgers für die Berechnung oder Optimierung eines Brillenglases für das zumindest eine Auge des Brillenträgers, wobei das zumindest eine Auge des Brillenträgers (insbesondere anstelle oder zusätzlich zu der natürlichen Augenlinse) eine implantierte Intraokularlinse aufweist:
- zumindest eine Datenschnittstelle zum Bereitstellen von Intraokularlinsendaten der in das Auge des Brillenträgers implantierten Intraokularlinse und zum Bereitstellen von individuellen Refraktionsdaten des zumindest einen Auges des Brillenträgers; und
- ein Modellierungsmodul zum Festlegen eines individuellen Augenmodells, welches insbesondere zumindest
   -- eine Form und/oder Wirkung einer Hornhaut, insbesondere einer Hornhautvorderfläche, eines Modellauges;
   -- einen Hornhaut-Linsen-Abstand;
   -- Parameter der Linse des Modellauges; und
   -- einen Linsen-Netzhaut-Abstand
derart anhand der bereitgestellten Intraokularlinsendaten und ferner anhand von individuellen Messwerten für das Auge des Brillenträgers und/oder von Standardwerten und/oder anhand der bereitgestellten individuellen Refraktionsdaten festlegt, dass das Modellauge die bereitgestellten individuellen Refraktionsdaten aufweist, wobei das Festlegen der Parameter der Linse des Modellauges anhand der bereitgestellten Intraokularlinsendaten erfolgt. Das Festlegen des Linsen-Netzhaut-Abstands erfolgt vorzugsweise durch Messen und/oder Berechnen.

Vorzugsweise ist das Modellierungsmodul ausgelegt zum Ermitteln einer Augenlänge des Modellauges unter Berücksichtigung des gemessenen und/oder berechneten Linsen-Netzhaut-Abstands. Vorzugsweis umfasst die Vorrichtung außerdem eine Anzeigeeinrichtung zum Anzeigen des gemessenen und/oder berechneten Linsen-Netzhaut-Abstands und/oder der ermittelten Augenlänge. Besonders bevorzugt ist die Vorrichtung als Aberrometer und/oder als Topograf ausgebildet.

Vorzugsweise ist das Modellierungsmodul ausgelegt, eine Konsistenzprüfung des festgelegten Augenmodells, insbesondere des festgelegten Vor-OP-Augenmodells und/oder des festgelegten Nach-OP-Augenmodells, durchzuführen. Ferner vorzugsweise ist das Modellierungsmodul ausgelegt, etwaige Inkonsistenzen, insbesondere mit Hilfe von analytischen und/oder probabilistischen Verfahren (Wahrscheinlichkeitsrechnung z.B. unter Verwendung von Bayes'sche Statistik und/oder eines Maximum Likelihood Ansatzes), aufzulösen.

Insbesondere wird im Rahmen der vorliegenden Erfindung eine Vorrichtung zum Ermitteln relevanter individueller Parameter zumindest eines Auges eines Brillenträgers für die Berechnung oder Optimierung eines Brillenglases für das zumindest eine Auge des Brillenträgers bereitgestellt, wobei im Rahmen einer Operation eine Intraokularlinse in das zumindest eine Auge des Brillenträgers implantiert wurde und wobei die Vorrichtung umfasst:
- zumindest eine Datenschnittstelle zum Bereitstellen von individuellen Nach-OP-Refraktionsdaten des zumindest einen Auges des Brillenträgers; und
- ein Modellierungsmodul zum Ermitteln eines Linsen-Netzhaut-Abstands des Auges des Brillenträgers und zum Festlegen eines individuellen Nach-OP-Augenmodells, welches insbesondere zumindest
   -- eine Form und/oder Wirkung einer Hornhaut, insbesondere einer Hornhautvorderfläche, eines Modellauges des Nach-OP-Augenmodells;
   -- einen Hornhaut-Linsen-Abstand des Modellauges des Nach-OP-Augenmodells;
   -- Parameter der Linse des Modellauges des Modellauges des Nach-OP-Augenmodells; und
   -- einen Linsen-Netzhaut-Abstand des Modellauges des Nach-OP-Augenmodells
derart anhand des ermittelten Linsen-Netzhaut-Abstands und ferner anhand von individuellen Messwerten für das Auge des Brillenträgers (ermittelt vor oder nach der OP) und/oder von Standardwerten und/oder anhand der bereitgestellten individuellen Nach-OP-Refraktionsdaten festlegt, dass das Modellauge des Nach-OP-Augenmodells die bereitgestellten individuellen Nach-OP-Refraktionsdaten aufweist, wobei der Linsen-Netzhaut-Abstand des Modellauges des Nach-OP-Augenmodells durch den ermittelten Linsen-Netzhaut-Abstand des Auges des Brillenträgers festgelegt ist.

Ein weiterer unabhängiger Aspekt zur Lösung der Aufgabe betrifft eine Vorrichtung zum Herstellen eines Brillenglases, umfassend:
Berechnungs- oder Optimierungsmittel, welche ausgelegt sind, das Brillenglas nach einem erfindungsgemäßen Verfahren zum Berechnen oder Optimieren eines Brillenglases zu berechnen oder zu optimieren; und
Bearbeitungsmittel, welche ausgelegt sind, das Brillenglas gemäß dem Ergebnis der Berechnung bzw. Optimierung zu bearbeiten.

Die Vorrichtung zum Herstellen eines Brillenglases kann einstückig bzw. als eine eigenständige Maschine ausgestaltet sein, d.h. sämtliche Komponenten der Vorrichtung (insbesondere die Berechnungs- oder Optimierungsmittel und die Bearbeitungsmittel) können Bestandteil ein und desselben Systems bzw. ein und derselben Maschine sein. In einer bevorzugten Ausführungsform ist die Vorrichtung zum Herstellen eines Brillenglases jedoch nicht einstückig ausgestaltet, sondern durch verschiedene (insbesondere jeweils eigenständige) Systeme bzw. Maschinen realisiert. So können z.B. die Berechnungs- oder Optimierungsmittel als ein erstes System (insbesondere umfassend einen Computer) und die Bearbeitungsmittel als ein zweites System (insbesondere eine Maschine umfassend die Bearbeitungsmittel) realisiert sein. Dabei können sich die verschiedenen Systeme an unterschiedlichen Orten befinden, d.h. örtlich voneinander getrennt sein. Beispielsweise können sich ein oder mehrere Systeme im Frontend und ein oder mehrere weitere Systeme im Backend befinden. Die einzelnen Systeme können sich z.B. an unterschiedlichen Firmenstandorten befinden oder durch unterschiedliche Firmen betrieben werden. Die einzelnen Systeme weisen dabei insbesondere Kommunikationsmittel auf, um Daten untereinander auszutauschen (beispielsweise über einen Datenträger). Vorzugsweise können die verschiedenen Systeme der Vorrichtung direkt miteinander kommunizieren, insbesondere über ein Netzwerk (z.B. über ein lokales Netzwerk und/oder über das Internet). Die obigen Ausführungen zu der Vorrichtung zum Herstellen eines Brillenglases gelten nicht nur für diese Vorrichtung, sondern allgemein für sämtliche im Rahmen der vorliegenden Erfindung beschriebenen Vorrichtungen. Insbesondere kann eine hierin beschriebene Vorrichtung als ein System ausgestaltet sein. Das System kann insbesondere mehrere Vorrichtungen (ggf. örtlich getrennt) umfassen, welche ausgelegt sind, einzelne Verfahrensschritte eines entsprechenden Verfahrens auszuführen.

Außerdem bietet die Erfindung ein Computerprogrammerzeugnis bzw. ein Computerprogrammprodukt, insbesondere in Form eines Speichermediums oder eines Datenstroms, welches Programmcode enthält, der ausgelegt ist, wenn geladen und ausgeführt auf einem Computer, ein erfindungsgemäßes Verfahren zum Berechnen oder Optimieren eines Brillenglases durchzuführen. Insbesondere ist unter einem Computerprogrammprodukt ein auf einem Datenträger gespeichertes Programm zu verstehen. Insbesondere ist der Programmcode auf einem Datenträger gespeichert. Mit anderen Worten umfasst das Computerprogrammprodukt computerlesbare Anweisungen, welche, wenn geladen in einen Speicher eines Computers und ausgeführt von dem Computer, bewirken, dass der Computer ein erfindungsgemäßes Verfahren durchführt.

Mittels eines erfindungsgemäßen Verfahrens und/oder mittels einer erfindungsgemäßen Vorrichtung kann ein Brillenglas hergestellt werden.

Außerdem bietet die Erfindung eine (nicht beanspruchte) Verwendung eines nach dem Herstellungsverfahren gemäß der vorliegenden Erfindung, insbesondere in einer bevorzugten Ausführungsform, hergestellten Brillenglases in einer vorgegebenen durchschnittlichen oder individuellen Gebrauchsstellung des Brillenglases vor den Augen eines bestimmten Brillenträgers zur Korrektur einer Fehlsichtigkeit des Brillenträgers.

Insbesondere kann ein erfindungsgemäßes computerimplementiertes Verfahren in Form einer Bestell- und/oder Branchensoftware bereitgestellt werden. Insbesondere können in einem solchen Verfahren die für die Berechnung und/oder Optimierung und/oder Herstellung eines Brillenglases benötigten Daten, insbesondere der Intraokularlinsendaten und/oder der Rezeptdaten und/oder der individuellen Refraktionsdaten (Vor-OP und/oder Nach-OP Refraktionsdaten) des zumindest einen Auges des Brillenträgers, erfasst und/oder übertragen werden. Die Übertragung der Intraokularlinsendaten kann z.B. vom Hersteller der Intraokularlinsendaten zum Berechner und/oder Hersteller des Brillenglases erfolgen. Die Übertragung der Rezeptdaten und/oder von individuellen Refraktionsdaten kann z.B. vom Optiker und/oder Augenarzt bzw. Chirurg zum Berechner und/oder Hersteller des Brillenglases erfolgen. Alternativ oder zusätzlich kann es möglich sein, diese Daten von einer Datenbank, insbesondere mit Hilfe einer Typ- und/oder Seriennummer der implantierten IOL bzw. mit Hilfe eines Patientencodes (z.B. Kunden- bzw. Patientennummer, Name, usw.) abzurufen. Mess- bzw. Refraktionsdaten können z.B. auch direkt von einem Messgerät abgerufen werden. Für die Übertragung der Daten kann ein gängiges oder ein speziell für das erfindungsgemäße Verfahren entwickeltes Übertragungs-Protokoll verwendet werden. Alternativ oder zusätzlich können die zu übertragenen Daten, zumindest teilweise, auch manuell über eine Eingabeeinheit eingegeben werden. Beispielsweise kann ein Augenarzt bzw. Chirurg auf diese Weise die sogenannte A-Konstante bzw. IOL-Konstante der verwendeten Intraokularlinse übermitteln. Insbesondere kann auch ein Linsen- bzw. IOL-Pass auf Basis der übermittelten Daten semi- oder vollautomatisch erstellt werden.

Eine erfindungsgemäße Vorrichtung und/oder ein erfindungsgemäßes System, z.B. zum Bestellen eines Brillenglases, kann insbesondere einen Computer und/oder Datenserver umfassen, der ausgelegt ist, um über ein Netzwerk (z.B. Internet) kommunizieren kann. Der Computer ist insbesondere ausgelegt, ein computerimplementiertes Verfahren, z.B. eine Bestellsoftware zum Bestellen zumindest eines Brillenglases, und/oder eine Übertragungssoftware zum Übertragung von relevanten Daten (insbesondere Intraokularlinsendaten und/oder Rezeptdaten und/oder Refraktionsdaten), und/oder eine Ermittlungs-Software zum Ermitteln relevanter individueller Parameter zumindest eines Auges eines Brillenträgers, und/oder eine Berechnungs- bzw. Optimierungs-Software zur Berechnung und/oder Optimierung eines herzustellenden Brillenglases, gemäß der vorliegenden Erfindung auszuführen.

Für die oben genannten weiteren unabhängigen Aspekte bzw. Ansätze und insbesondere für diesbezügliche bevorzugte Ausführungsformen gelten auch die vor- oder nachstehend gemachten Ausführungen zu den Ausführungsformen des ersten Aspekts. Insbesondere gelten für einen unabhängigen Aspekt der vorliegenden Erfindung und für diesbezügliche bevorzugte Ausführungsformen auch die vor- und nachstehend gemachten Ausführungen zu den Ausführungsformen der jeweils anderen unabhängigen Aspekte.

Bevorzugte Ausführungsformen und Beispiele der Erfindung werden nachfolgend zumindest teilweise unter Bezugnahme auf die beigefügten Zeichnungen beispielhaft erläutert. Dabei zeigt:
- Figur 1: eine schematische Darstellung des physiologischen und physikalischen Modells eines Brillenglases und eines Auges zusammen mit einem Strahlverlauf in einer vorgegebenen Gebrauchsstellung;
- Figur 2: ein Graph mit einer beispielhaften Abhängigkeit von *P^{mess} S_{IOL}* und *L_{2,IOL}* zur Illustration bzw. Erklärung eines Verfahrens zur Parameterbestimmung unter Zwangsbedingungen gemäß eines bevorzugten Ausführungsbeispiels der vorliegenden Erfindung.

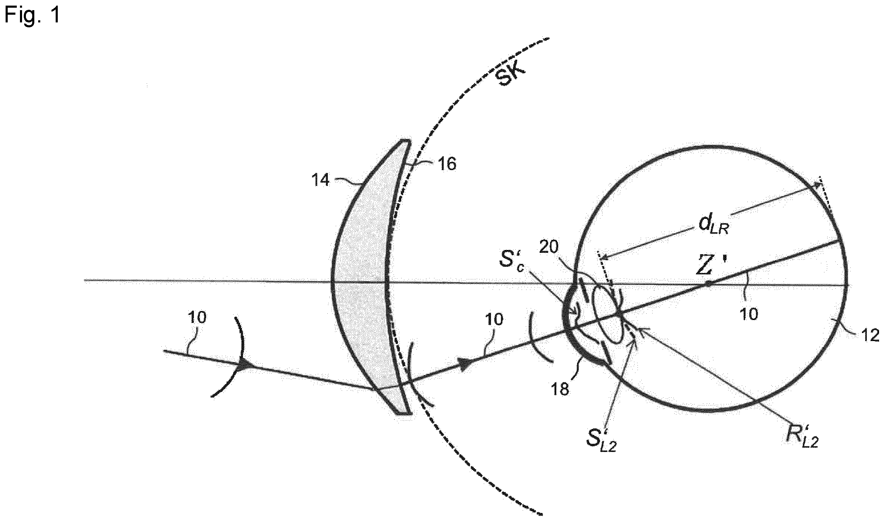

Figur 1 zeigt eine schematische Darstellung des physiologischen und physikalischen Modells eines Brillenglases und eines Auges in einer vorgegebenen Gebrauchsstellung zusammen mit einem beispielhaften Strahlverlauf, welches einer individuellen Brillenglasberechnung bzw. -optimierung gemäß einer bevorzugten Ausführungsform der Erfindung zugrunde liegt.

Hierbei wird pro Durchblickspunkt des Brillenglases vorzugsweise nur ein einziger Strahl berechnet (der Hauptstrahl 10, der vorzugsweise durch den Augendrehpunkt Z' läuft), begleitend aber außerdem auch die Ableitungen der Pfeilhöhen der Wellenfront nach den transversalen (zum Hauptstrahl senkrechten) Koordinaten. Diese Ableitungen werden bis zur gewünschten Ordnung berücksichtigt, wobei die zweiten Ableitungen die lokalen Krümmungseigenschaften der Wellenfront beschreiben und die höheren Ableitungen mit den Abbildungsfehlern höherer Ordnungen zusammenhängen.

Bei der Durchrechnung von Licht durch das Brillenglas bis in das Auge 12 gemäß dem individuell bereitgestellten Augenmodell werden die lokalen Ableitungen der Wellenfronten im Endeffekt an einer geeigneten Position im Strahlverlauf ermittelt, um sie dort mit einer Referenzwellenfront zu vergleichen, welche in einem Punkt auf der Retina des Auges 12 konvergiert. Insbesondere werden die beiden Wellenfronten (d.h. die vom Brillenglas kommende Wellenfront und die Referenzwellenfront) in einer Bewertungsfläche miteinander verglichen.

**Unter "Position"** ist dabei nicht einfach nur ein bestimmter Wert der z-Koordinate (in Lichtrichtung) gemeint, sondern ein solcher Koordinatenwert in Kombination mit der Angabe aller Flächen, durch die vor Erreichen der Bewertungsfläche gebrochen wurde. In einer bevorzugten Ausführungsform wird durch alle brechenden Flächen einschließlich der Linsenrückfläche gebrochen. In diesem Fall dient als Referenzwellenfront vorzugsweise eine sphärische Wellenfront, deren Krümmungsmittelpunkt auf der Retina des Auges 12 liegt.

Besonders bevorzugt wird ab dieser letzten Brechung nicht weiter propagiert, so dass der Krümmungsradius dieser Referenzwellenfront gerade dem Abstand zwischen Linsenrückfläche und Retina entspricht. In einer außerdem bevorzugten Ausführungsform wird nach der letzten Brechung noch propagiert, und zwar bevorzugt bis zur Austrittspupille AP des Auges 12. Diese liegt beispielsweise im Abstand ${d}_{AR} = {d}_{LR}^{\left(b\right)} = {d}_{LR} - {d}_{LR}^{\left(a\right)} > {d}_{LR}$ vor der Retina und damit sogar vor der Linsenrückfläche, so dass die Propagation in diesem Fall eine Rückpropagation ist (die Bezeichnungen ${d}_{LR}^{\left(a\right)} , {d}_{LR}^{\left(b\right)}$ werden weiter unten noch bei der Aufzählung der Schritte 1-6 beschrieben). Auch in diesem Fall ist die Referenzwellenfront sphärisch mit Krümmungsmittelpunkt auf der Retina, besitzt aber Krümmungsradius *1*/*d_{AR}.*

Hierzu wird angenommen, dass eine sphärische Wellenfront *w*₀ vom Objektpunkt ausgeht und bis zur ersten Brillenglasfläche 14 propagiert. Dort wird sie gebrochen und anschließend propagiert sie bis zur zweiten Brillenglasfläche 16, wo sie wieder gebrochen wird. Die vom Brillenglas austretenden Wellenfront *w_{g1}* propagiert anschließend entlang des Hauptstrahls in Richtung des Auges 12 (propagierte Wellenfront w_{g2}) bis sie auf die Cornea 18 trifft, wo sie wiederum gebrochen wird (Wellenfront w_{c}). Nach einer weiteren Propagation innerhalb der Augenvorderkammer bis zur Augenlinse 20, wird die Wellenfront auch von der Augenlinse 20 wiederum gebrochen, wodurch beispielsweise an der Rückfläche der Augenlinse 20 oder an der Austrittspupille des Auges die resultierenden Wellenfront wₑ entsteht. Diese wird mit der sphärischen Referenzwellenfront wₛ verglichen und die Abweichungen werden für alle Durchblickspunkte in der Zielfunktion (vorzugsweise mit entsprechenden Gewichtungen für die einzelnen Durchblickspunkte) bewertet.

Somit wird die Fehlsichtigkeit nicht mehr nur durch eine dünne sphäro-zylindrische Linse beschrieben, wie dies in vielen herkömmlichen Verfahren üblich war, sondern vorzugsweise werden die Hornhauttopographie, die Augenlinse, die Abstände im Auge und die Deformation der Wellenfront (einschließlich der Abbildungsfehler niedriger Ordnung - also Sphäre, Zylinder und Achslage - sowie vorzugsweise auch einschließlich der Abbildungsfehler höherer Ordnung) im Auge direkt berücksichtigt. Dabei wird im Augenmodell, das nach dem erfindungsgemäßen Verfahren konstruiert wird, die Glaskörperlänge *d_{LR}* individuell berechnet.

Vorzugsweise liefert eine Aberrometermessung die individuellen Wellenfrontdeformationen des realen fehlsichtigen Auges für Ferne und Nähe (Abweichungen, keine absoluten Brechwerte) und die individuellen mesopischen und photopischen Pupillendurchmesser. Aus einer Messung der Hornhauttopographie (flächenhafte Vermessung der Cornea-Vorderfläche) erhält man vorzugsweise eine individuelle reale Hornhautvorderfläche, die im Allgemeinen nahezu 75% des Gesamtbrechwertes des Auges ausmacht. In einer bevorzugten Ausführungsform ist es nicht notwendig, die Cornea-Rückfläche zu vermessen. Sie wird vorzugsweise wegen des geringen Brechzahlunterschiedes zum Kammerwasser und wegen der geringen Hornhautdicke in guter Näherung nicht durch eine separate brechende Fläche, sondern durch eine Anpassung des Brechungsindex der Cornea beschrieben. Generell sollen in dieser Beschreibung fett gesetzte Kleinbuchstaben Vektoren und fett gesetzte Großbuchstaben Matrizen bezeichnen, wie z.B. die (2 x 2) - Vergenzmatrizen bzw. -Brechwertmatrizen $S = \left(\begin{matrix}{S}_{xx} & {S}_{xy} \\ {S}_{xy} & {S}_{yy}\end{matrix}\right) , C = \left(\begin{matrix}{C}_{xx} & {C}_{xy} \\ {C}_{xy} & {C}_{yy}\end{matrix}\right) , L = \left(\begin{matrix}{L}_{xx} & {L}_{xy} \\ {L}_{xy} & {L}_{yy}\end{matrix}\right) , 1 = \left(\begin{matrix}1 & 0 \\ 0 & 1\end{matrix}\right)$ und kursiv gesetzte wie d skalare Größen.

Weiterhin sollen fette, kursiv gesetzte Großbuchstaben Wellenfronten oder Flächen als Ganzes bezeichnen. So ist z.B. S die Vergenzmatrix der gleichnamigen Wellenfront S, nur dass S außer den Aberrationen 2-ter Ordnung, die in S zusammengefasst sind, auch die Gesamtheit aller Aberrationen höherer Ordnung (=HOA für engl. *Higher Order Aberrations)* der Wellenfront mit umfasst. Mathematisch gesehen steht S für die Menge aller Parameter, die notwendig sind, um eine Wellenfront (ausreichend genau) in Bezug auf ein gegebenes Koordinatensystem zu beschreiben. Vorzugsweise steht S für einen Satz von Zernike-Koeffizienten mit einem Pupillenradius oder einen Satz aus Koeffizienten einer Taylorreihe. Besonders bevorzugt steht S für die Menge aus einer Vergenzmatrix S zur Beschreibung der Wellenfronteigenschaften 2. Ordnung und einem Satz von Zernike-Koeffizienten (mit einem Pupillenradius), der zur Beschreibung aller restlichen Wellenfronteigenschaften außer der 2.Ordnung dient oder einem Satz von Koeffizienten gemäß einer Zerlegung nach Taylor. Für Flächen statt Wellenfronten gelten analoge Aussagen.

Unter anderem können folgende Daten im Prinzip direkt gemessen werden:
- Die Wellenfront *S_{M},* die durch den Laserspot auf der Retina und den Durchgang durch das Auge erzeugt wird (aus aberrometrischer Messung)
- Form der Hornhautvorderfläche C (durch Hornhaut-Topographie)
- Abstand zwischen Hornhaut und Linsenvorderfläche *d_{CL}* (durch Pachymetrie). Diese Größe kann auch indirekt durch die Messung des Abstandes zwischen der Hornhaut und der Regenbogenhaut ermittelt werden, dabei können gegebenenfalls Korrekturwerte angewendet werden. Derartige Korrekturen können der Abstand zwischen der Linsenvorderfläche und der Regenbogenhaut aus bekannten Augenmodellen (z.B. Literaturwerte) sein.
- Krümmung der Linsenvorderfläche in einer Richtung *L₁ₓₓ* (durch Pachymetrie) Dabei kann ohne Einschränkung der Allgemeinheit die x-Ebene beispielsweise derart definiert werden, dass dieser Schnitt in der x-Ebene liegt. Wird das Koordinatensystem so definiert, dass diese Ebene schief liegt, muss die Ableitung durch die Funktionen des entsprechenden Winkels ergänzt werden. Es wird nicht gefordert, dass es sich dabei um einen Hauptschnitt handelt. Beispielsweise kann es sich um den Schnitt in der horizontalen Ebene handeln.

Weiterhin können folgende Daten - je nach Ausführungsform - entweder gemessen oder der Literatur entnommen werden:
- Dicke der Linse *d_{L}*
- Krümmung der Linsenrückfläche in derselben Richtung wie die Linsenvorderfläche *L_{2,xx}* (durch Pachymetrie)

Damit gibt es für die Linsenrückfläche folgende Möglichkeiten:
- Messung von *L_{2,xx} (L_{2,M})* und Annahme einer Rotationssymmetrie *L_{2,xx} = L_{2,yy} = L₂ = L_{2,M}* und *L_{2,xy} = L_{2,yx} =* 0
- Entnahme von *L_{2,xx}* aus der Literatur *(L_{2,Lit})* und Annahme einer Rotationssymmetrie *L_{2,xx} = L_{2,yy} = L₂ = L_{2,M}* und *L_{2,xy} = L_{2,yx} =* 0
- Entnahme der vollständigen (unsymmetrischen) Form *L₂* aus der Literatur *(L_{2,Lit})*
- Messung von *L_{2,xx} (L_{2,M})* und Annahme eines Zylinders oder einer anderweitig spezifizierten Asymmetrie *a_{Lit}* aus der Literatur *L_{2,xx} = L_{2,M}* und *L_{2,xy} = L_{2,yx} = f(L_{2,xx}, a_{Lit})* sowie *L_{2,yy} = g(L_{2,xx}, a_{Lit})*

Folgende Daten können der Literatur entnommen werden:
- Brechungsindices *n_{CL}* von Hornhaut und Augenvorderkammer sowie des Kammerwassers *n_{LR}* und der der Linse *n_{L}*

Damit verbleiben insbesondere der Abstand *d_{LR}* zwischen Linsenrückfläche und Netzhaut sowie die Komponenten *L_{1,yy}* und *L_{1,xy} = L_{1,yx}* der Linsenvorderfläche als unbekannte Parameter. Zur Vereinfachung des Formalismus kann ersterer auch als Vergenzmatrix *D_{LR} = D_{LR} .* **1** mit *D_{LR} = n_{LR}*/*d_{LR}* geschrieben werden. Weiterhin wird generell die Größe *τ* verwendet, die definiert ist als *τ* = *d*/*n* (wobei für den Brechungsindex als *n* immer der entsprechende Index zu verwenden ist wie für *d* und *τ,* z.B. als *τ_{LR} = d_{LR}*/*n_{LR}, τ_{CL} = d_{CL}*/*n_{CL}).*

Die Modellierung des Durchgangs der Wellenfront durch das verwendete Augenmodell, das nach dem erfindungsgemäßen Verfahren konstruiert wird, also nach dem Durchgang durch die Flächen des Brillenglases, kann in einer bevorzugten Ausführungsform, in der die Linse über eine Vorder- und eine Rückfläche beschrieben wird, wie folgt beschrieben werden, wobei die Transformationen der Vergenzmatrizen explizit angegeben wird:
1. Brechung der Wellenfront ***S*** mit der Vergenzmatrix ***S*** an der Hornhaut ***C*** mit der Flächenbrechwertmatrix **C** zur Wellenfront ***S*'_{C}** mit Vergenzmatrix ***S*'_{C}** = **S** + **C**
2. Propagation um die Vorderkammertiefe *d_{CL}* (Abstand zwischen Hornhaut und Linsenvorderfläche) zur Wellenfront S_{L1} mit Vergenzmatrix S_{L1} = *S'*_{*C*/}*(1 - τ_{CL} ·* S') ${S}_{L 1} = \frac{S {′}_{C}}{\left(1-{τ}_{CL}⋅S{′}_{C}\right)}$
3. Brechung an der Linsenvorderfläche ***L*₁** mit der Flächenbrechwertmatrix **L₁** zur Wellenfront ***S'*_{*L*1}** mit der Vergenzmatrix ***S'*_{*L*1}** = ***S*_{*L*1}** + **L₁**
4. Propagation um die Linsendicke *d_{L}* zur Wellenfront S_{L2} mit Vergenzmatrix S_{L2} = S'_{L1}/(1 - *τ_{L} · S'_{L1})*
5. Brechung an der Linsenrückfläche *L₂* mit der Flächenbrechwertmatrix *L₂* zur Wellenfront S'_{L2} mit Vergenzmatrix S'_{L2} = S_{L2} + L₂
6. Propagation um den Abstand zwischen Linse und Netzhaut *d_{LR}* zur Wellenfront *S_{R}* mit der Vergenzmatrix *S_{R} = S'_{L2}*/*(1 - τ_{LR} ·* S'_{L2})

Jeder der Schritte 2, 4, 6, bei denen die Abstände *τ_{CL}, τ_{CL},* bzw. *τ_{CL}* propagiert wird, kann dabei aufgeteilt werden in zwei Teilpropagationen 2a,b), 4a,b) bzw. 6a,b) nach dem folgenden Schema, das für den Schritt 6a,b) explizit lautet:
6a. Propagation um den Abstand ${d}_{LR}^{\left(a\right)}$ zwischen Linse und Zwischenebene zur Wellenfront *S_{LR}* mit der Vergenzmatrix ${S}_{LR} = S {′}_{L 2} / \left(1-{τ}_{LR}^{\left(a\right)}S{′}_{L 2}\right)$
6b. Propagation um den Abstand ${d}_{LR}^{\left(b\right)}$ zwischen Zwischenebene und Netzhaut zur Wellenfront *S_{R}* mit der Vergenzmatrix ${S}_{R} = {S}_{LR} / \left(1-{τ}_{LR}^{\left(b\right)}{S}_{LR}\right)$

Dabei können ${τ}_{LR}^{\left(a\right)} = {d}_{LR}^{\left(a\right)} / {n}_{LR}^{\left(a\right)}$ und ${τ}_{LR}^{\left(b\right)} = {d}_{LR}^{\left(b\right)} / {n}_{LR}^{\left(b\right)}$ positiv oder negativ sein, wobei stets ${n}_{LR}^{\left(a\right)} = {n}_{LR}^{\left(b\right)} = {n}_{LR}$ und ${τ}_{LR}^{\left(a\right)} + {τ}_{LR}^{\left(b\right)} = {τ}_{LR}$ sein soll. In jedem Fall lassen sich Schritt 6a und 6b durch ${S}_{R} = S {′}_{L 2} / \left(1-\left({τ}_{LR}^{\left(a\right)}+{τ}_{LR}^{\left(b\right)}\right)S{′}_{L 2}\right) = S {′}_{L 2} / \left(1-{τ}_{LR}S{′}_{L 2}\right)$ wieder zusammenfassen. Die Aufteilung in Schritt 6a und 6b bietet aber Vorteile, und bevorzugt kann die Zwischenebene in die Ebene der Austrittspupille AP gelegt werden, die vorzugsweise vor der Linsenrückfläche liegt. In diesem Fall ist ${τ}_{LR}^{\left(a\right)} < 0$ und ${τ}_{LR}^{\left(b\right)} > 0$.

Analog zur Aufteilung von Schritt 6 in 6a,b) kann auch die Aufteilung der Schritte 2,4 erfolgen.

Entscheidend für die Wahl der Bewertungsfläche der Wellenfront ist also nicht nur die absolute Lage in Bezug auf die z-Koordinate (in Lichtrichtung), sondern auch die Anzahl der Flächen, durch die bis zur Bewertungsfläche schon gebrochen wurde. So kann ein- und dieselbe Ebene mehrmals durchlaufen werden. Beispielhaft wird die Ebene der AP (die normalerweise zwischen der Linsenvorderfläche und der Linsenrückfläche liegt) vom Licht formal zum ersten Mal durchlaufen nach einem gedachten Schritt 4a, bei dem von der Linsenvorderfläche um die Länge ${τ}_{L}^{\left(a\right)} > 0$ propagiert wird. Zum zweiten Mal wird die gleiche Ebene erreicht nach Schritt 6a, wenn nach der Brechung durch die Linsenrückfläche wieder zur AP-Ebene zurückpropagiert wird, d.h. ${τ}_{LR}^{\left(a\right)} = - {τ}_{L} + {τ}_{L}^{\left(a\right)} = - {τ}_{L}^{\left(b\right)} < 0$, was gleichbedeutend ist mit ${τ}_{LR}^{\left(a\right)} = {τ}_{LR} - {τ}_{LR}^{\left(b\right)} < 0$*.* Bei den Wellenfronten *S_{AP},* die sich im Text auf die AP beziehen, soll (falls nicht explizit anders erwähnt) vorzugsweise immer die Wellenfront *S_{AP} = S_{LR}* gemeint sein, die Ergebnis von Schritt 6a ist."

Auf diese Schritte 1 bis 6 wird im weiteren Verlauf der Beschreibung immer wieder Bezug genommen. Sie beschreiben einen bevorzugten Zusammenhang zwischen der Vergenzmatrix S einer Wellenfront S an der Hornhaut und den Vergenzmatrizen aller daraus hervorgehenden Zwischenwellenfronten an den brechenden Zwischenflächen des Auges, insbesondere der Vergenzmatrix S'_{L2} einer Wellenfront S'_{L2} nach der Augenlinse (oder sogar einer Wellenfront *S_{R}* an der Netzhaut). Diese Zusammenhänge können sowohl dazu verwendet werden, a-priori nicht bekannte Parameter (z.B. *d_{LR}* oder L₁) zu berechnen und so das Modell entweder individuell oder generisch mit Werten zu belegen, als auch um mit dann belegten Modellen die Ausbreitung der Wellenfront im Auge zur Optimierung von Brillengläsern zu simulieren.

In einer bevorzugten Ausführungsform werden die Flächen und Wellenfronten bis in zweiter Ordnung behandelt, wozu eine Darstellung durch Vergenzmatrizen ausreichend ist. Eine später noch beschriebene andere bevorzugte Ausführungsform berücksichtigt und nutzt auch höhere Ordnungen von Abbildungsfehlern.

In einer Beschreibung in zweiter Ordnung besitzt das Augenmodell in einer bevorzugten Ausführungsform zwölf Parameter als Freiheitsgrade des Modells, die belegt werden müssen. Diese umfassen vorzugsweise die drei Freiheitsgrade der Flächenbrechwertmatrix C der Cornea C, die jeweils drei Freiheitsgrade der Flächenbrechwertmatrizen L₁ und *L₂* für die Linsenvorder- bzw. Rückfläche, sowie jeweils einen für die Längenparameter Vorderkammertiefe *d_{CL}* , Linsendicke *d_{L}* und die Glaskörperlänge *d_{LR}.*

Belegungen dieser Parameter können im Prinzip auf mehrere Weisen erfolgen:
i) Direkte, also individuelle Messung eines Parameters
ii) A priori gegebener Wert eines Parameters, z.B. als Literaturwert oder aus einer Schätzung, beispielsweise durch Vorliegen eines Messwertes für eine andere Größe, die anhand einer vorangegangenen Populationsanalyse auf bekannte Weise mit dem zu bestimmenden Parameter korreliert
iii) Berechnung aus Konsistenzbedingungen, z.B. Verträglichkeit mit einer bekannten Refraktion

Die gesamte Anzahl df₂ von Freiheitsgraden des Augenmodells in zweiter Ordnung *(df* steht für ,degree of freedom', Index '2' für 2.Ordnung) setzt sich also zusammen aus: ${df}_{2} = {df}_{2} \left(i\right) + {df}_{2} \left(ii\right) + {df}_{2} \left(iii\right)$

Liegen beispielsweise für alle zwölf Modellparameter direkte Messwerte vor, dann ist *df₂(i) = 12, df₂(ii)* = 0 und *df₂(iii)* = 0, was nachfolgend der Einfachheit halber durch die Schreibweise df₂ = 12 + 0 + 0 ausgedrückt wird. In einem solchen Fall ist auch die objektive Refraktion des betreffenden Auges festgelegt, so dass eine objektive Refraktionsbestimmung nicht mehr zusätzlich durchgeführt werden müsste.

Ein zentraler Aspekt der Erfindung betrifft aber gerade das Ziel, nicht alle Parameter direkt messen zu müssen. So ist es insbesondere deutlich einfacher, die Refraktion des betreffenden Auges zu messen bzw. objektiv und/oder subjektiv zu bestimmen, als alle Parameter des Modellauges individuell zu vermessen. Vorzugsweise liegt somit zumindest eine Refraktion, also Messdaten zur Wellenfront S_{M} des Auges bis zur zweiten Ordnung vor, die den Daten der Vergenzmatrix S_{M} entsprechen. Bei einer Belegung des Augenmodells auf Basis rein objektiv gemessener Daten können diese Werte aberrometrische Messungen oder autorefraktometrischen Messungen entnommen werden, bzw. laut (ii) anderweitig gegebener Daten belegt werden. Eine Berücksichtigung subjektiver Verfahren (d.h. subjektive Refraktion), sei es als Ersatz der objektiven Messung der Refraktion oder durch die Kombination beider Ergebnisse, wird später noch beschrieben. Die drei Bedingungen der Übereinstimmung mit den drei unabhängigen Parametern der Vergenzmatrix S_{M} erlauben es damit, drei Parameter des Augenmodells abzuleiten, was in der oben eingeführten Notation *df₂(iii)* = 3 entspricht.

Die Erfindung nutzt also die Möglichkeit, in Fällen, in denen nicht alle Modellparameter direkten Messungen zugänglich sind, oder diese Messungen sehr aufwändig wären, die fehlenden Parameter sinnvoll zu belegen. Liegen beispielsweise höchstens für neun Modellparameter direkte Messwerte vor *(df₂(i)* ≤ 9), dann kann man die genannten Bedingungen der Refraktion benutzen, um drei der Modellparameter zu berechnen *(df₂(iii) =* 3). Falls genau *df₂(i)* = 9 gilt, dann sind alle zwölf Modellparameter durch die Messungen und die Berechnung eindeutig bestimmt, und es gilt *(df₂(ii)* = 0). Ist dagegen *df₂(i)* < 9, dann ist *df₂(ii)* = 9 - *df₂(i)* > 0, d.h. das Modell ist unterbestimmt in dem Sinne, dass *df₂(ii)* Parameter a priori festgelegt werden müssen.

Mit dem Bereitstellen einer individuellen Refraktion, also Messdaten zur Wellenfront *S_{M}* des Auges insbesondere bis zur zweiten Ordnung liegen die nötigen Daten der Vergenzmatrix S_{M} entsprechend vor. Gemäß einem in WO 2013/104548 A1 beschriebenen herkömmlichen Verfahren werden insbesondere die Parameter {C, *d_{CL}, S_{M}}* gemessen. Hingegen werden herkömmlicherweise unter anderem die beiden Längenparameter *d_{L}* und *d_{LR}* (bzw. *D_{LR})* a priori festgelegt (z.B. durch Literaturwerte oder Schätzung). In WO 2013/104548 A1 werden insbesondere die beiden Fälle unterschieden, in denen entweder *L₂* a priori festgelegt und L₁ daraus berechnet wird, oder umgekehrt. Als Berechnungsvorschrift offenbart die genannte Offenlegungsschrift hierzu Gl.(4) bzw. Gl.(5). Für beide Fälle gilt df₂ = 4 + 5 + 3.

In der Bezeichnungsweise der oben genannten Schritte 1 bis 6 geschieht die Anpassung von L₁ an die Messungen insbesondere dadurch, dass man zum einen die gemessene Vergenzmatrix S_{M} mittels der Schritte 1, 2 durch die ebenfalls gemessene Matrix C hindurch rechnet und bis zur objektseitigen Seite der Linsenvorderfläche propagiert. Zum anderen rechnet man von einer gedachten punktförmigen Lichtquelle auf der Netzhaut eine Kugelwelle mittels der rückwärts durchlaufenen Schritte 6, 5, 4 von hinten nach vorne, indem man diese Kugelwelle an der zuvor festgelegten Flächenbrechwertmatrix *L₂* der Linsenrückfläche bricht und die dann erhaltene Wellenfront von der Linsenrückfläche bis zur bildseitigen Seite der Linsenvorderfläche propagiert. Die Differenz der so bestimmten Vergenzmatrizen **S**_{*L*1} und **S**'_{*L*1}, die objektseitig bzw. bildseitig der Linsenvorderfläche liegen muss durch die Matrix **L**₁ bewirkt worden sein, denn bei der aberrometrischen Messung geht die gemessene Wellenfront aus einer Wellenfront hervor, die von einem Punkt auf der Netzhaut ausgeht, und deshalb aufgrund der Umkehrbarkeit der Strahlengänge identisch mit derjenigen einfallenden Wellenfront ist (**S** = **S***_{M}),* die auf diesen Punkt der Netzhaut zusammenläuft. Dies führt zu Gleichung (4) in der genannten Offenlegungsschrift: ${L}_{1} \left({D}_{LR}\right) = \frac{{D}_{LR} ⋅ 1 - {L}_{2}}{1 + {τ}_{L} ⋅ \left({D}_{LR}⋅1-{L}_{2}\right)} - \frac{{S}_{M} + C}{1 - {τ}_{CL} \left({S}_{M}+C\right)}$ (1a)

Der andere Fall in der genannten Offenlegungsschrift betrifft die Anpassung der Matrix **L**₂ an die Messungen, nachdem die Matrix **L**₁ festgelegt worden ist. Ein Unterschied besteht jetzt lediglich darin, dass die gemessene Wellenfront **S***_{M}* den Schritten 1, 2, 3, 4 unterzogen wird und die angenommene Wellenfront aus der punktförmigen Lichtquelle nur dem Schritt 6, und dass der zur Anpassung der Linsenrückfläche **L**₂ zu erfolgende fehlende Schritt jetzt Schritt 5 ist, entsprechend Gl. (5) der genannten Offenlegungsschrift: ${L}_{2} = {D}_{LR} - \left(\frac{{S}_{M} + C}{1 - {τ}_{CL} \left({S}_{M}+C\right)}+{L}_{1}\right) {\left(1-{τ}_{L}\left(\frac{{S}_{M} + C}{1 - {τ}_{CL} \left({S}_{M}+C\right)}+{L}_{1}\right)\right)}^{- 1}$ (1b)

Der zentrale Gedanke der Erfindung besteht darin, zumindest den Längenparameter *d_{LR}* (bzw. *D_{LR}*) aus anderen gemessenen Daten und a-priori-Annahmen zu anderen Freiheitsgraden zu berechnen und nicht wie herkömmlich selbst a-priori anzunehmen. Im Rahmen der vorliegenden Erfindung stellte sich heraus, dass dies deshalb eine bemerkenswerte Verbesserung der individuellen Anpassung bei vergleichsweise geringem Aufwand brachte, weil sich die Wellenfrontdurchrechnung als sehr empfindlich von diesem Längenparameter abhängig herausstellte. Dies bedeutet, dass es erfindungsgemäß ein Vorteil ist, wenn zumindest der Längenparameter *d_{LR}* zu den *df₂(iii) =* 3 Parametern gehört, die berechnet wird. Dieser Parameter ist insbesondere einer direkten Messung schlecht zugänglich, er variiert zwischen unterschiedlichen Probanden stärker und diese Variationen beeinflusst die Abbildung des Auges vergleichsweise stark.

Vorzugsweise stehen die Daten der Vergenzmatrix S_{M} und besonders bevorzugt auch die Daten zu C aus individuellen Messungen zur Verfügung. In einem weiteren bevorzugten Aspekt, der vorzugsweise auch in den folgenden Ausführungsformen berücksichtigt wird, wird bei einer Annahme von Daten zur Linsenrückfläche von einer sphärischen Rückfläche, d.h. einer Rückfläche ohne astigmatische Komponenten ausgegangen.

In einer bevorzugten Ausführungsform der Erfindung liegen also zur Cornea C Messdaten bis zur zweiten Ordnung vor, die den Daten der Flächenbrechwertmatrix C entsprechen. Obgleich diese Werte topographischen Messungen entnommen werden können, sind letztere nicht notwendig. Vielmehr sind topometrische Messungen ausreichend. Diese Situation entspricht dem Fall df₂ = 3 + 6 + 3, wobei insbesondere die Vorderkammertiefe *d_{CL}* einer der sechs a priori festzulegenden Parameter ist.

Soweit keine weiteren individuellen Messungen vorgenommen werden, liegt eine Situation mit df₂ = 3 + 6 + 3 vor. Um *d_{LR}* eindeutig bestimmen zu können, müssen also sechs Parameter aus {L₁, L₂, *d_{L}, d_{CL}}* durch Annahmen bzw. Literaturwerte belegt werden. Die übrigen beiden ergeben sich zusätzlich zu *d_{LR}* aus der Rechnung. In einer bevorzugten Ausführungsform werden die Parameter der Linsenrückfläche, die mittlere Krümmung der Linsenvorderfläche und die beiden Längenparameter *d_{L}* und *d_{CL}* a priori (als vorgegebene Standardwerte) belegt.

In einem für die Erfindung besonders wichtigen Fall ist zusätzlich die Vorderkammertiefe *d_{CL}* also der Abstand zwischen der Hornhaut und Linsenvorderfläche, beispielsweise aus pachymetrischen oder OCT-Messungen bekannt. Damit umfassen die gemessenen Parameter {C, *d_{CL}, S_{M}}.* Diese Situation entspricht dem Fall df₂ = 4 + 5 + 3. Das Problem ist demnach mathematisch noch unterbestimmt, es müssen also fünf Parameter aus {L₁, L₂, *d_{L}}* durch Annahmen bzw. Literaturwerte a priori festgelegt werden. Hierbei handelt es sich in einer bevorzugten Ausführungsform um die Parameter der Linsenrückfläche, die mittlere Krümmung der Linsenvorderfläche und die Linsendicke.

Allein für die Genauigkeit der individuellen Anpassung ist es von Vorteil, möglichst viele Parameter mit individuellen Messungen belegen zu können. In einer bevorzugten Ausführungsform wird dazu zusätzlich die Linsenkrümmung in einem Normalschnitt auf Basis einer individuellen Messung bereitgestellt. Dadurch ergibt sich dann eine Situation gemäß df₂ = 5 + 4 + 3, und es reicht aus, vier Parameter aus *{L_{1yy}, α_{L1},* L₂, *d_{L}}* a priori festzulegen. Auch hier handelt es sich in einer bevorzugten Ausführungsform wieder um die Parameter der Linsenrückfläche und die Linsendicke. Die genaue Durchrechnung wieder weiter unten noch beschrieben.

Insbesondere alternativ zum Normalschnitt der Linsenvorderfläche und besonders bevorzugt zusätzlich zur Vorderkammertiefe kann auch die Linsendicke aus einer individuellen Messung zur Verfügung gestellt werden. Dadurch entfällt die Notwendigkeit, diesen Parameter mit Modelldaten oder Schätzparametern zu belegen *(df₂* = 5 + 4 + 3). Ansonsten gilt das oben bereits ausgeführte. Diese Ausführungsform ist besonders vorteilhaft, wenn ein Pachymeter eingesetzt wird, dessen Messtiefe die Erkennung der Linsenrückfläche erlaubt, nicht aber eine hinreichend sichere Bestimmung der Linsenkrümmungen.

Zusätzlich zur Vorderkammertiefe und einem Normalschnitt der Linsenvorderfläche können in einer bevorzugten Ausführungsform ein (z.B. Messung in zwei Normalschnitten) oder zwei weitere Parameter (Messung beider Hauptschnitte und der Achslage) der Linsenvorderfläche durch eine individuelle Messung erfasst werden. Diese zusätzlichen Informationen können insbesondere auf zwei Arten ausgenutzt werden:
- Aufgabe von a priori Annahmen: Es können ein bzw. zwei der ansonsten a priori getroffenen Annahmen aufgegeben und aus durch Berechnung bestimmt werden. In diesem Fall ergeben sich die Situationen *df₂* = 6 + 3 + 3 bzw. *df₂ =* 7 + 2 + 3. So kann im ersten Fall die mittlere Krümmung der Rückfläche (bei Annahme einer astigmatismusfreien Rückfläche) und im zweiten Fall bei gegebener mittlerer Krümmung der Flächenastigmatismus (incl. Achslage) bestimmt werden. Alternativ kann in beiden Fällen auch die Linsendicke aus den Messungen bestimmt werden.
   Ein derartiges Vorgehen bedarf jedoch im Allgemeinen einer gewissen Umsicht, da verrauschte Messdaten leicht zu einem "Weglaufen" der freigegebenen Parameter führen können. Dadurch kann das Modell insgesamt deutlich schlechter anstatt besser werden. Eine Möglichkeit dies zu verhindern besteht darin, anatomisch sinnvolle Grenzwerte für diese Parameter vorzugeben und die Variation der Parameter auf diesen Bereich zu beschränken. Selbstverständlich können diese Grenzen auch abhängig von den gemessenen Werten vorgegeben werden.
- Verringerung der Messunsicherheit: Werden dagegen weiterhin die gleichen a priori Annahmen getroffen (vorzugsweise also {L₂, *d_{L}}),* liegen die Situationen df₂ = 6 + 4 + 3 bzw. df₂ = 7 + 4 + 3 vor, das System ist also mathematisch überbestimmt.
   Anstelle einer einfachen analytischen Bestimmung von *D_{LR}* gemäß nachfolgender Ausführungen wird *D_{LR}* (und gegebenenfalls der noch fehlende Parameter aus L₁) so bestimmt ("fit"), dass der Abstand zwischen dem sich aus den Gleichungen ergebenden L₁ und dem gemessenen L₁ (bzw. dem um den fehlenden Parameter ergänzten gemessenen L₁) minimal wird. Durch dieses Vorgehen kann - offensichtlich - eine Verringerung der Messunsicherheit erreicht werden.

In einer weiteren bevorzugten Implementierung werden die Vorderkammertiefe, zwei oder drei Parameter der Linsenvorderfläche und die Linsendicke individuell gemessen.

Die Berechnung der übrigen Größen erfolgt dabei analog, wobei die a priori Annahme der Linsendicke durch die entsprechende Messung ersetzt werden kann.

**In** einer weiteren bevorzugten Implementierung werden individuelle Messungen der Vorderkammertiefe, mindestens eines Parameters der Linsenvorderfläche, der Linsendicke und mindestens eines Parameter der Linsenrückfläche bereitgestellt. Hierbei handelt es sich um eine Ergänzung der oben genannten Fälle. Die jeweiligen zusätzlich gemessenen Parameter können analog den schrittweisen Erweiterungen der obigen Abschnitte erfolgen. Diese Fälle sind besonders vorteilhaft, wenn die oben erwähnten Pachymetrieeinheiten, die in einer Ebene, zwei Ebenen oder über die ganze Fläche messen in der Messtiefe entsprechend erweitert und so präzise sind, dass sich die Krümmungsdaten hinreichend genau ermitteln lassen.

Stellt man die bereits oben genannte Formel (1b), nämlich ${L}_{2} = {D}_{LR} - \left(\frac{{S}_{M} + C}{1 - {τ}_{CL} \left({S}_{M}+C\right)}+{L}_{1}\right) {\left(1-{τ}_{L}\left(\frac{{S}_{M} + C}{1 - {τ}_{CL} \left({S}_{M}+C\right)}+{L}_{1}\right)\right)}^{- 1}$ zur Berechnung der Linsenrückfläche bei gegebener Augenlänge (wobei D_{LR}=n_{LR}/d_{LR} die inverse Glaskörperlänge d_{LR}, multipliziert mit dem Brechungsindex *n_{LR}* ist), nach *D_{LR}* um, so erhält man ${D}_{LR} = {L}_{2} + \left(\frac{{S}_{M} + C}{1 - {τ}_{CL} \left({S}_{M}+C\right)}+{L}_{2}\right) \times {\left(1-{τ}_{L}\left(\frac{{S}_{M} + C}{1 - {τ}_{CL} \left({S}_{M}+C\right)}+{L}_{2}\right)\right)}^{- 1}$ zur Berechnung von D_{LR}. Da *D_{LR}* ein Skalar ist, müssen alle Größen zur Berechnung auch skalar genommen werden. Vorzugsweise sind S_{M}, C, L₁ und *L₂* jeweils die sphärischen Äquivalente der Fehlsichtigkeit, der Cornea, der Linsenvorderfläche bzw. der Linsenrückfläche. Hat man so die Glaskörperlänge *d_{LR}* berechnet (und damit die Augenlänge als d_{A}=d_{C}+d_{CL}+d_{L}+d_{LR}), dann kann eine der Flächen bezüglich des Zylinders und der HOA nochmal abgewandelt werden, vorzugsweise die Linsenvorderfläche L₁, um sie konsistent anzupassen.

Zur Berechnung können die Werte des sogenannten Bennett&Rabbetts-Auges für die Brechkräfte der Linsenflächen verwendet werden, die z.B. aus Tabelle 12.1 des Buches "Bennett & Rabbets' Clinical Visual Optics", dritte Auflage, von Ronald B. Rabbetts, Butterworth-Heinemann, 1998, ISBN-10: 0750618175 entnommen werden können. Die oben beschriebene Berechnung führt auf Ergebnisse, die sehr gut mit der Bevölkerungsstatistik verträglich sind, welche besagt, dass kurzsichtige Fehlsichtigkeiten tendenziell zu großen Augenlängen und umgekehrt führen (siehe z.B. C. W. Oyster: "The Human Eye", 1998). Die beschriebene Berechnung ist aber noch genauer, da eine direkte Verwendung der Korrelation aus der Bevölkerungsstatistik zu unphysikalischen Werten für die Augenlinse führen kann, was durch das erfindungsgemäße Verfahren vermieden wird. Umso wichtiger ist eine genaue Kenntnis der Linsenparameter für ein Verfahren, bei dem diese als gegeben angenommen werden. Hatte ein Kunde beispielsweise vor seiner Kataraktoperation eine Fehlsichtigkeit von -10 dpt, dann muss er eine Augenlänge zwischen 28 mm und 30 mm besitzen. Nach der OP würde aber aufgrund seiner Emmetropie auf eine Augenlänge von 24 mm geschlossen werden, welche nicht mit der tatsächlichen Augenlänge übereinstimmt.

Nachfolgend werden bevorzugte Ausführungsbeispiele der Erfindung erläutert.

### Beispiele unter Verwendung von Bayes'scher-Statistik

Ziel des Bayes'sche Statistik benutzenden Verfahrens ist es, möglichst alle zur Verfügung stehenden Informationsquellen über ein Auge bzw. ein Augenpaar in konsistenter Art und Weise zu nutzen, um eine im Lichte dieser Information optimale Korrektion des Auges bzw. der Augen mit einer ophthalmischen Linse (z.B. einem Brillenglas) herzustellen.

In der Regel ist diese Information unvollständig und/oder ungenau, was bisher oft dazu führte, dass ausschließlich vereinfachte Augenmodelle zur Berechnung von ophthalmischen Linsen benutzt werden. Ein solches vereinfachtes Augenmodell ist z.B. ein Auge, welches allein durch seine Refraktion charakterisiert ist, da diese mit einer gewissen Genauigkeit (z.B. mit einem Fehler von ±0.75 dpt im sphärischen Äquivalent) bestimmt werden kann. Möchte man jedoch komplexere Augenmodelle zur Berechnung von ophthalmischen Linsen verwenden, so ist es sinnvoll, z.B. die Information über die Augenlänge, sowie Lage und Krümmung der brechenden Flächen der Cornea und Augenlinse in die Berechnung einfließen zu lassen, diese sollte jedoch nur so stark berücksichtigt werden, wie es im Rahmen ihrer Genauigkeit möglich ist. **In** der Bayes'schen Statistik (siehe z.B. D. S. Sivia: "Data Analysis - A Bayesian Tutorial", Oxford University Press, 2006, ISBN-13: 978-0198568322 oder E. T. Jaynes: "Probability Theory", Cambridge University Press, 2003, ISBN-13: 978-0521592710) wird Information immer in Form von Wahrscheinlichkeitsverteilungen (im Falle von kontinuierlichen Parametern sind es Wahrscheinlichkeitsdichten) beschrieben.

**In** diesem Sinne können einem individuellen Augenmodell mit einem gegebenen Parametersatz eine Wahrscheinlichkeit bzw. Wahrscheinlichkeitsdichte zugeordnet werden. Individuelle Augenmodelle, die mit der vorhandenen Information (z.B. objektive Wellenfrontmessung und Biometrie des Auges) konsistent sind, besitzen eine höhere Wahrscheinlichkeit bzw. Wahrscheinlichkeitsdichte, da z.B. die Propagation und Brechung einer Wellenfront, welche eine Punktlichtquelle auf der Netzhaut nach Austritt aus dem Auge erzeugen würde, im Rahmen der Messgenauigkeit der objektiven Wellenfrontmessung die gemessenen Daten gut wiedergibt, und gleichermaßen die Parameter des individuellen Auges mit der vorhandenen Information über die Biometrie des Auges im Rahmen der z.B. aus der Literatur bekannten Verteilungen übereinstimmen. Individuelle Augenmodelle, die mit der vorhandenen Information nicht konsistent sind, werden dementsprechend niedrigen Wahrscheinlichkeiten bzw. Wahrscheinlichkeitsdichten zugeordnet. Die Wahrscheinlichkeit bzw. Wahrscheinlichkeitsdichte eines individuellen Augenmodells kann als $prob \left({ϑ}_{i}\left|{d}_{i}, I\right.\right)$ geschrieben werden, wobei ϑᵢ die Parameter des individuellen Augenmodells i bezeichnen, und dᵢ die gemessenen Daten sind (diese können z.B. die aktuelle oder die vor einer Augenoperation gemessene Refraktion, die gemessene Form und/oder Brechungseigenschaften der Cornea, die gemessene Augenlänge oder andere am individuellen Auge gemessene Größen umfassen). Mit *I* wird der aktuelle Wissensstand bei Auswertung der Daten, d.h. die vorhandene Hintergrundinformation (z.B. über den Messprozess der Refraktion, die Verteilung der Parameter des individuellen Augenmodells oder anderen damit in Verbindung stehenden Größen in der Bevölkerung) zusammengefasst. Der senkrechte Strich '|' bedeutet dabei, dass die Verteilung der Größen links von '|' bei gegebenen (d.h. festen) Größen rechts von '|' gemeint ist.

Auch die im Messprozess gewonnene Information, in dem die Daten dᵢ gemessen werden, kann als Wahrscheinlichkeitsverteilung der Daten dᵢ bei gegebenen Parametern ϑᵢ des individuellen Augenmodells i aufgefasst werden: $prob \left({d}_{i}\left|{ϑ}_{i}, I\right.\right)$

Die Genauigkeit des Messprozesses spiegelt sich in der Breite der Verteilung wieder: Eine genaue Messung besitzt eine engere Verteilung als eine ungenaue Messung, welche eine breite bzw. breitere Verteilung der Daten dᵢ besitzt.

Möchte man nun die Verteilung der Parameter eines individuellen Auges bei gegebenen Daten und Hintergrundinformation berechnen, so kann folgende Proportionalität verwendet werden: $prob \left({ϑ}_{i}\left|{d}_{i}, I\right.\right) ∝ prob \left({ϑ}_{i}\left|I\right.\right) prob \left({d}_{i}\left|{ϑ}_{i}, I\right.\right)$

Mit dem Term *prob(ϑᵢ*|*I)* wird das Hintergrundwissen über die Parameter des individuellen Augenmodells beschrieben. Dies kann z.B. Information aus der Literatur sein, aber auch Information aus Daten vergangener Messungen. Dies können sowohl Daten derselben Person, für die die ophthalmische Linse gefertigt werden soll, als auch Daten aus Messungen einer Vielzahl von anderen Personen sein.

Die Wahrscheinlichkeit dient hierbei als Konsistenzmaß. Mit den Messungen konsistente Parameterwerte des individuellen Augenmodells sind insbesondere dort zu finden, wo sowohl prob(ϑᵢ|*I)* als auch prob(dᵢ|ϑᵢ, I) hoch sind.

Die Wahrscheinlichkeit bzw. Wahrscheinlichkeitsdichte prob(ϑᵢ|*dⱼ, I)* kann ebenfalls geeignet normiert werden, um die Proportionalität als Gleichung zu schreiben.

Der Term prob(ϑᵢ|*dᵢ, I)* kann auch Parameter der Augenlinse enthalten. So kann ein Teil der Parameter *ϑᵢ* z.B. die Brechkraft der Augenlinse, deren Position und/oder Orientierung im Auge, oder andere Größen wie Brechungsindex und Krümmungen bzw. Form der Flächen umfassen.

Die Augenlinse kann eine natürliche Linse sein. In diesem Fall können Literaturdaten über die Parameter von natürlichen Augenlinsen verwendet werden (z.B. Verteilungen der Krümmungen von Vorder- und/oder Rückfläche, Brechungsindex, etc.).

Ist die Augenlinse eine Intraokularlinse, dürfen die Verteilungen der Parameter von natürlichen Augenlinsen nicht verwendet werden. Stattdessen sind die Parameter der Intraokularlinse zu verwenden, sofern sie individuell bekannt sind. Andernfalls können Verteilungen dieser Parameter aus Literaturstudien von operierten Augen verwendet werden. Sind solche Informationen nicht vorhanden, so kann eine innerhalb sinnvoller Grenzen flache Verteilung gewählt werden. Bei Parameter, die positiv definit sind und Längenskalen definieren (z.B. Krümmungsradien oder Abstände) können auch Verteilungen gewählt werden, die flach im Logarithmus dieser Parameter sind.

Formal sind die Fälle "natürliche Augenlinse" bzw. "Intraokularlinse als Augenlinse" durch unterschiedliche Zustände des Hintergrundwissens *I* zu beschreiben (d.h. *I = I_{NL} bzw. I = I_{IOL}).*

Die Wahrscheinlichkeit bzw. Wahrscheinlichkeitsdichte prob(ϑᵢ|dᵢ, I) kann einen oder mehrere Faktoren besitzen. Jeder Faktor repräsentiert dabei die Information über einen einzigen oder mehrere Parameter des individuellen Augenmodells. So kann z.B. die Verteilung unterschiedlicher unabhängiger Parameter ${ϑ}_{i}^{1}$ und ${ϑ}_{i}^{2}$ aus unterschiedlichen Literaturquellen als Produkt dargestellt werden $prob \left({ϑ}_{i}\left|{d}_{i}, I\right.\right) = prob \left({ϑ}_{i}^{1},{ϑ}_{i}^{2}\left|{d}_{i}, I\right.\right) = prob \left({ϑ}_{i}^{1}\left|{d}_{i}, I\right.\right) prob \left({ϑ}_{i}^{2}\left|{d}_{i}, I\right.\right) .$

Durch *prob(ϑᵢ*|*I)* können ungewollt Parameter des Augenmodells verfälscht werden. So kann z.B. wenn die "wahre" Refraktion als Parameter des individuellen Augenmodells aufgefasst wird, der wahrscheinlichste Wert der "wahren" Refraktion von der gemessenen Refraktion abweichen. Ist dies nicht erwünscht, so sollte eine im entsprechenden Parameter (z.B. Sphärischen Äquivalent der Refraktion) innerhalb sinnvoll gewählter Grenzen (z.B. zwischen -30 dpt und + 20 dpt für das sphärische Äquivalent M, ± 5 dpt für die astigmatischen Komponenten J₀ und J₄₅) eine konstante Verteilung gewählt werden.

Sind Parameter des individuellen Augenmodells oder andere mit den Parametern oder Messdaten zusammenhängende Größen exakt oder mit einer hohen Genauigkeit bekannt, so kann ihre Verteilung als Dirac-Delta-Distribution genähert werden. Die Gleichungen in diesen Parametern oder Größen können beidseitig integriert werden, was gegebenenfalls spätere Berechnungen vereinfacht.

### Beschreibung der Verfahren

Im Folgenden werden zwei mögliche Verfahren zur Berechnung einer ophthalmischen Linse vorgestellt (Bayes A und Bayes B). Im Verfahren Bayes A wird die vorhandene Information genutzt, um ein (einziges) individuelles Augenmodell aufzustellen, mit dessen Hilfe eine ophthalmische Linse berechnet wird, die für dieses Augenmodell optimal ist. Das Augenmodell kann z.B. durch den Parametersatz ${ϑ}_{i}^{max}$*,* der die Wahrscheinlichkeit bzw. Wahrscheinlichkeitsdichte *prob(ϑᵢ*|*dᵢ, I)* maximiert, gegeben bzw. belegt sein. Auch andere Parametersätze können gewählt werden, z.B. der Erwartungswert 〈*ϑᵢ*〉 oder der Median ${ϑ}_{i}^{med}$ der Parameter ϑᵢ bezüglich der Verteilung *prob(*ϑ*ᵢ*|*dᵢ, I).*

Vorteilhafter - jedoch rechnerisch anspruchsvoller - ist das Verfahren Bayes B. Es besitzt im Vergleich zu Bayes A deshalb Vorteile, weil eine Untermenge von individuellen Augenmodellen mit unterschiedlichen Parametersätzen zu ophthalmischen Linsen führen können, welche sehr ähnliche (sogar identische) Eigenschaften haben (z.B. Brechkraft in einem Bezugspunkt der ophthalmischen Linse, oder die Verteilung des Refraktionsdefizits über ein gegebenes Gebiet der ophthalmischen Linse, oder ähnliche Kriterien zur Bestimmung der Güte einer ophthalmischen Linse). Insgesamt kann deshalb eine ophthalmische Linse, die nicht mit dem wahrscheinlichsten individuellen Augenmodell berechnet wurde, eine optimale Korrektion für eine Untermenge von individuellen Augenmodellen darstellen, welche insgesamt eine höhere Wahrscheinlichkeit besitzen als das wahrscheinlichste individuelle Augenmodell. Es ist deshalb vorteilhaft, nach der die Verteilung von Augenmodellen optimal korrigierenden ophthalmischen Linse zu suchen, anstatt nur das wahrscheinlichste individuelle Augenmodell zu bestimmen und dafür eine ophthalmische Linse zu fertigen.

In beiden Verfahren kann somit eine mit der zur Verfügung stehenden Information konsistente ophthalmische Linse (z.B. ein Brillenglas) berechnet werden.

### Verfahren Bayes A

Insbesondere können eine oder mehrere der folgenden Schritte durchgeführt werden:
- Bereitstellen einer initialen Verteilung von Parametern eines Augenmodells (idealerweise als multivariate Wahrscheinlichkeitsverteilung aller Parameter des Augenmodells, gegebenenfalls auch marginale Verteilungen; die Wahrscheinlichkeitsverteilung entspricht der Information über die Verteilung der Parameter des Augenmodells in der Population der Personen);
- Bereitstellen von bereits bekannten (im besten Fall gemessenen) Daten von Eigenschaften eines individuellen Auges (idealerweise mit Wahrscheinlichkeitsverteilung bzw. Messfehler; die Wahrscheinlichkeitsverteilung entspricht der Ungenauigkeit der Messungen)
   Die bereits bekannten Daten können dabei umfassen: bereits bekannte aktuelle subjektive und/oder objektive Refraktion, bereits bekannte frühere subjektive und/oder objektive Refraktion (z.B. vor einer Operation), Wirkung und/oder Form und/oder Position (am wichtigsten ist die axiale Position) bestimmter brechender Flächen des Auges, Größe und/oder Form und/oder Position der Eintrittspupille, Brechungsindex der brechenden Medien, Brechungsindexverlauf in den brechenden Medien, Opazität; ggf. Bestimmung dieser Größen abhängig von der Akkommodation des Auges auf ein Fixationsobjekt (Target) in gegebener naher Entfernung;
- Bestimmen der Parameter eines individuellen Augenmodells basierend auf der initialen Verteilung der Parameter des Augenmodells und den bereits bekannten bzw. gemessenen Daten des individuellen Auges unter Verwendung von Wahrscheinlichkeitsrechnung. Dabei findet idealerweise eine Bestimmung der Wahrscheinlichkeitsverteilung, oder z.B. des Parametersatzes, der ein Maximum der Wahrscheinlichkeitsverteilung kennzeichnet, statt.
   Insbesondere können dabei Berechnungsverfahren wie Markov Chain Monte Carlo, Variational Inference, Maximum Likelihood, Maximum Posterior, oder Particle Filter verwendet werden;
   Ziel ist es hierbei, die Parameter des individuellen Augenmodells auszuwählen, welche sowohl mit der bereitgestellten initialen Verteilung von Augenmodellen als auch mit den bereitgestellten bereits bekannten Daten konsistent sind. Als Konsistenzmaß wird das Produkt der Wahrscheinlichkeit bzw. Wahrscheinlichkeitsdichte der Daten bei gegebenen Parametern des Augenmodells mit der Wahrscheinlichkeit bzw. Wahrscheinlichkeitsdichte der Parameter des Augenmodells verwendet.
- Berechnen / Optimieren / Auswählen einer ophthalmischen Linse, bei der zumindest ein Parameter des individuellen Augenmodells verwendet wird.

Die im ersten Schritt bereitgestellte initiale Verteilung der Parameter von Augenmodellen kann in einer parametrisierten Form vorliegen, z.B. (ggf. multivariate) Normalverteilung, andere Verteilung der exponential-Familie, Cauchy-Verteilung, Dirichlet-Prozess, etc., oder als ein Satz von Samples, d.h. ein oder mehrere (ggf. multidimensionale) Datensätze. Ist die initiale Verteilung der Parameter von Augenmodellen parametrisiert, so nennt man die Parameter dieser Verteilung "Hyperparameter".

Der dritte Schritt (d.h das Bestimmen der Parameter eines individuellen Augenmodells) kann dabei die Bestimmung einer multivariaten Wahrscheinlichkeitsverteilung beinhalten, welche sowohl die Parameter des individuellen Augenmodells als auch die Hyperparameter der initialen Verteilung der Parameter des Augenmodells beinhaltet. Um daraus die Verteilung der Parameter des individuellen Augenmodells zu berechnen, muss die Verteilung marginalisiert werden, d.h. es wird über die Hyperparameter integriert. Die Integrale können mit gängigen numerischen Verfahren (z.B. mittels Markov Chain Monte Carlo oder Hybrid Monte Carlo) und/oder analytischen Verfahren gelöst werden. Die Wahrscheinlichkeit bzw. die Wahrscheinlichkeitsdichte der Parameter des Augenmodells kann in diesem Fall mit Hilfe folgender Gleichung berechnet werden: $\begin{matrix}prob \left({ϑ}_{i}\left|{d}_{i}, I\right.\right) ∝ prob \left({d}_{i}\left|{ϑ}_{i}, I\right.\right) \int dλ prob \left({ϑ}_{i},λ\left|I\right.\right) \\ = prob \left({d}_{i}\left|{ϑ}_{i}, I\right.\right) \int dλ prob \left({ϑ}_{i},λ\left|I\right.\right) prob \left(λ\left|I\right.\right)\end{matrix}$

Dabei bezeichnet *prob(*ϑᵢ|λ, *I)* die Wahrscheinlichkeit bzw. Wahrscheinlichkeitsdichte, die Parameter des individuellen Augenmodells ϑᵢ in der durch die Hyperparameter λ charakterisierten Population zu finden. Die Integrale sind über die gesamten Definitionsbereiche aller Hyperparameter λ auszuführen.

### Verfahren Bayes B

Alternativ oder zusätzlich zum Verfahren Bayes A können eine oder mehrere der folgenden Schritte durchgeführt werden:
- Bereitstellen der Verteilung von zumindest einem Parameter eines individuellen Augenmodells;
- Berechnen der Wahrscheinlichkeitsverteilung der Parameter von virtuellen ophthalmischen Linsen oder Berechnen eines Ensembles von ophthalmischen Linsen durch Optimierung / Berechnung / Auswählen von virtuellen ophthalmischen Linsen unter Verwendung zumindest eines Parameters des individuellen Augenmodells;
- Fertigen einer ophthalmischen Linse, mit dem Ziel, dass die gefertigten Parameter der ophthalmischen Linse die Parameter der virtuellen ophthalmischen Linse mit der höchsten Wahrscheinlichkeit erreichen.

Im ersten Schritt kann die analog der Schritte 1 bis 3 des Verfahrens Bayes A berechnete Verteilung bereitgestellt werden. Im zweiten Schritt werden die wahrscheinlichsten Parameter Lᵢ der ophthalmischen Linse bestimmt, d.h. anhand der Wahrscheinlichkeitsverteilung bzw. Wahrscheinlichkeitsdichte $\begin{matrix}prob \left({L}_{i}\left|{d}_{i}, I\right.\right) = \int {dϑ}_{i} prob \left({ϑ}_{i},{L}_{i}\left|{d}_{i}, I\right.\right) = \int {dϑ}_{i} prob \left({L}_{i}\left|{ϑ}_{i}, I\right.\right) prob \left({ϑ}_{i}\left|{d}_{i}, I\right.\right) \\ = \int {dϑ}_{i} δ \left({L}_{i}-L\left({ϑ}_{i}\right)\right) prob \left({ϑ}_{i}\left|{d}_{i}, I\right.\right)\end{matrix}$ werden die Parameter der ophthalmischen Linse ${L}_{i}^{max}$ bestimmt, die prob(Lᵢ|dᵢ, I) maximieren. Dabei bezeichnet Lᵢ zunächst die Parameter einer beliebigen ophthalmischen Linse, und im Falle von Lᵢ = *L(ϑᵢ)* die Parameter der ophthalmischen Linse, die bei einer Optimierung einer ophthalmischen Linse mit Hilfe eines individuellen Augenmodells mit den Parametern ϑᵢ entsteht. Die Dirac-Delta-Distribution wird mit δ(.) bezeichnet.

Die Parameter der ophthalmischen Linse können z.B. Pfeilhöhen, Brechkraft an einem Bezugspunkt der ophthalmischen Linse, Brechkraftverteilung über ein Gebiet der ophthalmischen Linse, Refraktionsfehler an einem Bezugspunkt der ophthalmischen Linse, oder die Verteilung der Refraktionsfehler über ein Gebiet der ophthalmischen Linse sein.

Dabei ist es wichtig, dass die Funktion L(ϑᵢ) nichtlinear sein kann, und deshalb das Maximum der Wahrscheinlichkeitsdichte prob(ϑᵢ|dᵢ, I) (bezüglich ϑᵢ) mit *L(*ϑ*ᵢ)* nicht notwendigerweise auf das Maximum der Wahrscheinlichkeitsdichte prob(Lᵢ|dᵢ, I) abgebildet wird.

Ist die Funktion L(ϑᵢ) stückweise invertierbar, so kann die oben beschriebene Gleichung auch mit Hilfe von partieller Integration gelöst werden. Es bieten sich auch andere Methoden an wie z.B. numerische Methoden wie Particle Filter, Markov Chain Monte Carlo, oder Methoden der parametrischen Inferenz, mit welchen eine Verteilung der Parameter der ophthalmischen Linse Lᵢ berechnet werden können.

Sowohl im Verfahren Bayes A als auch im Verfahren Bayes B ergibt sich unabhängig sowohl von der Anzahl und Art der durch Messung bekannten Größen (d.h. der Daten dᵢ und der Form der Likelihood prob(dᵢ|ϑ*ᵢ, I))* als auch von der Anzahl und Art der Parameter des Augenmodells ϑᵢ immer ein konsistentes Augenmodell (Verfahren Bayes A und B) und ggf. eine zu dem Ensemble der möglichen konsistenten Augenmodelle passende Wahl der Parameter der ophthalmischen Linse (Verfahren Bayes B).

### Beispiele auf der Basis von Wahrscheinlichkeitsbetrachtungen zur Auflösung von Inkonsistenzen

### Hintergrund zum Maximum-Likelihood-Ansatz

### Prinzipielle Vorgehensweise

Die Ausgangssituation besteht darin, dass insgesamt *N* Parameter *xᵢ, 1 ≤ i ≤ N* eines Modells belegt werden sollen und folgende Informationen vorliegen:
- Mittelwerte *µᵢ* , Standardabweichungen *σᵢ* und Korrelationskoeffizienten *ρᵢⱼ* (mit *1 ≤ i, j ≤ N)* dieser *N* Parameter in der Bevölkerung;
- Entweder liegen keine Messwerte vor *(k = 0),* oder zu *k* dieser Parameter (wobei 1 ≤ *k ≤ N)* liegen Messwerte ${x}_{i}^{mess}$*,* 1 ≤ *i* ≤ *k* vor. Die Wahrscheinlichkeitsverteilung für den Messwert ${x}_{i}^{mess}$ jedes Parameters x, wird durch eine Zufallsgröße *Xᵢ* beschrieben. Bevorzugt liegt zu jedem Messwert ein Zuverlässigkeitsmaß vor, z.B. eine Standardabweichung ${σ}_{i}^{mess}$ der Zufallsgröße *X₁, 1 ≤ i ≤ k*
- Zu *q = N - k* dieser Parameter liegen keine Messwerte vor;
- Insgesamt sind nur K der N Parameter unabhängig, weil das Modell Konsistenzbedingungen verlangt, die sich durch *Q = N - K* Zwangsbedingungen ausdrücken lassen.

Beispielhaft können vorliegen:
- Beispiel ohne HOA
   - Parameter (*N* = 15 ) : Cornea (SZA), Linsenvorderfläche (SZA), Linsenrückfläche (SZA), Fehlsichtigkeit (SZA), Augenlänge, Linsendicke, Vorderkammertiefe;
   - Messdaten (k = 13 ): Cornea (SZA), Linsenvorderfläche (SZA), Linsenrückfläche (SZA), Fehlsichtigkeit (SZA), Vorderkammertiefe;
   - Zwangsbedingungen (*Q* = 3 ): Fehlsichtigkeit (SZA) *=* theor. Fehlsichtigkeit (SZA) (berechnet aus belegtem Augenmodell);
- Beispiel mit HOA (bis radiale Ordnung *n = 6 )*
   - Parameter ( *N = 103* ): Cornea (SZA+HOA), Linsenvorderfläche (SZA+HOA), Linsenrückfläche (SZA+HOA), Fehlsichtigkeit (SZA+HOA), Augenlänge, Linsendicke, Vorderkammertiefe;
   - Messdaten *( k = 101 ):* Cornea (SZA+HOA), Linsenvorderfläche (SZA+HOA), Linsenrückfläche (SZA+HOA), Fehlsichtigkeit (SZA+HOA), Vorderkammertiefe;
   - Zwangsbedingungen (Q = 25): Fehlsichtigkeit (SZA+HOA) *=* theor. Fehlsichtigkeit (SZA+HOA) (berechnet aus belegtem Augenmodell).

**Das zu lösende** Grundproblem besteht darin, dass bei Messwerten, die vom Bevölkerungsmittel abweichen, entschieden werden muss, ob die Messung verworfen werden muss (z.B. wenn sie unplausibel ist), oder übernommen werden muss. Wenn alle Messwerte für sich genommen zwar plausibel sind, aber gegen eine der Konsistenzbedingungen verstoßen, dann dürfen sie gar nicht alle übernommen werden. Vielmehr muss dann ein Ausgleich gesucht werden zwischen den verschiedenen Messwerten: Diejenigen, die eine sehr hohe Mess-Zuverlässigkeit besitzen, sollten zumindest nahezu beibehalten werden, während unsichere Messwerte eher angepasst werden. Vorzugsweise werden aus den bekannten Informationen die bestmöglichen Werte für alle N Parameter ermittelt.

Der Erfindungsgedanke beruht insbesondere darauf, dass annahmehalber die N Parameter bestimmte (unbekannte aber zunächst feste) Werte besitzen. Unter dieser Annahme wird im Lichte der oben genannten Informationen (statistische Größen aus der Bevölkerung, Zuverlässigkeitsmaße der Messungen) die bedingte Wahrscheinlichkeitsdichte ${P}^{bed} \left(\left.{X}_{1},…,{X}_{N}\right|{x}_{1},…,{x}_{N}\right)$ (1) für den Ausgang der Messungen aufgestellt, wobei *X₁,...,X_{N}* die Zufallsgrößen sind, die bei festen gegebenen wahren Werten x₁,...,x_{N} variieren. Anschließend wird die Wahrscheinlichkeit für die beobachteten Messwerte *P^{par}* quantifiziert, indem die Funktion *P^{bed}* für die *k* Messwerte ausgewertet und für die restlichen *q = N - k* (nicht gemessenen) Parameter marginalisiert wird: ${P}^{par} \left({x}_{1},…,{x}_{N}\right) : = \int {P}^{bed} \left({X}_{1}={x}_{1}^{mess},…,{X}_{k}={x}_{k}^{mess},{X}_{k + 1},…,{X}_{N}\left|{x}_{1},…,{x}_{N}\right.\right) {dX}_{k + 1} ⋯ {dX}_{N}$ (2)

Diese Wahrscheinlichkeitsdichte wird als Funktion *P^{par}(x₁,...,x_{N})* der angenommenen *N* Parameter x₁,...,x_{N} aufgefasst. Diejenigen *N* Parameterwerte, für welche diese Funktion ein Maximum annimmt, gelten dann bevorzugt als die bestmöglichen Werte (Maximum-Likelihood-Ansatz): $\frac{\partial {P}^{par} \left({x}_{1},…,{x}_{N}\right)}{\partial {x}_{i}} = 0 , 1 \leq i \leq N$ (3)

**Alternativ** zur Marginalisierung in GI.(2) können die N Parameterwerte auch dadurch festgelegt werden, dass die letzten *q=N-k* Parameter *xₖ₊₁,...,x_{N}* gleich den Mittelwerten der Grundgesamtheit gesetzt werden, ${x}_{i} = μ , k + 1 \leq i \leq N$ (4) während die die ersten k Parameter x₁,...,xₖ so bestimmt werden, dass ihre Erwartungswerte gleich den Messwerten sind: $\left〈\left.{X}_{i}\right|{x}_{1},…,{x}_{k},{x}_{k + 1}={μ}_{k + 1},…,{x}_{N}={μ}_{N}\right〉 = {x}_{i}^{mess}$ (5)

Als weitere Alternative können statt der Maximumsbildung nach Gleichung (3) oder der Erwartungswertbildung nach Gleichung (5) auch die Mediane als Kriterium verwendet werden.

Als ebenso weitere Alternative können die Maximumsbildung nach Gleichung (3) und die Erwartungswertbildung nach Gleichung (5) sowie die Medianbestimmung auch beliebig kombiniert werden, um die N Parameterwerte zu bestimmen.

### Hintergrund zum Maximum-Posterior-Ansatz

Das Vorwissen über die Bevölkerung wird durch die Verteilung *P^{pop}(x₁,...,x_{N})* beschrieben, die dem Prior der Bayes'schen Beschreibung entsprechen kann. Die gesamte Wahrscheinlichkeitsdichte, die sowohl die Verteilung von Messwerten als auch von Modellparametern beschreibt, ist damit durch die Verteilungsfunktion ${P}^{ges} \left({X}_{1},…,{X}_{k},{x}_{1},…,{x}_{N}\right) = {P}^{mess} \left(\left.{X}_{1},…{X}_{k}\right|{x}_{1},…,{x}_{N}\right) \times {P}^{pop} \left({x}_{1},…,{x}_{N}\right)$ (6) gegeben, die bis auf eine Konstante dem Posterior der Bayes'schen Beschreibung entsprechen kann. Deshalb wird auf diesen Ansatz auch als Maximum-Posterior Bezug genommen.

Bevorzugt ist *P^{pop}* durch die multivariate Normalverteilung ${P}^{pop} \left(x\right) = \sqrt{\frac{det \left({C}^{- 1}\right)}{{\left(2π\right)}^{N}}} Exp \left(-\frac{1}{2}{\left(x-μ\right)}^{T}{C}^{- 1}\left(x-μ\right)\right)$ (7) beschrieben, wobei µ der Vektor der Mittelwerte und C die Kovarianzmatrix sind: $μ = \left(\begin{matrix}{μ}_{1} \\ {μ}_{2} \\ ⋮ \\ {μ}_{n}\end{matrix}\right) , C = \left(\begin{matrix}{σ}_{1}^{2} & {ρ}_{12} {σ}_{1} {σ}_{2} & ⋯ & {ρ}_{1 n} {σ}_{1} {σ}_{n} \\ {ρ}_{12} {σ}_{1} {σ}_{2} & {σ}_{2}^{2} & & {ρ}_{2 n} {σ}_{2} {σ}_{n} \\ ⋮ & & ⋱ & ⋮ \\ {ρ}_{1 n} {σ}_{1} {σ}_{n} & {ρ}_{2 n} {σ}_{2} {σ}_{n} & ⋯ & {σ}_{n}^{2}\end{matrix}\right)$ (7a)

**Die Messung** wird durch die Verteilung *P^{mess}(X₁,...,Xₖ* I *x₁,...,x_{N})* beschrieben. Bevorzugt sind die Messungen unabhängig ${P}^{mess} \left(\left.{X}_{1},…,{X}_{k}\right|{x}_{1},…,{x}_{N}\right) = {P}_{1}^{mess} \left(\left.{X}_{1}\right|{x}_{1}\right) ⋯ {P}_{k}^{mess} \left(\left.{X}_{k}\right|{x}_{k}\right)$ (8)

Die gesamte Verteilungsfunktion *P^{ges}* (bis auf den Vorfaktor der Posterior) ist dann gegeben durch ${P}^{ges} \left({X}_{1},…,{X}_{k};{x}_{1},…,{x}_{N}\right) = {P}_{1}^{mess} \left(\left.{X}_{1}\right|{x}_{1}\right) ⋯ {P}_{k}^{mess} \left(\left.{X}_{k}\right|{x}_{k}\right) \times {P}^{pop} \left({x}_{1},…,{x}_{N}\right)$ (8a)

Ganz besonders bevorzugt ist jede der Messungen normalverteilt mit Erwartungswert *xᵢ* und Standardabweichung ${σ}_{i}^{meas}$ ${P}_{i}^{mess} \left({X}_{i}\left|{x}_{i}\right.\right) = \frac{1}{\sqrt{2 π} {σ}_{i}^{mess}} Exp \left(-\frac{1}{2 {\left({σ}_{i}^{mess}\right)}^{2}}{\left({X}_{i}-{x}_{i}\right)}^{2}\right)$ (8b)

Die gesamte Verteilungsfunktion *P^{ges}* ist dann dadurch gegeben, dass die Normalverteilung aus Gl.(8b) in Gl.(8a) eingesetzt wird.

Der Erfindungsgedanke besteht darin, *P^{ges}* als Funktion der Parameter x₁,...,x_{N} zu maximieren. Um das Maximum-Posterior-Kriterium anzuwenden, bildet man bevorzugt die Ableitungen des Logarithmus $\begin{matrix}\frac{\partial }{\partial {x}_{i}} log {P}^{mess} \left({X}_{1},…,{X}_{k},{x}_{1},…,{x}_{N}\right) \\ = \frac{\partial }{\partial {x}_{i}} \left[log {P}^{ges}\left({X}_{1},…,{X}_{k}\left|{x}_{1},…,{x}_{N}\right.\right)+log {P}^{pop}\left({x}_{1},…,{x}_{N}\right)\right] \\ = \frac{\partial {P}^{mess} \left({X}_{1},…,{X}_{k}\left|{x}_{1},…,{x}_{N}\right.\right) / \partial {x}_{i}}{{P}^{mess} \left({X}_{1},…,{X}_{k}\left|{x}_{1},…,{x}_{N}\right.\right)} + \frac{\partial {P}^{pop} \left({x}_{1},…,{x}_{N}\right)}{{P}^{pop} \left({x}_{1},…,{x}_{N}\right)} , 1 \leq i \leq N\end{matrix}$ (9) $\begin{matrix}\frac{\partial }{\partial {x}_{i}} log {P}^{ges} \left({X}_{1},…,{X}_{k},{x}_{1},…,{x}_{N}\right) \\ = \frac{\partial }{\partial {x}_{i}} \left[log {P}_{1}^{mess}\left({X}_{1}\left|{x}_{1}\right.\right)+…+log {P}_{k}^{mess}\left({X}_{k}\left|{x}_{k}\right.\right)+log {P}^{pop}\left({x}_{1},…,{x}_{N}\right)\right] \\ = \frac{\partial {P}_{i}^{mess} \left({X}_{i}\left|{x}_{1}\right.\right) / \partial {x}_{i}}{{P}_{i}^{mess} \left({X}_{i}\left|{x}_{i}\right.\right)} + \frac{\partial {P}^{pop} \left({x}_{1},…,{x}_{N}\right)}{{P}^{pop} \left({x}_{1},…,{x}_{N}\right)} , 1 \leq i \leq N\end{matrix}$ (10)

Sind die Verteilungen, wie besonders bevorzugt, multivariat normalverteilt, stell Gleichung (9) bzw. Gleichung (10) ein lineares Gleichungssystem mit N Gleichungen und N Variablen dar, das nach x₁,...,x_{N} aufgelöst werden kann.

### a) Keine Zwangsbedingungen

Liegen keine Zwangsbedingungen vor, und ist Gleichung (9) lösbar, dann ergeben sich eindeutige Lösungen für x₁,...,x_{N} . Sind die Verteilungen, wie besonders bevorzugt, multivariat normalverteilt, und falls die Messunsicherheiten deutlich kleiner sind als die Variationsbreiten der Bevölkerung, ${σ}_{i}^{mess} ≪ {σ}_{i}$, 1 ≤ i ≤ k , dann ergeben sich die Lösungen $\begin{matrix}{x}_{i} \approx {x}_{i}^{mess} , & 1 \leq i \leq k \\ {x}_{i} \approx {μ}_{i} + Δ {x}_{i} , & k + 1 \leq i \leq N\end{matrix}$ (11) d.h. für alle Parameter, für die Messwerte vorliegen, glaubt man im Wesentlichen den Messwerten, und für die übrigen Werte erhält man die Mittelwerte *µᵢ* der Bevölkerung plus Verschiebungen △xᵢ aufgrund der Korrelationen mit den gemessenen Werten. Eine Ausführungsform der Erfindung besteht dann darin, für 1 ≤ *i* ≤ *k* die Messwerte direkt zu übernehmen und deren leichte Verschiebung aufgrund der unterliegenden Grundgesamtheit zu vernachlässigen.

### b) Zwangsbedingungen

Liegen Zwangsbedingungen zwischen den Parametern vor, dann genügt auch jedes Mitglied der Population diesen Zwangsbedingungen. Zwangsbedingungen können beschrieben werden durch ${f}_{j} \left({x}_{1},…,{x}_{N}\right) = 0 , 1 \leq j \leq Q ⇔ f \left({x}_{1},…,{x}_{N}\right) = 0$ (12) d.h. durch Q Funktionen *fⱼ* der Parameter x₁,...,x_{N} , die sich in einen Vektor f zusammenfassen lassen, und die per Forderung gleich Null sein sollen. Bevorzugt sind die Funktionen *fⱼ* linear oder lineare Näherungen an die gegebenen Zwangsbedingungen.

Im bevorzugten Fall multivariater Verteilungen hat das zur Folge, dass die Spalten der Kovarianzmatrix linear abhängig sind, also dass die Kovarianzmatrix einen Rang r < *N* besitzt und daher nicht mehr invertierbar ist. Eine Verteilungsdichte *P^{pop}(x₁,...,x_{N})* lässt sich dann gar nicht mehr angeben.

Eine Möglichkeit in der Praxis besteht darin, die Kovarianzmatrix C zu regularisieren, indem man eine oder mehrere der in ihr enthaltenen Korrelationen *ρᵢⱼ* oder Standardabweichungen *σᵢ* um *ε* verschiebt und dann *x₁,...,x_{N}* bestimmt. Die so erhaltenen Lösungen erfüllen dann für ε → 0 automatisch die Zwangsbedingungen.

Im Rahmen der Erfindung ist erkannt worden, dass dieses Verfahren aber Nachteile birgt. Zum einen muss man die Verteilung in der Bevölkerung kennen, und zum anderen ist ihre Kovarianzmatrix entweder singulär oder schlecht konditioniert. Recherchiert man nämlich die Korrelationen *ρᵢⱼ* und Standardabweichungen σᵢ, dann genügen kleine Ungenauigkeiten in den Angaben oder unvollständige Informationen dafür, dass die Kovarianzmatrix regulär ist, aber eventuell dann numerisch instabile Lösungen für die gesuchten Parameter erzeugt.

Es wurde jedoch im Rahmen der Erfindung erkannt, dass dieses Problem umgangen werden kann, indem man entweder auf Basis der Verteilung (Maximum-Likelihood-Ansatz) ${P}^{mess} \left({x}_{1},…,{x}_{N}\right) : = {P}^{mess} \left({X}_{1}={x}_{1}^{mess},…,{X}_{k}={x}_{k}^{mess}\left|{x}_{1},…,{x}_{N}\right.\right)$ (13a) oder auf Basis der Verteilung (Maximum-Posterior-Ansatz) ${P}^{ges} \left({x}_{1},…,{x}_{N}\right) : = {P}^{ges} \left({X}_{1}={x}_{1}^{mess},…,{X}_{k}={x}_{k}^{mess};{x}_{1},…,{x}_{N}\right)$ (13b) arbeitet. Hierzu kann vorzugsweise das Substitutionsverfahren verwendet werden.

### Maximum-Likelihood-Verfahren mit Zwangsbedingungen und Substitution

Man nimmt die ersten K Parameter x^{u} *:=(x₁,...,x_{K})^{T}* als unabhängig an und löst Gleichung (12) nach den restlichen *Q = N - K* abhängigen Parametern x*^{a} := (x_{K+1},...,x_{N})^{T}* auf, die man dann als Funktion *x^{a}(x^{u})* der unabhängigen Parameter x^{u} auffassen und in f substituieren kann. Dann lauten die Zwangsbedingungen als Funktion von x" : $f \left({x}^{u},{x}^{a}\left({x}^{u}\right)\right) = 0$ (14).

**Dabei ist** es im Rahmen der Erfindung nicht erforderlich, die Funktion x^{a}(x^{u}) explizit zu kennen. Im Rahmen der Erfindung benötigt man nur deren Jacobi-Matrix $\partial {x}^{a} / \partial {x}^{u} : = \partial {x}_{i}^{a} / \partial {x}_{j}^{u}$*, 1* ≤ *i* ≤ *Q, 1* ≤ *j ≤ K,* und diese ist nach dem Satz von der impliziten Funktion gegeben durch $\frac{\partial {x}^{a}}{\partial {x}^{u}} = - {\left(\frac{\partial f}{\partial {x}^{a}}\right)}^{- 1} \frac{\partial f}{\partial {x}^{u}}$ (15) wobei *∂f*/*∂x^{a}* die quadratische Jacobi-Matrix von f bezüglich x^{a} ist und ∂f/∂x^{u} die i.a. rechteckige Jacobi-Matrix von f bezüglich x^{u}. Zu maximieren ist die Wahrscheinlichkeitsdichte *P^{mess}(x^{u},x^{a}(x^{u}))* als Funktion von x^{u}, also $\begin{matrix}\frac{\partial }{\partial {x}^{u}} {P}^{mess} \left({x}^{u},{x}^{a}\left({x}^{u}\right)\right) = \frac{\partial {P}^{mess}}{\partial {x}^{u}} + \frac{\partial {P}^{mess}}{\partial {x}^{a}} \frac{\partial {x}^{a}}{\partial {x}^{u}} \\ = \frac{\partial {P}^{mess}}{\partial {x}^{u}} - \frac{\partial {P}^{mess}}{\partial {x}^{a}} {\left(\frac{\partial f}{\partial {x}^{a}}\right)}^{- 1} \frac{\partial f}{\partial {x}^{u}} \\ = 0\end{matrix}$ (16).

Das Gleichungssystem (16) sind *K* Gleichungen, die nach den für K unabhängigen Parametern x^{u} aufgelöst werden können. Die restlichen Parameter *x^{a}* erhält man durch Einsetzen in den Zusammenhang x^{a}(x^{u}).

### Maximum-Likelihood-Verfahren mit Zwangsbedingungen und Lagrange-Parametern

Alternativ kann im Rahmen der Erfindung der ganze Parametersatz als unabhängig aufgefasst werden, wenn man statt der Funktion *P^{mess}(x₁,...,x_{N})* die Lagrange-Funktion ${P}^{mess , Lagrange} \left({x}_{1},…,{x}_{N},λ\right) = {P}^{mess ,} \left({x}_{1},…,{x}_{N}\right) + λ f \left({x}_{1},…,{x}_{N}\right)$ (17) maximiert, wobei λ *=* (λ₁,...,λ_{Q}) ein Q-dimensionaler Vektor aus Lagrange-Multiplikatoren ist. Zu maximieren ist dann durch Nullsetzen der *N + Q* Ableitungen $\begin{matrix}\frac{\partial }{\partial {x}_{i}} {P}^{mess , Lagrange} \left({x}_{1},…,{x}_{N},λ\right) = 0 , & 1 \leq i \leq N \\ \frac{\partial }{\partial {λ}_{j}} {P}^{mess , Lagrange} \left({x}_{1},…,{x}_{N},λ\right) = 0 , & 1 \leq j \leq Q\end{matrix}$ (18).

Auflösen von Gleichung (18) nach den *N + Q* Unbekannten (x₁,...,x_{N}) und *(*λ₁*,...,*λ_{Q}*)* führt auf die Lösungen für die Parameter.

Anstatt die Zwangsbedingungen mit Substitution oder Langrange-Parametern zu behandeln, kann man alternativ (zum Beispiel im Fall von lokal verschwindenden Gradienten der zu maximierenden Funktion) einen gedämpften Hamilton-Formalismus mit Reibungsterm verwenden.

Analog kann das Verfahren der GI.(16) bis (18) auf die Funktion *P^{ges}*(x₁,...,x_{N}) statt *P^{mess}(x₁,...,x_{N})* angewendet werden und stellt dann ein Maximum-Posterior-Verfahren mit Zwangsbedingungen dar.

### Ausführungsbeispiel mit konkreten beispielhaften Zahlenwerten

Als Ausgangssituation wird einfachheitshalber ein Auge betrachtet, das rotationssymmetrisch um die optische Achse ist und daher weder eine zylindrische Verordnung, noch eine zylindrische Cornea, noch zylindrische Linsenflächen aufweist. Beispielhafte Werte bzw. Parameter sind im Einzelnen vor der IOL-Operation: $\begin{matrix}S = - 7.0 dpt ; & Fehlsichtigkeit \left(gemessen\right) \\ C = 41.2 dpt ; & Brechkraftder Cornea \left(gemessen\right) \\ {L}_{1} = 7.82 dpt ; & Brechkraftder Linsenvorderfläche \left(Literatur\right) \\ {L}_{2} = 13.28 dpt ; & Brechkraftder Linsenrückfläche \left(Literatur\right) \\ {d}_{CL} = 3.6 mm ; & Vorderkammertiefe \left(gemessen\right) \\ {d}_{L} = 3.7 mm ; & Linsendicke \left(Literatur\right) \\ {n}_{CL} = 1.336 ; & Brechungs - Index Vorderkammer \left(Literatur\right) \\ {n}_{L} = 1.422 ; & Brechungs - Index Linse \left(Literatur\right) \\ {n}_{LR} = 1.336 ; & Brechungs - Index Glaskörper \left(Literatur\right)\end{matrix}$ (19).

Nach der IOL-Operation werden z.B. folgende Werte bzw. Parameter übermittelt: $\begin{matrix}{S}_{IOL}^{mess} = 0.0 dpt ; & Fehlsichtigkeit \left(gemessen\right) \\ {L}_{2 , IOL}^{mess} = 3.2 dpt ; & Brechkraftder Linsenrückfläche \left(Herstellerangabe\right)\end{matrix}$ (20).

Alle anderen Parameter nach der IOL-Operation sind der Einfachheit halber als unverändert angenommen.

### Mit Hilfe der Gleichung

${D}_{LR} = {L}_{2} + \left({L}_{1}+\frac{S + C}{1 - {τ}_{CL} \left(S+C\right)}\right) {\left(1-{τ}_{L}\left({L}_{1}+\frac{S + C}{1 - {τ}_{CL} \left(S+C\right)}\right)\right)}^{- 1}$ (21) kann man die reduzierte inverse Glaskörperlänge *(D_{LR} = n_{LR}* /*d_{LR},* wobei *d_{LR}* die Glaskörperlänge ist; weiter sind *τ_{CL} = d_{cL}* / *n_{CL}* sowie *τ_{L} = d_{CLL}* / *n_{L})* berechnen und damit die Augenlänge *d_{A} = d_{CL} +d_{L} +d_{LR}.* Glaskörperlänge und Augenlänge haben einen so direkten Zusammenhang, dass im Folgenden die Glaskörperlänge statt der Augenlänge betrachtet werden kann.

Wendet man Gleichung (21) auf die Situationen vor und nach der OP an, so erhält man formal vor der IOL-Operation ${D}_{LR} = 64.69 dpt$ (22a) und formal nach der IOL-Operation ${D}_{LR , IOL}^{mess} = 65.65 dpt$ (22b).

Da sich aber die Glaskörperlänge durch die OP nicht geändert haben kann, besteht hiermit eine Inkonsistenz, die im Rahmen der vorliegenden Erfindung aufgelöst werden kann.

Um ein möglichst einfaches Beispiel zu wählen, kann als Ausgangssituation der Fall betrachtet werden, dass es in der Grundpopulation keine Schwankungsbreiten und keine Korrelationen gibt, und dass nur die nachträglich gemessene Fehlsichtigkeit sowie die IOL selbst mit Unsicherheiten behaftet sind: $\begin{matrix}{σ}_{S , IOL}^{mess} = 0.25 dpt ; & Fehlsichtigkeit \left(Std.abw. des Messeverfahrens\right) \\ {σ}_{L 2 , IOL}^{mess} = 0.4 dpt ; & Brechkraftder Linsenrückfläche \left(Herstellertoleranz\right)\end{matrix}$ (23).

Nun können im Rahmen der Erfindung die wahren Werte von *S_{IOL},L_{2,IOL}* ermittelt werden, die erwartungsgemäß beide von Gleichung (20) abweichen werden.

Im beispielhaften Fall ist *P^{pop}* ≡ 1, und die aufgrund der Messungen zunächst anzunehmende Wahrscheinlichkeitsdichte für die Verteilung von *S_{IOL},L_{2,IOL}* ist $\begin{matrix}{P}^{mess} \left(\left.{S}_{IOL}^{mess}, {L}_{2 , IOL}^{mess}\right|{S}_{IOL},{L}_{2 , IOL}\right) = {P}_{1}^{mess} \left(\left.{S}_{IOL}^{mess}\right|{S}_{IOL}\right) ⋯ {P}_{k}^{mess} \left(\left.{L}_{2 , IOL}^{mess}\right|{L}_{2 , IOL}\right) \\ = \frac{1}{2 {πσ}_{S , IOL}^{mess} {σ}_{L 2 , IOL}^{mess}} \times \\ Exp \left(-\frac{1}{2 {\left({σ}_{S , IOL}^{mess}\right)}^{2}}{\left({S}_{IOL}-{S}_{IOL}^{mess}\right)}^{2}\right) \times \\ Exp \left(-\frac{1}{2 {\left({σ}_{L 2 , IOL}^{mess}\right)}^{2}}{\left({L}_{2 , IOL}-{L}_{2 , IOL}^{mess}\right)}^{2}\right)\end{matrix}$ (24).

Nun gilt aber die Zwangsbedingung, dass *S_{IOL},L_{2,IOL}* nach Einsetzen in Gleichung (21) denselben Wert für *D_{LR}* ergeben müssen wie vor der OP. Daher lautet die Gleichung für die Zwangsbedingung ${D}_{LR} = {L}_{2 , IOL} + \left({L}_{1}+\frac{{S}_{IOL} + C}{1 - {τ}_{CL} \left({S}_{IOL}+C\right)}\right) {\left(1-{τ}_{L}\left({L}_{1}+\frac{{S}_{IOL} + C}{1 - {τ}_{CL} \left({S}_{IOL}+C\right)}\right)\right)}^{- 1}$ (25) **was aufgelöst** nach *L_{2,JOL}* als Funktion von *S_{IOL}* ergibt: ${L}_{2 , IOL} \left({S}_{IOL}\right) = {D}_{LR} - \left({L}_{1}+\frac{{S}_{IOL} + C}{1 - {τ}_{CL} \left({S}_{IOL}+C\right)}\right) {\left(1-{τ}_{L}\left({L}_{1}+\frac{{S}_{IOL} + C}{1 - {τ}_{CL} \left({S}_{IOL}+C\right)}\right)\right)}^{- 1}$ (26).

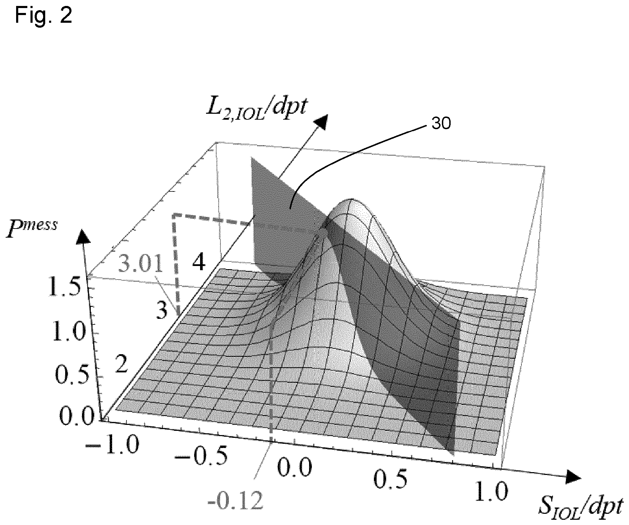

Die Zwangsbedingung bedeutet, dass man sich nur auf der in der Figur 2 gezeigten Schnittfläche 30 bewegen darf.

Substituiert man *L_{2,IOL}(S_{IOL})* in Gleichung (24) und maximiert man nach *S_{IOL},* d.h. löst man $\frac{d}{{dS}_{IOL}} {P}^{mess} \left({S}_{IOL}^{mess},{L}_{2 , IOL}^{mess}\left|{S}_{IOL},{L}_{2 , IOL}\left({S}_{IOL}\right)\right.\right) = 0$ (27) **nach** *S_{IOL}* auf, erhält man $\begin{matrix}{S}_{IOL} = - 0.12 dpt \\ {L}_{2 , IOL} \left({S}_{IOL}\right) = 3.01 dpt\end{matrix}$ (28).

Beide Größen *S_{IOL},L_{2,JOL}* gehen also in Richtung Minus im Vergleich zu den Messwerten, aber nicht im gleichen Maße. Vielmehr sucht das Verfahren eine Balance im Lichte der unterschiedlichen Standardabweichungen und der unsymmetrischen Lage der Zwangsbedingung relativ zur Gaußglocke.

Inkonsistenzen im Augenmodell können nicht nur bei einer berechneten Augenlänge (bzw. einem berechneten Linsen-Netzhaut-Abstand) auftreten, sondern z.B. auch bei einer Messung der Augenlänge. Solche Inkonsistenzen lassen sich analog zum oben beschrieben Beispiel einer berechneten Augenlänge auflösen. Selbstverständlich können auch komplexere Beispiele angegeben werden, bei denen die Augenlänge selbst auch nicht fest ist, oder ggf. Korrelationen auftreten.

### Bezugszeichenliste

- 10: Hauptstrahl
- 12: Auge
- 14: erste Fläche des Brillenglases (Vorderfläche)
- 16: zweite Fläche des Brillenglases (Rückfläche)
- 18: Hornhautvorderfläche
- 20: Augenlinse
- 30: Schnittfläche

## Patentansprüche

1. Computerimplementiertes Verfahren zum Berechnen oder Optimieren eines Brillenglases für zumindest ein Auge eines Brillenträgers zum Zweck der Herstellung des Brillenglases für das zumindest eine Auge, welchem im Rahmen einer Operation eine Intraokularlinse implantiert wurde, umfassend:
- ein Verfahren zum Ermitteln relevanter individueller Parameter des zumindest einen Auges des Brillenträgers;
- Vorgeben einer ersten Fläche (14) und einer zweiten Fläche (16) für das zu berechnende bzw. optimierende Brillenglas;
- Ermitteln des Verlaufs eines Hauptstrahls (10) durch zumindest einen Durchblickspunkt (i) zumindest einer zu berechnenden oder optimierenden Fläche (14; 16) des Brillenglases in das Modellauge (12);
- Auswerten einer Aberration einer entlang des Hauptstrahls aus einer auf die erste Fläche des Brillenglases auftreffenden sphärischen Wellenfront resultierenden Wellenfront an einer Bewertungsfläche im Vergleich zu einer in einem Punkt auf der Netzhaut des Augenmodells konvergierenden Wellenfront;
- iteratives Variieren der zumindest einen zu berechnenden oder optimierenden Fläche (14; 16) des Brillenglases, bis die ausgewertete Aberration einer vorgegebenen Sollaberration entspricht;
wobei das Verfahren zum Ermitteln relevanter individueller Parameter des zumindest einen Auges des Brillenträgers die folgenden Schritte umfasst:
- Bereitstellen von individuellen Refraktionsdaten des zumindest einen Auges des Brillenträgers; und
- Festlegen eines individuellen Augenmodells, in welchem zumindest
-- eine Form und/oder Wirkung einer Hornhaut, insbesondere einer Hornhautvorderfläche (18), eines Modellauges (12);
-- ein Hornhaut-Linsen-Abstand;
-- Parameter der Linse des Modellauges; und
-- ein Linsen-Netzhaut-Abstand
als Parameter des individuellen Augenmodells festgelegt werden, wobei das Festlegen der Parameter des individuellen Augenmodells derart anhand von Daten zur Sehschärfekorrektur des zumindest einen, die Intraokularlinse aufweisenden Auges und ferner anhand von individuellen Messwerten für das Auge des Brillenträgers und/oder von Standardwerten und/oder anhand der bereitgestellten individuellen Refraktionsdaten erfolgt, dass das Modellauge (12) die bereitgestellten individuellen Refraktionsdaten aufweist, wobei die Daten zur Sehschärfekorrektur einen ermittelten Linsen-Netzhaut-Abstand und/oder Intraokularlinsendaten umfassen;
wobei das Augenmodell ein konsistentes Augenmodell ist, und wobei eine initiale Verteilung von Parametern des Augenmodells und individuelle Daten von Eigenschaften des zumindest einen Auges bereitgestellt werden, und wobei die Parameter des individuellen Augenmodells auf Basis der initialen Verteilung von Parametern des Augenmodells und der individuellen Daten unter Verwendung von Wahrscheinlichkeitsrechnung bestimmt werden.

2. Computerimplementiertes Verfahren nach Anspruch 1, wobei:
a) im Fall von bekannten Intraokularlinsendaten das Festlegen der Parameter des individuellen Augenmodells derart anhand von Intraokularlinsendaten und ferner anhand von individuellen Messwerten für das Auge des Brillenträgers und/oder von Standardwerten und/oder anhand der bereitgestellten individuellen Refraktionsdaten erfolgt, dass das Modellauge (12) die bereitgestellten individuellen Refraktionsdaten aufweist, wobei das Festlegen der Parameter der Linse des Modellauges (12) anhand der Intraokularlinsendaten erfolgt; und/oder
b) im Fall von unbekannten Intraokularlinsendaten ein Linsen-Netzhaut-Abstand des Auges des Brillenträgers durch Messen und/oder auf Basis eines individuellen Vor-OP-Augenmodells ermittelt wird, und das Festlegen der Parameter des individuellen Augenmodells derart anhand des ermittelten Linsen-Netzhaut-Abstands und ferner anhand von individuellen Messwerten für das Auge des Brillenträgers und/oder von Standardwerten und/oder anhand der bereitgestellten individuellen Refraktionsdaten erfolgt, dass das Modellauge (12) die bereitgestellten individuellen Refraktionsdaten aufweist, wobei der Linsen-Netzhaut-Abstand des Modellauges (12) durch den ermittelten Linsen-Netzhaut-Abstand des Auges des Brillenträgers festgelegt wird.

3. Computerimplementiertes Verfahren nach einem der vorangehenden Ansprüche, wobei zumindest das Festlegen des Linsen-Netzhaut-Abstands durch Messen und/oder Berechnen erfolgt.

4. Computerimplementiertes Verfahren nach einem der vorangehenden Ansprüche,
wobei die Intraokularlinsendaten zumindest einen Defokus der Vorderfläche der Intraokularlinse, einen Defokus der Rückfläche der Intraokularlinse und eine Dicke der Intraokularlinse umfassen; und/oder
wobei die Intraokularlinsendaten zumindest einen Defokus des Brechwerts der Intraokularlinse umfassen, und/oder
wobei die Intraokularlinsendaten eine Angabe zur A-Konstante umfassen; und/oder
wobei die Intraokularlinsendaten auf Basis von Typ- oder Seriennummer-Informationen bereitgestellt werden.

5. Computerimplementiertes Verfahren nach einem der vorangehenden Ansprüche, ferner umfassend die Schritte:
- Durchführen einer Konsistenzprüfung des festgelegten Augenmodells, und
- Auflösen etwaiger Inkonsistenzen, insbesondere mit Hilfe von analytischen und/oder numerischen und/oder probabilistischen Verfahren, wobei das Auflösen etwaiger Inkonsistenzen vorzugsweise dadurch erfolgt, dass:
ein oder mehrere Parameter des Augenmodells angepasst werden, wobei vorzugsweise eine Anpassung von mehreren Parametern des Augenmodells vorgenommen und die Anpassung auf die mehreren Parameter des Augenmodells aufgeteilt wird; und/oder
zumindest ein neuer Parameter zum Augenmodell hinzugefügt und derart festgelegt wird, dass das Augenmodell konsistent wird; und/oder
eine Zielwirkung des Brillenglases angepasst wird.

6. Computerimplementiertes Verfahren nach einem der vorangehenden Ansprüche, wobei die bereitgestellten individuellen Refraktionsdaten des zumindest einen Auges des Brillenträgers individuelle Nach-OP Refraktionsdaten des zumindest einen Auges des Brillenträgers sind, und wobei das individuelle Augenmodell ein Nach-OP-Augenmodell ist.

7. Computerimplementiertes Verfahren nach Anspruch 6, ferner umfassend
- Bereitstellen von individuellen Vor-OP-Refraktionsdaten des zumindest einen Auges des Brillenträgers, wobei
das Bestimmen eines Linsen-Netzhaut-Abstands des Auges des Brillenträgers auf Basis eines individuellen Vor-OP-Augenmodells unter Verwendung der bereitgestellten individuellen Vor-OP-Refraktionsdaten basiert.

8. Computerimplementiertes Verfahren nach Anspruch 7, wobei in dem Vor-OP-Augenmodell zumindest
-- eine Form und/oder Wirkung einer Hornhaut, insbesondere einer Hornhautvorderfläche (18), eines Modellauges (12) des Vor-OP-Augenmodells;
-- ein Hornhaut-Linsen-Abstand des Modellauges (12) des Vor-OP-Augenmodells;
-- Parameter der Linse des Modellauges (12) des Vor-OP-Augenmodells; und
-- ein Linsen-Netzhaut-Abstand des Modellauges (12) des Vor-OP-Augenmodells
derart anhand von individuellen Messwerten für das Auge des Brillenträgers und/oder von Standardwerten und/oder anhand der bereitgestellten individuellen Vor-OP-Refraktionsdaten festgelegt werden, dass das Modellauge (12) die bereitgestellten individuellen Vor-OP-Refraktionsdaten aufweist, wobei zumindest das Festlegen des Linsen-Netzhaut-Abstands insbesondere durch Messen und/oder Berechnen erfolgt.

9. Verfahren zum Herstellen eines Brillenglases, umfassend:
Berechnen oder Optimieren eines Brillenglases nach dem Verfahren zum Berechnen oder Optimieren eines Brillenglases gemäß einem der vorangehenden Ansprüche; und
Fertigen des so berechneten oder optimierten Brillenglases.

10. Vorrichtung zum Berechnen oder Optimieren eines Brillenglases für zumindest ein Auge eines Brillenträgers zum Zweck der Herstellung des Brillenglases für das zumindest eine Auge, welches eine implantierte Intraokularlinse aufweist, umfassend:
- eine Vorrichtung zum Ermitteln relevanter individueller Parameter des zumindest einen Auges des Brillenträgers;
- eine Flächenmodelldatenbank zum Vorgeben einer ersten Fläche (14) und einer zweiten Fläche (16) für das zu berechnende bzw. optimierende Brillenglas;
- ein Hauptstrahlermittlungsmodul zum Ermitteln des Verlaufs eines Hauptstrahls (10) durch zumindest einen Durchblickspunkt (i) zumindest einer zu berechnenden oder optimierenden Fläche (14; 16) des Brillenglases in das Modellauge (12);
- ein Auswertemodul zum Auswerten einer Aberration einer entlang des Hauptstrahls aus einer auf die erste Fläche des Brillenglases auftreffenden sphärischen Wellenfront resultierenden Wellenfront an einer Bewertungsfläche im Vergleich zu einer in einem Punkt auf der Netzhaut des Augenmodells konvergierenden Wellenfront; und
- ein Optimierungsmodul zum iterativen Variieren der zumindest einen zu berechnenden oder optimierenden Fläche (14; 16) des Brillenglases, bis die ausgewertete Aberration einer vorgegebenen Sollaberration entspricht;
wobei die Vorrichtung zum Ermitteln relevanter individueller Parameter des zumindest einen Auges des Brillenträgers umfasst:
- zumindest eine Datenschnittstelle zum Bereitstellen von individuellen Refraktionsdaten des zumindest einen Auges des Brillenträgers; und
- ein Modellierungsmodul zum Festlegen eines individuellen Augenmodells, welches zumindest
-- eine Form und/oder Wirkung einer Hornhaut, insbesondere einer Hornhautvorderfläche (18), eines Modellauges (12);
-- einen Hornhaut-Linsen-Abstand;
-- Parameter der Linse des Modellauges; und
-- einen Linsen-Netzhaut-Abstand
als Parameter des individuellen Augenmodells festlegt, wobei das Festlegen der Parameter des individuellen Augenmodells derart anhand von Daten zur Sehschärfekorrektur des zumindest einen, die Intraokularlinse aufweisenden Auges und ferner anhand von individuellen Messwerten für das Auge des Brillenträgers und/oder von Standardwerten und/oder anhand der bereitgestellten individuellen Refraktionsdaten erfolgt, dass das Modellauge (12) die bereitgestellten individuellen Refraktionsdaten aufweist, wobei die Daten zur Sehschärfekorrektur einen ermittelten Linsen-Netzhaut-Abstand und/oder Intraokularlinsendaten umfassen;
wobei das Augenmodell ein konsistentes Augenmodell ist, und wobei die Vorrichtung zum Ermitteln relevanter individueller Parameter des zumindest einen Auges des Brillenträgers ausgelegt ist, die Parameter des individuellen Augenmodells auf Basis einer initialen Verteilung von Parametern des Augenmodells und individuellen Daten von Eigenschaften des zumindest einen Auges unter Verwendung von Wahrscheinlichkeitsrechnung zu bestimmen.

11. Vorrichtung zum Herstellen eines Brillenglases, umfassend:
Berechnungs- oder Optimierungsmittel, welche ausgelegt sind, das Brillenglas nach einem Verfahren zum Berechnen oder Optimieren eines Brillenglases gemäß einem der Ansprüche 1 bis 8 zu berechnen oder zu optimieren; und
Bearbeitungsmittel, welche ausgelegt sind, das Brillenglas gemäß dem Ergebnis der Berechnung bzw. Optimierung zu bearbeiten.

12. Computerprogrammerzeugnis, welches Programmcode enthält, der ausgelegt ist, wenn geladen und ausgeführt auf einem Computer, ein Verfahren zum Berechnen oder Optimieren eines Brillenglases gemäß einem der Ansprüche 1 bis 8 durchzuführen.

## Claims

1. A computer-implemented method for calculating or optimizing a spectacle lens for at least one eye of a spectacle wearer for the purpose of manufacturing the spectacle lens for the at least one eye into which an intraocular lens has been implanted as part of surgery, comprising:
- a method for determining relevant individual parameters of the at least one eye of the spectacle wearer;
- specifying a first surface (14) and a second surface (16) for the spectacle lens to be calculated or optimized;
- identifying the course of a main ray (10) through at least one visual point (*i*) of at least one surface (14; 16) of the spectacle lens to be calculated or optimized into the model eye (12);
- evaluating an aberration of a wavefront resulting from a spherical wavefront incident on the first surface of the spectacle lens along the main ray on an evaluation surface compared to a wavefront converging at a point on the retina of the eye model;
- iteratively varying the at least one surface (14; 16) of the spectacle lens to be calculated or optimized until the evaluated aberration corresponds to a predetermined target aberration;
wherein the method for determining relevant individual parameters of the at least one eye of the spectacle wearer comprises the following steps:
- providing individual refraction data for the at least one eye of the spectacle wearer; and
- defining an individual eye model in which at least
-- a shape and/or power of a cornea, in particular a corneal front surface (18), of a model eye (12);
-- a cornea-lens distance;
-- parameters of the lens of the model eye; and
-- a lens-retina distance
are defined as parameters of the individual eye model, wherein the parameters of the individual eye model are defined based on visual acuity correction data of the at least one eye containing the intraocular lens, as well as based on individual measurement values for the spectacle wearer's eye and/or standard values and/or based on the provided individual refraction data, such that the model eye (12) includes the provided individual refraction data, wherein the data for visual acuity correction comprise a determined lens-retina distance and/or intraocular lens data;
wherein the eye model is a consistent eye model, and wherein an initial distribution of parameters of the eye model and individual data regarding characteristics of the at least one eye are provided, and wherein the parameters of the individual eye model are determined based on the initial distribution of parameters of the eye model and the individual data using probability calculations.

2. A computer-implemented method according to claim 1, wherein:
a) in the case of known intraocular lens data, the parameters of the individual eye model are determined on the basis of intraocular lens data and, furthermore, on the basis of individual measurement values for the spectacle wearer's eye and/or standard values and/or on the basis of the provided individual refraction data, such that the model eye (12) has the provided individual refraction data, wherein the parameters of the lens of the model eye (12) are determined based on the intraocular lens data; and/or
b) in the case of unknown intraocular lens data, a lens-retina distance of the spectacle wearer's eye is determined by measurement and/or based on an individual pre-surgery eye model, and the parameters of the individual eye model are determined based on the determined lens-retina distance and, furthermore, based on individual measurement values for the spectacle wearer's eye and/or standard values and/or the provided individual refraction data, such that the model eye (12) includes the provided individual refraction data, wherein the lens-retina distance of the model eye (12) is determined by the determined lens-retina distance of the spectacle wearer's eye.

3. A computer-implemented method according to any one of the preceding claims, wherein at least the determination of the lens-retina distance is performed by measuring and/or calculating.

4. A computer-implemented method according to any one of the preceding claims,
wherein the intraocular lens data comprise at least a defocus of the front surface of the intraocular lens, a defocus of the back surface of the intraocular lens, and a thickness of the intraocular lens; and/or
wherein the intraocular lens data comprise at least a defocus of the refractive power of the intraocular lens, and/or
wherein the intraocular lens data includes a specification of the A-constant; and/or
wherein the intraocular lens data is provided based on type or serial number information.

5. A computer-implemented method according to any one of the preceding claims, further comprising the steps of:
- performing a consistency check on the defined eye model, and
- resolving any inconsistencies, in particular using analytical and/or numerical and/or probabilistic methods, wherein the resolution of any inconsistencies is preferably achieved in that:
one or more parameters of the eye model are adjusted, wherein, preferably, an adjustment of several parameters of the eye model is performed and the adjustment is distributed among the several parameters of the eye model; and/or
at least one new parameter is added to the eye model and defined such that the eye model becomes consistent; and/or
a target power of the spectacle lens is adjusted.

6. A computer-implemented method according to any one of the preceding claims, wherein the provided individual refraction data of the at least one eye of the spectacle wearer is individual post-surgery refraction data of the at least one eye of the spectacle wearer, and wherein the individual eye model is a post-surgery eye model.

7. A computer-implemented method according to claim 6, further comprising
- providing individual pre-surgery refraction data for the at least one eye of the spectacle wearer, wherein
the determination of a lens-retina distance of the spectacle wearer's eye is based on an individual pre-surgery eye model using the provided individual pre-surgery refraction data.

8. A computer-implemented method according to claim 7, wherein the pre-surgery eye model includes at least
-- a shape and/or power of a cornea, in particular a corneal front surface (18), of a model eye (12) of the pre-surgery eye model;
-- a cornea-lens distance of the model eye (12) of the pre-surgery eye model;
-- parameters of the lens of the model eye (12) of the pre-surgery eye model; and
-- a lens-retina distance of the model eye (12) of the pre-surgery eye model are defined on the basis of individual measurement values for the spectacle wearer's eye and/or standard values and/or the provided individual pre-surgery refraction data, such that the model eye (12) has the provided individual pre-surgery refraction data, wherein at least defining the lens-retina distance takes place, in particular, by measuring and/or calculating.

9. A method for manufacturing a spectacle lens, comprising:
calculating or optimizing a spectacle lens according to the method for calculating or optimizing a spectacle lens as set forth in any of the preceding claims; and
manufacturing the spectacle lens thus calculated or optimized.

10. A device for calculating or optimizing a spectacle lens for at least one eye of a spectacle wearer for the purpose of manufacturing the spectacle lens for the at least one eye, which has an implanted intraocular lens, comprising:
- a device for determining relevant individual parameters of the at least one eye of the spectacle wearer;
- a surface model database for specifying a first surface (14) and a second surface (16) for the spectacle lens to be calculated or optimized;
- a main ray identification module for identifying the course of a main ray (10) through at least one visual point (*i*) of at least one surface (14; 16) of the spectacle lens to be calculated or optimized into the model eye (12);
- an evaluation module for evaluating an aberration of a wavefront resulting from a spherical wavefront incident on the first surface of the spectacle lens along the main ray on an evaluation surface compared to a wavefront converging in one point on the retina of the eye model; and
- an optimization module for iteratively varying the at least one surface (14; 16) of the spectacle lens to be calculated or optimized until the evaluated aberration corresponds to a predetermined target aberration;
wherein the device for determining relevant individual parameters of the at least one eye of the spectacle wearer comprises:
- at least one data interface for providing individual refraction data of the at least one eye of the spectacle wearer; and
- a modeling module for defining an individual eye model, which at least defines
-- a shape and/or power of a cornea, in particular a corneal front surface (18), of a model eye (12);
-- a cornea-lens distance;
-- parameters of the lens of the model eye; and
-- a lens-retina distance
as parameters of the individual eye model, wherein the parameters of the individual eye model are determined based on visual acuity correction data for the at least one eye containing the intraocular lens, as well as on individual measurement values for the spectacle wearer's eye and/or standard values and/or the provided individual refraction data, such that the model eye (12) comprises the provided individual refraction data, wherein the visual acuity correction data comprise a determined lens-retina distance and/or intraocular lens data;
wherein the eye model is a consistent eye model, and wherein the device is configured to determine relevant individual parameters of the at least one eye of the spectacle wearer by determining the parameters of the individual eye model based on an initial distribution of eye model parameters and individual data regarding properties of the at least one eye, using probability calculations.

11. A device for manufacturing a spectacle lens, comprising:
calculation or optimization means configured to calculate or optimize the spectacle lens according to a method for calculating or optimizing a spectacle lens in accordance with any one of claims 1 to 8; and
machining means configured to machine the spectacle lens in accordance with the result of the calculation or optimization.

12. A computer program product containing program code configured to, when loaded and executed on a computer, perform a method for calculating or optimizing a spectacle lens according to any one of claims 1 to 8.

## Revendications

1. Procédé mis en œuvre par ordinateur pour calculer ou optimiser un verre de lunettes destiné à au moins un œil d'un porteur de lunettes, en vue de la fabrication dudit verre de lunettes pour ledit au moins un œil, dans lequel une lentille intraoculaire a été implantée dans le cadre d'une opération, comprenant :
- un procédé permettant de déterminer les paramètres individuels pertinents dudit au moins un œil du porteur de lunettes ;
- la définition d'une première surface (14) et d'une deuxième surface (16) pour le verre de lunettes à calculer ou à optimiser ;
- la détermination du tracé d'un rayon principal (10) passant par au moins un point de visée (*i*) d'au moins une surface (14; 16) du verre de lunettes à calculer ou à optimiser, dans l'œil modèle (12) ;
- évaluation d'une aberration d'un front d'onde résultant, le long du rayon principal, d'un front d'onde sphérique incident sur la première surface du verre de lunettes, au niveau d'une surface d'évaluation, par rapport à un front d'onde convergeant en un point sur la rétine du modèle d'œil ;
- modification itérative de la au moins une surface (14; 16) du verre de lunettes à calculer ou à optimiser, jusqu'à ce que l'aberration évaluée corresponde à une aberration de consigne prédéfinie ;
le procédé de détermination des paramètres individuels pertinents du au moins un œil du porteur de lunettes comprenant les étapes suivantes :
- la mise à disposition de données de réfraction individuelles d'au moins un œil du porteur de lunettes ; et
- la définition d'un modèle oculaire individuel, dans lequel au moins
-- une forme et/ou un effet d'une cornée, en particulier d'une surface antérieure de la cornée (18), d'un œil modèle (12) ;
-- une distance cornée-lentille ;
-- des paramètres du cristallin de l'œil modèle ; et
-- une distance cristallin-rétine sont définis comme paramètres du modèle oculaire individuel, la définition des paramètres du modèle oculaire individuel s'effectuant de telle sorte, à partir de données relatives à la correction de l'acuité visuelle dudit au moins un œil comportant une lentille intraoculaire et, en outre, à partir de valeurs de mesure individuelles pour l'œil du porteur de lunettes et/ ou de valeurs standard et/ou à partir des données de réfraction individuelles fournies, de telle sorte que l'œil modèle (12) présente les données de réfraction individuelles fournies, les données de correction de l'acuité visuelle comprenant une distance cristallin-rétine déterminée et/ou des données relatives à la lentille intraoculaire ;
le modèle oculaire étant un modèle oculaire cohérent, et une distribution initiale des paramètres du modèle oculaire ainsi que des données individuelles relatives aux caractéristiques dudit au moins un œil étant fournies, et les paramètres du modèle oculaire individuel étant déterminés sur la base de la distribution initiale des paramètres du modèle oculaire et des données individuelles, à l'aide du calcul des probabilités.

2. Procédé mis en œuvre par ordinateur selon la revendication 1, dans lequel :
a) dans le cas de données de lentille intraoculaire connues, la détermination des paramètres du modèle oculaire individuel s'effectue à partir des données de lentille intraoculaire, ainsi qu'à partir de valeurs de mesure individuelles relatives à l'œil du porteur de lunettes et/ou de valeurs standard et/ou à partir des données de réfraction individuelles fournies, de telle sorte que l'œil modèle (12) présente les données de réfraction individuelles fournies, la détermination des paramètres de la lentille de l'œil modèle (12) s'effectuant à partir des données relatives à la lentille intraoculaire ; et/ou
b) dans le cas où les données relatives à la lentille intraoculaire sont inconnues, une distance cristallin-rétine de l'œil du porteur de lunettes est déterminée par mesure et/ou sur la base d'un modèle oculaire individuel préopératoire, et la définition des paramètres du modèle oculaire individuel s'effectue ainsi à partir de la distance cristallin-rétine déterminée, ainsi qu'à partir de valeurs de mesure individuelles relatives à l'œil du porteur de lunettes et/ ou de valeurs standard et/ou à partir des données de réfraction individuelles fournies, de telle sorte que l'œil modèle (12) présente les données de réfraction individuelles fournies, la distance cristallin-rétine de l'œil modèle (12) étant déterminée par la distance cristallin-rétine déterminée de l'œil du porteur de lunettes.

3. Procédé mis en œuvre par ordinateur selon l'une des revendications précédentes, dans lequel au moins la détermination de la distance cristallin-rétine s'effectue par mesure et/ou calcul.

4. Procédé mis en œuvre sur ordinateur selon l'une des revendications précédentes, dans lequel les données relatives à la lentille intraoculaire comprennent au moins un défocalage de la face antérieure de la lentille intraoculaire, un défocalage de la face postérieure de la lentille intraoculaire et une épaisseur de la lentille intraoculaire ; et/ou
dans lequel les données relatives à la lentille intraoculaire comprennent au moins une défocalisation de la puissance de la lentille intraoculaire, et/ou
dans lequel les données relatives à la lentille intraoculaire comprennent une indication de la constante A ; et/ou
dans lequel les données relatives à la lentille intraoculaire sont fournies sur la base d'informations de type ou de numéro de série.

5. Procédé mis en œuvre par ordinateur selon l'une des revendications précédentes, comprenant en outre les étapes suivantes :
- effectuer un contrôle de cohérence du modèle oculaire défini, et
- résoudre d'éventuelles incohérences, notamment à l'aide de procédés analytiques et/ou numériques et/ou probabilistes, la résolution d'éventuelles incohérences s'effectuant de préférence en :
un ou plusieurs paramètres du modèle oculaire sont ajustés, un ajustement de plusieurs paramètres du modèle oculaire étant de préférence effectué et réparti entre lesdits plusieurs paramètres du modèle oculaire ; et/ou
au moins un nouveau paramètre est ajouté au modèle oculaire et défini de telle sorte que le modèle oculaire devienne cohérent ; et/ou
un effet cible du verre de lunettes est ajusté.

6. Procédé mis en œuvre par ordinateur selon l'une des revendications précédentes, dans lequel les données de réfraction individuelles fournies concernant au moins un œil du porteur de lunettes sont des données de réfraction individuelles postopératoires concernant au moins un œil du porteur de lunettes, et dans lequel le modèle oculaire individuel est un modèle oculaire postopératoire.

7. Procédé mis en œuvre par ordinateur selon la revendication 6, comprenant en outre
- la mise à disposition de données de réfraction individuelles préopératoires d'au moins un œil du porteur de lunettes,
la détermination d'une distance lentille-rétine de l'œil du porteur de lunettes étant basée sur un modèle oculaire individuel préopératoire à l'aide des données de réfraction individuelles préopératoires mises à disposition.

8. Procédé mis en œuvre par ordinateur selon la revendication 7, dans lequel le modèle oculaire préopératoire comprend au moins
-- une forme et/ou un effet d'une cornée, en particulier d'une surface antérieure de la cornée (18), d'un œil modèle (12) du modèle oculaire préopératoire ;
-- une distance cornéo-lenticulaire de l'œil modèle (12) du modèle oculaire préopératoire ;
-- des paramètres de la lentille de l'œil modèle (12) du modèle oculaire préopératoire ; et
-- une distance lenticulo-rétinienne de l'œil modèle (12) du modèle oculaire préopératoire
sont déterminés à partir de valeurs de mesure individuelles relatives à l'œil du porteur de lunettes et/ou de valeurs standard et/ou à partir des données de réfraction préopératoires individuelles fournies, de telle sorte que l'œil modèle (12) présente les données de réfraction préopératoires individuelles fournies, la détermination de la distance cristallin-rétine s'effectuant notamment par mesure et/ou calcul.

9. Procédé de fabrication d'un verre de lunettes, comprenant :
le calcul ou l'optimisation d'un verre de lunettes selon le procédé de calcul ou d'optimisation d'un verre de lunettes conformément à l'une des revendications précédentes ; et
la fabrication du verre de lunettes ainsi calculé ou optimisé.

10. Dispositif destiné à calculer ou à optimiser un verre de lunettes pour au moins un œil d'un porteur de lunettes, en vue de la fabrication dudit verre de lunettes pour ledit au moins un œil comportant une lentille intraoculaire implantée, comprenant :
- un dispositif destiné à déterminer les paramètres individuels pertinents dudit au moins un œil du porteur de lunettes ;
- une base de données de modèles de surfaces permettant de définir une première surface (14) et une deuxième surface (16) pour le verre de lunettes à calculer ou à optimiser ;
- un module de détermination du rayon principal destiné à déterminer le tracé d'un rayon principal (10) passant par au moins un point de visée (*i*) d'au moins une surface (14; 16) du verre de lunettes à calculer ou à optimiser, dans l'œil modèle (12) ;
- un module d'évaluation destiné à évaluer, au niveau d'une surface d'évaluation, l'aberration d'un front d'onde résultant, le long du rayon principal, d'un front d'onde sphérique incident sur la première surface du verre de lunettes, par rapport à un front d'onde convergeant en un point sur la rétine du modèle d'œil ; et
- un module d'optimisation destiné à faire varier de manière itérative la au moins une surface (14 ; 16) du verre de lunettes à calculer ou à optimiser, jusqu'à ce que l'aberration évaluée corresponde à une aberration de consigne prédéfinie ;
le dispositif destiné à déterminer les paramètres individuels pertinents du au moins un œil du porteur de lunettes comprenant :
- au moins une interface de données destinée à fournir des données de réfraction individuelles concernant ledit au moins un œil du porteur de lunettes ; et
- un module de modélisation destiné à définir un modèle oculaire individuel, qui comprend au moins
-- une forme et/ou un effet d'une cornée, en particulier d'une surface antérieure de la cornée (18), d'un œil modèle (12) ;
-- une distance cornéo-lenticulaire ;
-- des paramètres du cristallin de l'œil modèle ; et
- une distance cristallin-rétine
en tant que paramètres du modèle oculaire individuel, la définition des paramètres du modèle oculaire individuel s'effectuant à partir de données relatives à la correction de l'acuité visuelle dudit au moins un œil comportant une lentille intraoculaire, ainsi qu'à partir de valeurs de mesure individuelles pour l'œil du porteur de lunettes et/ou de valeurs standard et/ou à partir des données de réfraction individuelles fournies, de sorte que l'œil modèle (12) comporte les données de réfraction individuelles fournies, les données de correction de l'acuité visuelle comprenant une distance lentille-rétine déterminée et/ou des données relatives à la lentille intraoculaire ;
le modèle oculaire étant un modèle oculaire cohérent, et le dispositif étant conçu pour déterminer les paramètres individuels pertinents dudit au moins un œil du porteur de lunettes, en déterminant les paramètres du modèle oculaire individuel sur la base d'une distribution initiale de paramètres du modèle oculaire et de données individuelles relatives aux caractéristiques dudit au moins un œil, à l'aide du calcul des probabilités.

11. Dispositif destiné à la fabrication d'un verre de lunettes, comprenant :
des moyens de calcul ou d'optimisation, qui sont conçus pour calculer ou optimiser le verre de lunettes selon un procédé de calcul ou d'optimisation d'un verre de lunettes conformément à l'une des revendications 1 à 8 ; et
des moyens d'usinage, conçus pour usiner le verre de lunettes conformément au résultat du calcul ou de l'optimisation.

12. Produit logiciel contenant un code de programme conçu pour, lorsqu'il est chargé et exécuté sur un ordinateur, mettre en œuvre un procédé de calcul ou d'optimisation d'un verre de lunettes selon l'une des revendications 1 à 8.
